# EUROPEAN PATENT APPLICATION

(11) **EP 2 248 910 A1**
(43) Date of publication of application: **10.11.2010**
(21) Application number: 10170170.4
(22) Date of filing: 27.04.2001
(51) Int. Cl.: C12Q 1/37, G01N 33/569, C07K 14/00, A61K 39/00, A61P 35/00, A61P 31/00, A61P 33/00

(54) **Epitope synchronization in antigen presenting cells**

(30) Priority: 28.04.2000 US 560465; 28.04.2000 US 561074; 28.04.2000 US 561572; 28.04.2000 US 561571
(62) Divisional of application: 01930922.8
(71) Applicant: Mannkind Corporation, Valencia, California 91355 (US)
(72) Inventor: Simard, John J. L., Vancouver British Columbia Z6G 1A6 (CA); Diamond, David C., West Hills, CA 91304 (US); Lei, Xiang-Dong, Germantown, MD 20876 (US)
(74) Representative: Neuefeind, Regina

(57) **Abstract**

Disclosed herein are vaccines and methods for inducing an immune response against cancer cells and cells infected with intracellular parasites. Vaccines having housekeeping epitopes are disclosed. The housekeeping epitope is formed by housekeeping proteasomes in peripheral cells, but not by professional antigen presenting cells. A vaccine containing a housekeeping epitope that is derived from an antigen associated with a peripheral target cell can thus direct an immune response against the target cell. Methods of treatment are also disclosed, which involve administering a vaccine having a housekeeping epitope.

SEQUENCE LISTING

<110> CTL IMMUNOTHERAPIES CORP.
      SIMARD, John, J., L.
      DIAMOND, David, C.
      LEI, Xiang-Dong

<120> EPITOPE SYNCHRONIZATION IN ANTIGEN
  PRESENTING CELLS

<130> CTLIMM.004VPC

<150> US 09/561,572
<151> 2000-04-28

<150> US 09/561,571
<151> 2000-04-28

<150> US 09/561,074
<151> 2000-04-28

<150> US 09/560,465
<151> 2000-04-28

<160> 82

<170> FastSEQ for Windows Version 4.0

<210> 1
<211> 118
<212> PRT
<213> Homo sapiens

<400> 1
Met Pro Arg Glu Asp Ala His Phe Ile Tyr Gly Tyr Pro Lys Lys Gly
 1               5                  10                  15
His Gly His Ser Tyr Thr Thr Ala Glu Glu Ala Ala Gly Ile Gly Ile
            20                  25                  30
Leu Thr Val Ile Leu Gly Val Leu Leu Leu Ile Gly Cys Trp Tyr Cys
        35                  40                  45
Arg Arg Arg Asn Gly Tyr Arg Ala Leu Met Asp Lys Ser Leu His Val
    50                  55                  60
Gly Thr Gln Cys Ala Leu Thr Arg Arg Cys Pro Gln Glu Gly Phe Asp
65                  70                  75                  80
His Arg Asp Ser Lys Val Ser Leu Gln Glu Lys Asn Cys Glu Pro Val
                85                  90                  95
Val Pro Asn Ala Pro Pro Ala Tyr Glu Lys Leu Ser Ala Glu Gln Ser
            100                 105                 110
Pro Pro Pro Tyr Ser Pro
        115


<210> 2
<211> 188
<212> PRT
<213> Homo sapiens

<400> 2
Met Asn Gly Asp Asp Ala Phe Ala Arg Arg Pro Thr Val Gly Ala Gln
 1               5                  10                  15

Ile Pro Glu Lys Ile Gln Lys Ala Phe Asp Asp Ile Ala Lys Tyr Phe
            20                  25                  30
Ser Lys Glu Glu Trp Glu Lys Met Lys Ala Ser Glu Lys Ile Phe Tyr
        35                  40                  45
Val Tyr Met Lys Arg Lys Tyr Glu Ala Met Thr Lys Leu Gly Phe Lys
    50                  55                  60
Ala Thr Leu Pro Pro Phe Met Cys Asn Lys Arg Ala Glu Asp Phe Gln
65                  70                  75                  80
Gly Asn Asp Leu Asp Asn Asp Pro Asn Arg Gly Asn Gln Val Glu Arg
                85                  90                  95
Pro Gln Met Thr Phe Gly Arg Leu Gln Gly Ile Ser Pro Lys Ile Met
            100                 105                 110
Pro Lys Lys Pro Ala Glu Glu Gly Asn Asp Ser Glu Glu Val Pro Glu
        115                 120                 125
Ala Ser Gly Pro Gln Asn Asp Gly Lys Glu Leu Cys Pro Pro Gly Lys
    130                 135                 140
Pro Thr Thr Ser Glu Lys Ile His Glu Arg Ser Gly Pro Lys Arg Gly
145                 150                 155                 160
Glu His Ala Trp Thr His Arg Leu Arg Glu Arg Lys Gln Leu Val Ile
                165                 170                 175
Tyr Glu Glu Ile Ser Asp Pro Glu Glu Asp Asp Glu
            180                 185

<210> 3
<211> 180
<212> PRT
<213> Homo sapiens

<400> 3
Met Gln Ala Glu Gly Arg Gly Thr Gly Gly Ser Thr Gly Asp Ala Asp
1               5                   10                  15
Gly Pro Gly Gly Pro Gly Ile Pro Asp Gly Pro Gly Gly Asn Ala Gly
            20                  25                  30
Gly Pro Gly Glu Ala Gly Ala Thr Gly Gly Arg Gly Pro Arg Gly Ala
        35                  40                  45
Gly Ala Ala Arg Ala Ser Gly Pro Gly Gly Gly Ala Pro Arg Gly Pro
    50                  55                  60
His Gly Gly Ala Ala Ser Gly Leu Asn Gly Cys Cys Arg Cys Gly Ala
65                  70                  75                  80
Arg Gly Pro Glu Ser Arg Leu Leu Glu Phe Tyr Leu Ala Met Pro Phe
                85                  90                  95
Ala Thr Pro Met Glu Ala Glu Leu Ala Arg Arg Ser Leu Ala Gln Asp
            100                 105                 110
Ala Pro Pro Leu Pro Val Pro Gly Val Leu Leu Lys Glu Phe Thr Val
        115                 120                 125
Ser Gly Asn Ile Leu Thr Ile Arg Leu Thr Ala Ala Asp His Arg Gln
    130                 135                 140
Leu Gln Leu Ser Ile Ser Ser Cys Leu Gln Gln Leu Ser Leu Leu Met
145                 150                 155                 160
Trp Ile Thr Gln Cys Phe Leu Pro Val Phe Leu Ala Gln Pro Pro Ser
                165                 170                 175
Gly Gln Arg Arg
            180

<210> 4
<211> 529
<212> PRT
<213> Homo sapiens

<400> 4

<210> 5
<211> 228
<212> DNA
<213> Saccharomyces Cerevisiae

<400> 5
atgcagattt tcgtcaagac tttgaccggt aaaaccataa cattggaagt tgaatcttcc 60
gataccatcg acaacgttaa gtcgaaaatt caagacaagg aaggtatccc tccagatcaa 120
caaagattga tctttgccgg taagcagcta gaagacggta gaacgctgtc tgattacaac 180
attcagaagg agtccacctt acatcttgtg ctaaggctaa gaggtggc             228

<210> 6
<211> 581
<212> DNA
<213> Encephalomyocarditis Virus

<400> 6
aattccgccc ctctccctcc ccccccccta acgttactgg ccgaagccgc ttggaataag 60
gccggtgtgc gtttgtctat atgtgatttt ccaccatatt gccgtctttt ggcaatgtga 120
gggcccggaa acctggccct gtcttcttga cgagcattcc taggggtctt tcccctctcg 180
ccaaaggaat gcaaggtctg ttgaatgtcg tgaaggaagc agttcctctg gaagcttctt 240
gaagacaaac aacgtctgta gcgacccttt gcaggcagcg gaacccccca cctggcgaca 300
ggtgcctctg cggccaaaag ccacgtgtat aagatacacc tgcaaaggcg gcacaacccc 360
agtgccacgt tgtgagttgg atagttgtgg aaagagtcaa atggctctcc tcaagcgtat 420
tcaacaaggg gctgaaggat gcccagaagg taccccattg tatgggatct gatctggggc 480
ctcggtgcac atgctttaca tgtgtttagt cgaggttaaa aaaacgtcta ggccccccga 540
accacgggga cgtggttttc ctttgaaaaa cacgatgata a                    581

<210> 7
<211> 16
<212> PRT
<213> Homo sapiens

<400> 7
Met Leu Leu Ala Val Leu Tyr Cys Leu Leu Trp Ser Phe Gln Thr Ser
 1               5                  10                  15

<210> 8
<211> 22
<212> PRT
<213> Homo sapiens

<400> 8
His Ser Tyr Thr Thr Ala Glu Glu Ala Ala Gly Ile Thr Ile Leu Thr
 1               5                  10                  15
Val Ile Leu Gly Val Leu
            20

<210> 9

<211> 24
<212> PRT
<213> Homo sapiens

<400> 9
Glu Ala Ala Ser Ser Ser Ser Thr Leu Val Glu Val Thr Leu Gly Glu
1               5                   10                  15
Val Pro Ala Ala Glu Ser Pro Asp
            20


<210> 10
<211> 24
<212> PRT
<213> Homo sapiens

<400> 10
Glu Phe Leu Trp Gly Pro Arg Ala Leu Val Glu Thr Ser Tyr Val Lys
1               5                   10                  15
Val Leu His His Met Val Lys Ile
            20


<210> 11
<211> 19
<212> PRT
<213> Homo sapiens

<400> 11
Ala Pro Glu Glu Lys Ile Trp Glu Glu Leu Ser Val Leu Glu Val Phe
1               5                   10                  15
Glu Gly Arg


<210> 12
<211> 16
<212> PRT
<213> Homo sapiens

<400> 12
Glu Leu Met Glu Val Asp Pro Ile Gly His Leu Tyr Ile Phe Ala Thr
1               5                   10                  15


<210> 13
<211> 15
<212> PRT
<213> Homo sapiens

<400> 13
Phe Leu Trp Gly Pro Arg Ala Leu Val Glu Thr Ser Tyr Val Lys
1               5                   10                  15


<210> 14
<211> 16
<212> PRT
<213> Homo sapiens

<400> 14

Met Leu Leu Ala Val Leu Tyr Cys Leu Leu Trp Ser Phe Gln Thr Ser
1               5                   10                  15

<210> 15
<211> 22
<212> PRT
<213> Homo sapiens

<400> 15
His Ser Tyr Thr Thr Ala Glu Glu Ala Ala Gly Ile Thr Ile Leu Thr
1               5                   10                  15
Val Ile Leu Gly Val Leu
            20

<210> 16
<211> 24
<212> PRT
<213> Homo sapiens

<400> 16
Glu Ala Ala Ser Ser Ser Ser Thr Leu Val Glu Val Thr Leu Gly Glu
1               5                   10                  15
Val Pro Ala Ala Glu Ser Pro Asp
            20

<210> 17
<211> 24
<212> PRT
<213> Homo sapiens

<400> 17
Glu Phe Leu Trp Gly Pro Arg Ala Leu Val Glu Thr Ser Tyr Val Lys
1               5                   10                  15
Val Leu His His Met Val Lys Ile
            20

<210> 18
<211> 19
<212> PRT
<213> Homo sapiens

<400> 18
Ala Pro Glu Glu Lys Ile Trp Glu Glu Leu Ser Val Leu Glu Val Phe
1               5                   10                  15
Glu Gly Arg

<210> 19
<211> 16
<212> PRT
<213> Homo sapiens

<400> 19
Glu Leu Met Glu Val Asp Pro Ile Gly His Leu Tyr Ile Phe Ala Thr
1               5                   10                  15

<210> 20
<211> 15
<212> PRT
<213> Homo sapiens

<400> 20
Phe Leu Trp Gly Pro Arg Ala Leu Val Glu Thr Ser Tyr Val Lys
1               5                   10                  15


<210> 21
<211> 10
<212> PRT
<213> Homo sapiens

<400> 21
Leu Gln Leu Ser Asn Gly Asn Arg Thr Leu
1               5                   10


<210> 22
<211> 10
<212> PRT
<213> Homo sapiens

<400> 22
Leu Gln Leu Ser Asn Asp Asn Arg Thr Leu
1               5                   10


<210> 23
<211> 10
<212> PRT
<213> Homo sapiens

<400> 23
Leu Gln Leu Ser Asn Gly Asn Arg Thr Leu
1               5                   10


<210> 24
<211> 10
<212> PRT
<213> Homo sapiens

<400> 24
Leu Leu Val His Asn Leu Pro Gln His Leu
1               5                   10


<210> 25
<211> 10
<212> PRT
<213> Homo sapiens

<400> 25
Leu Ser Asn Asp Asn Arg Thr Leu Thr Leu
1               5                   10

<210> 26
<211> 10
<212> PRT
<213> Homo sapiens

<400> 26
Leu Ser Asn Gly Asn Arg Thr Leu Thr Leu
1               5                   10


<210> 27
<211> 10
<212> PRT
<213> Homo sapiens

<400> 27
Leu Ser Asn Gly Asn Arg Thr Leu Thr Leu
1               5                   10


<210> 28
<211> 10
<212> PRT
<213> Homo sapiens

<400> 28
Cys Gln Ala His Asn Ser Asp Thr Gly Leu
1               5                   10


<210> 29
<211> 10
<212> PRT
<213> Homo sapiens

<400> 29
Ile Ile Tyr Pro Asn Ala Ser Leu Leu Ile
1               5                   10


<210> 30
<211> 10
<212> PRT
<213> Homo sapiens

<400> 30
Ser Ala Asn Arg Ser Asp Pro Val Thr Leu
1               5                   10


<210> 31
<211> 10
<212> PRT
<213> Homo sapiens

<400> 31
Ser Val Ser Ala Asn Arg Ser Asp Pro Val
1               5                   10


<210> 32

<211> 10
<212> PRT
<213> Homo sapiens

<400> 32
Ser Ile Ser Ser Asn Asn Ser Lys Pro Val
1               5                   10


<210> 33
<211> 10
<212> PRT
<213> Homo sapiens

<400> 33
Trp Trp Val Asn Asn Gln Ser Leu Pro Val
1               5                   10


<210> 34
<211> 10
<212> PRT
<213> Homo sapiens

<400> 34
Trp Trp Val Asn Asn Gln Ser Leu Pro Val
1               5                   10


<210> 35
<211> 10
<212> PRT
<213> Homo sapiens

<400> 35
Ser Ile Ser Ser Asn Asn Ser Lys Pro Val
1               5                   10


<210> 36
<211> 10
<212> PRT
<213> Homo sapiens

<400> 36
Ser Asp Leu Val Asn Glu Glu Ala Thr Gly
1               5                   10


<210> 37
<211> 10
<212> PRT
<213> Homo sapiens

<400> 37
Ile Thr Pro Asn Asn Asn Gly Thr Tyr Ala
1               5                   10


<210> 38
<211> 10

<212> PRT
<213> Homo sapiens

<400> 38
Ser Leu Pro Val Ser Pro Arg Leu Gln Leu
1               5                   10


<210> 39
<211> 10
<212> PRT
<213> Homo sapiens

<400> 39
Ser Leu Pro Val Ser Pro Arg Leu Gln Leu
1               5                   10


<210> 40
<211> 10
<212> PRT
<213> Homo sapiens

<400> 40
Phe Ile Thr Ser Asn Asn Ser Asn Pro Val
1               5                   10


<210> 41
<211> 10
<212> PRT
<213> Homo sapiens

<400> 41
Arg Ser Gly Glu Asn Leu Asn Leu Ser Cys
1               5                   10


<210> 42
<211> 10
<212> PRT
<213> Homo sapiens

<400> 42
Ser Leu Pro Val Ser Pro Arg Leu Gln Leu
1               5                   10


<210> 43
<211> 10
<212> PRT
<213> Homo sapiens

<400> 43
Val Tyr Pro Glu Leu Pro Lys Pro Ser Ile
1               5                   10


<210> 44
<211> 10
<212> PRT

<213> Homo sapiens

<400> 44
Ser Thr Pro Phe Asn Val Ala Glu Gly Lys
1               5                   10


<210> 45
<211> 10
<212> PRT
<213> Homo sapiens

<400> 45
Asn Pro Pro Thr Thr Ala Lys Leu Thr Ile
1               5                   10


<210> 46
<211> 10
<212> PRT
<213> Homo sapiens

<400> 46
Cys Phe Val Ser Asn Leu Ala Thr Gly Arg
1               5                   10


<210> 47
<211> 10
<212> PRT
<213> Homo sapiens

<400> 47
Gly Ala Asn Leu Asn Leu Ser Cys His Ser
1               5                   10


<210> 48
<211> 10
<212> PRT
<213> Homo sapiens

<400> 48
Leu Ser Gly Ala Asn Leu Asn Leu Ser Cys
1               5                   10


<210> 49
<211> 10
<212> PRT
<213> Homo sapiens

<400> 49
Ser Tyr Leu Ser Gly Ala Asn Leu Asn Leu
1               5                   10


<210> 50
<211> 10
<212> PRT
<213> Homo sapiens

<400> 50
Cys Gly Ile Gln Asn Ser Val Ser Ala Asn
1               5                   10


<210> 51
<211> 10
<212> PRT
<213> Homo sapiens

<400> 51
Val Ser Ala Glu Leu Pro Lys Pro Ser Ile
1               5                   10


<210> 52
<211> 10
<212> PRT
<213> Homo sapiens

<400> 52
Ser Asn Ile Thr Glu Lys Asn Ser Gly Leu
1               5                   10


<210> 53
<211> 10
<212> PRT
<213> Homo sapiens

<400> 53
Gly Asn Ile Gln Gln His Thr Gln Glu Leu
1               5                   10


<210> 54
<211> 10
<212> PRT
<213> Homo sapiens

<400> 54
Ser Asn Pro Pro Ala Gln Tyr Ser Trp Leu
1               5                   10


<210> 55
<211> 10
<212> PRT
<213> Homo sapiens

<400> 55
Cys Gly Ile Gln Asn Glu Leu Ser Val Asp
1               5                   10


<210> 56
<211> 10
<212> PRT
<213> Homo sapiens

<400> 56
Ile Ser Pro Leu Asn Thr Ser Tyr Arg Ser
1               5                   10


<210> 57
<211> 10
<212> PRT
<213> Homo sapiens

<400> 57
Pro Gly Pro Ala Tyr Ser Gly Arg Glu Ile
1               5                   10


<210> 58
<211> 10
<212> PRT
<213> Homo sapiens

<400> 58
Phe Asn Val Ala Glu Gly Lys Glu Val Leu
1               5                   10


<210> 59
<211> 10
<212> PRT
<213> Homo sapiens

<400> 59
Ile Gly Val Leu Val Gly Val Ala Leu Ile
1               5                   10


<210> 60
<211> 10
<212> PRT
<213> Homo sapiens

<400> 60
Ser Ala Gly Ala Thr Val Gly Ile Met Ile
1               5                   10


<210> 61
<211> 10
<212> PRT
<213> Homo sapiens

<400> 61
Ser Lys Pro Val Glu Asp Lys Asp Ala Val
1               5                   10


<210> 62
<211> 10
<212> PRT
<213> Homo sapiens

<400> 62

Ser Ala Ser Gly His Ser Arg Thr Thr Val
1               5                   10

<210> 63
<211> 10
<212> PRT
<213> Homo sapiens

<400> 63
Arg Pro Gly Val Asn Leu Ser Leu Ser Cys
1               5                   10

<210> 64
<211> 10
<212> PRT
<213> Homo sapiens

<400> 64
Leu Ser Val Asp His Ser Asp Pro Val Ile
1               5                   10

<210> 65
<211> 10
<212> PRT
<213> Homo sapiens

<400> 65
Ser Asn Asp Asn Arg Thr Leu Thr Leu Leu
1               5                   10

<210> 66
<211> 10
<212> PRT
<213> Homo sapiens

<400> 66
Ser Asn Pro Val Glu Asp Glu Asp Ala Val
1               5                   10

<210> 67
<211> 10
<212> PRT
<213> Homo sapiens

<400> 67
Ser Asn Asn Ser Asn Pro Val Glu Asp Glu
1               5                   10

<210> 68
<211> 10
<212> PRT
<213> Homo sapiens

<400> 68
Gly Leu Asn Arg Thr Thr Val Thr Thr Ile

1               5                   10


<210> 69
<211> 10
<212> PRT
<213> Homo sapiens

<400> 69
Ile Thr Val Asn Asn Ser Gly Ser Tyr Thr
1               5                   10


<210> 70
<211> 10
<212> PRT
<213> Homo sapiens

<400> 70
Ser Thr Gln Glu Leu Phe Ile Pro Asn Ile
1               5                   10


<210> 71
<211> 10
<212> PRT
<213> Homo sapiens

<400> 71
Phe Gln Gln Ser Thr Gln Glu Leu Phe Ile
1               5                   10


<210> 72
<211> 10
<212> PRT
<213> Homo sapiens

<400> 72
Ser Ala Arg Arg Ser Asp Ser Val Ile Leu
1               5                   10


<210> 73
<211> 10
<212> PRT
<213> Homo sapiens

<400> 73
Val Ser Ala Arg Arg Ser Asp Ser Val Ile
1               5                   10


<210> 74
<211> 10
<212> PRT
<213> Homo sapiens

<400> 74
Ser Lys Pro Val Glu Asp Lys Asp Ala Val
1               5                   10

<210> 75
<211> 10
<212> PRT
<213> Homo sapiens

<400> 75
Thr Gly Gln Phe Arg Val Tyr Pro Glu Leu
1               5                   10


<210> 76
<211> 10
<212> PRT
<213> Homo sapiens

<400> 76
Lys Glu Val Leu Leu Leu Val His Asn Leu
1               5                   10


<210> 77
<211> 10
<212> PRT
<213> Homo sapiens

<400> 77
Ala Lys Leu Thr Ile Glu Ser Thr Pro Phe
1               5                   10


<210> 78
<211> 10
<212> PRT
<213> Homo sapiens

<400> 78
Phe Leu Pro Trp His Arg Leu Phe Leu Leu
1               5                   10


<210> 79
<211> 10
<212> PRT
<213> Homo sapiens

<400> 79
Phe Leu Pro Ser Asp Tyr Phe Pro Ser Val
1               5                   10


<210> 80
<211> 9
<212> PRT
<213> Homo sapiens

<400> 80
Ala Glu Met Gly Lys Tyr Ser Phe Tyr
1               5

<210> 81
<211> 72
<212> DNA
<213> SV40

<220>
<223> Synthetic Nucleotide Sequence

<400> 81
tggttgctga ctaattgaga tgcatgcttt gcatacttct gcctgctggg gagcctgggg 60
actttccaca cc 72

<210> 82
<211> 6
<212> DNA
<213> Artificial Sequence

<220>
<223> Synthetic Nucleotide Sequence

<400> 82
aacgtt 6

## Description

### Background of the Invention

### Field of the Invention

The invention disclosed herein relates to methods and compositions for inducing an antigen presenting cell to present a particular target cell-specific epitope, thereby promoting an effective cytotoxic T cell response to the target cell.

The invention further relates to the identification of target cell epitopes and epitope clusters, and also to epitope-encoding vectors, that can be used to generate immunologically active pharmaceutical compositions. These compositions, when administered, can stimulate the immune system of a subject to mount an immune response against a target cell displaying the target antigen. The invention is therefore useful in the treatment and prevention of neoplastic and viral disease.

### Description of the Related Art

### Neoplasia and the Immune System

The neoplastic disease state commonly known as cancer is thought to generally result from a single cell growing out of control. The uncontrolled growth state typically results from a multistep process in which a series of cellular systems fail, resulting in the genesis of a neoplastic cell. The resulting neoplastic cell rapidly reproduces itself, forms one or more tumors, and eventually may cause the death of the host.

Because the progenitor of the neoplastic cell shares the host's genetic material, neoplastic cells are largely exempt from the host's immune system. During immune surveillance, the process in which the host's immune system surveys and localizes foreign materials, a neoplastic cell will appear to the host's immune surveillance machinery as a "self" cell.

### Viruses and the Immune System

In contrast to cancer cells, virus infection involves the expression of clearly non-self antigens. As a result, many virus infections are successfully dealt with by the immune system with minimal clinical sequela. Moreover, it has been possible to develop effective vaccines for many of those infections that do cause serious disease. A variety of vaccine approaches have been successfully used to combat various diseases. These approaches include subunit vaccines consisting of individual proteins produced through recombinant DNA technology. Notwithstanding these advances, the selection and effective administration of minimal epitopes for use as viral vaccines has remained problematic.

In addition to the difficulties involved in epitope selection stands the problem of viruses that have evolved the capability of evading a host's immune system. Many viruses, especially viruses that establish persistent infections, such as members of the herpes and pox virus families, produce immunomodulatory molecules that permit the virus to evade the host's immune system. The effects of these immunomodulatory molecules on antigen presentation may be overcome by the targeting of select epitopes for administration as immunogenic compositions. To better understand the interaction of neoplastic cells and virally infected cells with the host's immune system, a discussion of the system's components follows below.

The immune system functions to discriminate molecules endogenous to an organism ("self" molecules) from material exogenous or foreign to the organism ("non-self" molecules). The immune system has two types of adaptive responses to foreign bodies based on the components that mediate the response: a humoral response and a cell-mediated response. The humoral response is mediated by antibodies, while the cell-mediated response involves cells classified as lymphocytes. Recent anticancer and antiviral strategies have focused on mobilizing the host immune system as a means of anticancer or antiviral treatment or therapy.

The immune system functions in three phases to protect the host from foreign bodies: the cognitive phase, the activation phase, and the effector phase. In the cognitive phase, the immune system recognizes and signals the presence of a foreign antigen or invader in the body. The foreign antigen can be, for example, a cell surface marker from a neoplastic cell or a viral protein. Once the system is aware of an invading body, antigen specific cells of the immune system proliferate and differentiate in response to the invader-triggered signals. The last stage is the effector stage in which the effector cells of the immune system respond to and neutralize the detected invader.

An array of effector cells implement an immune response to an invader. One type of effector cell, the B cell, generates antibodies targeted against foreign antigens encountered by the host. In combination with the complement system, antibodies direct the destruction of cells or organisms bearing the targeted antigen. Another type of effector cell is the natural killer cell (NK cell), a type of lymphocyte having the capacity to spontaneously recognize and destroy a variety of virus infected cells as well as malignant cell types. The method used by NK cells to recognize target cells is poorly understood.

Another type of effector cell, the T cell, has members classified into three subcategories, each playing a different role in the immune response. Helper T cells secrete cytokines which stimulate the proliferation of other cells necessary for mounting an effective immune response, while suppressor T cells down-regulate the immune response. A third category of T cell, the cytotoxic T cell (CTL), is capable of directly lysing a targeted cell presenting a foreign antigen on its surface.

### The Major Histocompatibility Complex and T Cell Target Recognition

T cells are antigen specific immune cells that function in response to specific antigen signals. B lymphocytes and the antibodies they produce are also antigen specific entities. However, unlike B lymphocytes, T cells do not respond to antigens in a free or soluble form. For a T cell to respond to an antigen, it requires the antigen to be bound to a presenting complex known as the major histocompatibilitycomplex (MHC).

MHC complex proteins provide the means by which T cells differentiate native or "self' cells from foreign cells. There are two types of MHC, class I MHC and class II MHC. T Helper cells (CD4⁺) predominately interact with class II MHC proteins while cytolytic T cells (CD8⁺) predominately interact with class I MHC proteins. Both MHC complexes are transmembrane proteins with a majority of their structure on the external surface of the cell. Additionally, both classes of MHC have a peptide binding cleft on their external portions. It is in this cleft that small fragments of proteins, native or foreign, are bound and presented to the extracellular environment.

Cells called antigen presenting cells (APCs) display antigens to T cells using the MHC complexes. For T cells to recognize an antigen, it must be presented on the MHC complex for recognition. This requirement is called MHC restriction and it is the mechanism by which T cells differentiate "self" from "non-self" cells. If an antigen is not displayed by a recognizable MHC complex, the T cell will not recognize and act on the antigen signal. T cells specific for the peptide bound to a recognizable MHC complex bind to these MHC-peptide complexes and proceed to the next stages of the immune response.

As discussed above, neoplastic cells are largely ignored by the immune system. A great deal of effort is now being expended in an attempt to harness a host's immune system to aid in combating the presence of neoplastic cells in a host. One such area of research involves the formulation of anticancer vaccines.

### Anticancer Vaccines

Among the various weapons available to an oncologist in the battle against cancer is the immune system of the patient. Work has been done in various attempts to cause the immune system to combat cancer or neoplastic diseases. Unfortunately, the results to date have been largely disappointing. One area of particular interest involves the generation and use of anticancer vaccines.

To generate a vaccine or other immunogenic composition, it is necessary to introduce to a subject an antigen or epitope against which an immune response may be mounted. Although neoplastic cells are derived from and therefore are substantially identical to normal cells on a genetic level, many neoplastic cells are known to present tumor-associated antigens (TuAAs). In theory, these antigens could be used by a subject's immune system to recognize these antigens and attack the neoplastic cells. Unfortunately, neoplastic cells appear to be ignored by the host's immune system.

A number of different strategies have been developed in an attempt to generate vaccines with activity against neoplastic cells. These strategies include the use of tumor associated antigens as immunogens. For example, U.S. Patent No. 5,993,828, describes a method for producing an immune response against a particular subunit of the Urinary Tumor Associated Antigen by administering to a subject an effective dose of a composition comprising inactivated tumor cells having the Urinary Tumor Associated Antigen on the cell surface and at least one tumor associated antigen selected from the group consisting of GM-2, GD-2, Fetal Antigen and Melanoma Associated Antigen. Accordingly, this patent describes using whole, inactivated tumor cells as the immunogen in an anticancer vaccine.

Another strategy used with anticancer vaccines involves administering a composition containing isolated tumor antigens. In one approach, MAGE-Al antigenic peptides were used as an immunogen. (See Chaux, P., et al., "Identification of Five MAGE-Al Epitopes Recognized by Cytolytic T Lymphocytes Obtained by In Vitro Stimulation with Dendritic Cells Transduced with MAGE-A1," J. Immunol., 163(5):2928-2936 (1999)). There have been several therapeutic trials using MAGE-Al peptides for vaccination, although the effectiveness of the vaccination regimes was limited. The results of some of these trials are discussed in Vose, J.M., "Tumor Antigens Recognized by T Lymphocytes," 10th European Cancer Conference, Day 2, Sept. 14, 1999.

In another example of tumor associated antigens used as vaccines, Scheinberg, *et al.* treated 12 chronic myelogenous leukemia (CML) patients already receiving interferon (IFN) or hydroxyurea with 5 injections of class I-associated bcr-abl peptides with a helper peptide plus the adjuvant QS-21. Scheinberg, D.A., et al., "BCR-ABL Breakpoint Derived Oncogene Fusion Peptide Vaccines Generate Specific Immune Responses in Patients with Chronic Myelogenous Leukemia (CML) [Abstract 1665], American Society of Clinical Oncology 35th Annual Meeting, Atlanta (1999). Proliferative and delayed type hypersensitivity (DTH) T cell responses indicative of T-helper activity were elicited, but no cytolytic killer T cell activity was observed within the fresh blood samples.

Additional examples of attempts to identify TAAs for use as vaccines are seen in the recent work of Cebon, *et al.* and Scheibenbogen, *et al.* Cebon *et al.* Immunized patients with metastatic melanoma using intradermallly administered MART-1₂₆₋₃₅ peptide with IL-12 in increasing doses given either subcutaneously or intravenously. Of the first 15 patients, 1 complete remission, 1 partial remission, and 1 mixed response were noted. Immune assays for T cell generation included DTH, which was seen in patients with or without IL-12. Positive CTL assays were seen in patients with evidence of clinical benefit, but not in patients without tumor regression. Cebon, et al., "Phase I Studies of Immunization with Melan-A and IL-12 in HLA A2+ Positive Patients with Stage III and IV Malignant Melanoma," [Abstract 1671], American Society of Clinical Oncology 35th Annual Meeting, Atlanta (1999).

Scheibenbogen, *et al.* immunized 18 patients with 4 HLA class I restricted tyrosinase peptides, 16 with metastatic melanoma and 2 adjuvant patients. Scheibenbogen, et al., "Vaccination with Tyrosinase peptides and GM-CSF in Metastatic Melanoma: a Phase II Trial," [Abstract 1680], American Society of Clinical Oncology 35th Annual Meeting, Atlanta (1999). Increased CTL activity was observed in 4/15 patients, 2 adjuvant patients, and 2 patients with evidence of tumor regression. As in the trial by Cebon *et al.,* patients with progressive disease did not show boosted immunity. In spite of the various efforts expended to date to generate efficacious anticancer vaccines, no such composition has yet been developed.

Vaccine strategies to protect against viral diseases have had many successes. Perhaps the most notable of these is the progress that has been made against the disease small pox, which has been driven to extinction. The success of the polio vaccine is of a similar magnitude.

Viral vaccines can be grouped into three classifications: live attenuated virus vaccines, such as vaccinia for small pox, the Sabin poliovirus vaccine, and measles mumps and rubella; whole killed or inactivated virus vaccines, such as the Salk poliovirus vaccine, hepatitis A virus vaccine and the typical influenza virus vaccines; and subunit vaccines, such as hepatitis B. Due to their lack of a complete viral genome, subunit vaccines offer a greater degree of safety than those based on whole viruses.

The paradigm of a successful subunit vaccine is the recombinant hepatitis B vaccine based on the viruses envelope protein. Despite much academic interest in pushing the subunit concept beyond single proteins to individual epitopes the efforts have yet to bear much fruit. Viral vaccine research has also concentrated on the induction of an antibody response although cellular responses also occur. However, many of the subunit formulations are particularly poor at generating a CTL response.

### Summary of the Invention

The present invention is directed to methods and compositions for inducing an antigen presenting cell to present a particular target cell-specific epitope, thereby promoting a prolonged, directed cytotoxic T cell response to the target cell.

### Vaccines Comprising Housekeeping Epitopes

In one aspect of the invention, there is provided a vaccine including a housekeeping epitope derived from an antigen associated with a target cell. Advantageously, the target cell may be a neoplastic cell. The neoplastic cell can be any transformed cell associated with solid tumors or lymphomas such as leukemia, carcinoma, lymphoma, astrocytoma, sarcoma, glioma, retinoblastoma, melanoma, Wilm's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, and brain cancer. Alternatively, the target cell can be infected by an intracellular parasite. For example, the intracellular parasite may be a virus such as an adenovirus, cytomegalovirus, Epstein-Barr virus, herpes simplex virus, human herpesvirus 6, varicella-zoster virus, hepatitis viruses, papilloma virus, parvovirus, polyomavirus, measles virus, rubella virus, human immunodeficiency virus (HIV), or human T cell leukemia virus. The intracellular parasite may be a bacterium, protozoan, fungus, or a prion. More particularly, the intracellular parasite can be *Chlamydia, Listeria, Salmonella, Legionella, Brucella, Coxiella, Rickettsia, Mycobacterium, Leishmania, Trypanasoma, Toxoplasma,* and *Plasmodium.*

The housekeeping epitope can be derived from an antigen associated with the target cell. The antigen can be MelanA (MART-I), gp100 (Pmel 17), tyrosinase, TRP-1, TRP-2, MAGE-1, MAGE-3, BAGE, GAGE-1, GAGE-2, CEA, RAGE, NY-ESO, SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CAM 17.1, NuMa, K-ras, β-Catenin, CDK4, Mum-1, p16, TAGE, PSMA, PSCA, CT7, telomerase, 43-9F, 5T4, 791Tgp72, alpha-fetoprotein , β-HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, GA733-2\KSA and the like. Optionally, the antigen can be a virus-associated antigen. In another aspect of the invention, the antigen can be a parasite-associated antigen.

In another aspect of the invention, the housekeeping epitope may include or encode a polypeptide of about 6 to about 23 amino acids in length. Preferably, the polypeptide is 9 or 10 amino acids in length. The polypeptide may be a synthetic polypeptide. Advantageously, the vaccine additionally includes buffers, detergents, surfactants, anti-oxidants, or reducing agents. In yet another aspect of the vaccine, the housekeeping epitope includes a nucleic acid. In a preferred embodiment, the housekeeping epitope is specific for at least one allele of MHC. The allele can encode types A1, A2, A3, A11, A24, A26, A29, B7, B8, B14, B18, B27, B35, B44, B62, B60, or B51.

In yet another aspect of the present invention, the vaccine may include an immune epitope. Optionally, the immune epitope is derived from a second antigen associated with the target cell. The first antigen and the second antigen may be the same or different. Advantageously, the housekeeping epitope is specific for a first allele of MHC, and the immune epitope is specific for a second allele of MHC. The first allele and second allele may be the same or different.

In still another aspect of the invention, the vaccine includes an epitope cluster (see below) that includes the immune epitope. The epitope cluster can be derived from a second antigen associated with the target cell. The first antigen and the second antigen may be the same or different. Advantageously, the epitope cluster includes or encodes a polypeptide having a length of at least 10 amino acids but less than about 60 amino acids. Preferably, the length of the polypeptide of the epitope cluster is less than about 80%, 50%, or 20% of the length of the second antigen.

In another aspect of the invention, the vaccine further includes a second housekeeping epitope derived from a second antigen associated with a second target cell. Optionally, the first antigen and the second antigen can be the same. Alternatively, the first and second antigen are different. Similarly, the first and second target cell may be the same or different.

The vaccine of the present invention may advantageously include a nucleic acid construct that encodes a housekeeping epitope derived from an antigen associated with a target cell. Preferably, the target cell is a neoplastic cell. The neoplastic cell can be any transformed cell associated with solid tumors or lymphomas such as leukemia, carcinoma, lymphoma, astrocytoma, sarcoma, glioma, retinoblastoma, melanoma, Wilm's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, and brain cancer. In contrast, the target cell can be a cell infected by an intracellular parasite. The intracellular parasite may be a virus. In particular, the virus may be an adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, or human T cell leukemia virus II. Optionally, the intracellular parasite is a bacterium, protozoan, fungus, or prion. More particularly, the intracellular parasite can be *Chlamydia, Listeria, Salmonella, Legionella, Brucella, Coxiella, Rickettsia, Mycobacterium, Leishmania, Trypanasoma, Toxoplasma,* and *Plasmodium.*

The antigen of the vaccine including a nucleic acid construct may be MelanA (MART-I), gp100 (Pmel 17), tyrosinase, TRP-1, TRP-2, MAGE-1, MAGE-3, BAGE, GAGE-1, GAGE-2, CEA, RAGE, NY-ESO, SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CAM 17.1, NuMa, K-ras, β-Catenin, CDK4, Mum-1, and p16. Alternatively, the antigen can be an antigen associated with a virus or viral infection. In still another embodiment, the antigen is an antigen associated with non-viral intracellular parasites.

The housekeeping epitope preferably encodes a polypeptide of about 6 to about 23 amino acids in length. More preferably, the housekeeping epitope encodes a polypeptide of 9 to 10 amino acids in length. Advantageously, the housekeeping epitope is specific for at least one allele of MHC. The allele can encode type A1, A2, A3, A11, A24, A26, A29, B7, B8, B14, B18, B27, B35, B44, B62, B60, or B51.

In another aspect of the invention, the vaccine includes an immune epitope. The immune epitope may be derived from a second antigen associated with the target cell. The first antigen and second antigen may be the same or different. Preferably, the housekeeping epitope is specific for a first allele of MHC and the immune epitope is specific for a second allele of MHC. The first allele and the second allele may be the same or different.

In still another aspect of the present invention, the vaccine with a nucleic acid construct additionally includes an epitope cluster. The epitope cluster includes an immune epitope. Preferably, the epitope cluster is derived from a second antigen associated with the target cell. The first antigen and the second antigen may be the same or different.

Advantageously, the epitope cluster includes or encodes a polypeptide having a length of at least 10 amino acids and less than about 60 amino acids. In a preferred embodiment, the epitope cluster includes or encodes a polypeptide with a length less than about 80% of the length of the second antigen. In another preferred embodiment, the length of the polypeptide is less than about 50 % of the length of the second antigen. In a particularly preferred embodiment, the length of the polypeptide is less than about 20% of the length of the second antigen.

In yet another aspect of the present invention, the vaccine including a nucleic acid construct further includes a second housekeeping epitope, wherein the second housekeeping epitope is derived from a second antigen associated with a second target cell. The first antigen and the second antigen can be the same or different. Preferably, the first target cell and the second target cell are different.

### Nucleic Acid Constructs

The invention provides a nucleic acid construct including a first coding region, wherein the first coding region includes a first sequence encoding at least a first polypeptide, wherein the first polypeptide includes a first housekeeping epitope derived from a first antigen associated with a first target cell. The first coding region can further include a second sequence encoding at least a second polypeptide, wherein the second polypeptide includes an second epitope derived from a second antigen associated with a second target cell. The first polypeptide and the second polypeptide can contiguous or non-contiguous. The second epitope can be a housekeeping epitope or an immune epitope. The first antigen and the second antigen can be the same or different; likewise, the first and second target cells can be the same or different. The target cell can be a neoplastic cell, such as, for example, leukemia, carcinoma, lymphoma, astrocytoma, sarcoma, glioma, retinoblastoma, melanoma, Wihn's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, or brain cancer. The first antigen can be, for example, MART-1/MelanA, gp100 (Pmel 17), tyrosinase, TRP-1, TRP-2, MAGE-1, MAGE-3, BAGE, GAGE-1, GAGE-2, p15, NY-ESO, products of an SSX gene family member, CT-7, and products of an SCP gene family member. The target cell can be infected by a virus such as, for example, adenovirus, cytomegalovirus, Epstein-Barr virus, herpes simplex virus 1 and 2, human herpesvirus 6, varicella-zoster virus, hepatitis B virus, hepatitis D virus, papillomavirus, parvovirus B 19, polyomavirus BK, polyomavirus JC, hepatitis C virus, measles virus, rubella virus, human immunodeficiency virus (HIV), human T-cell leukemia virus I, or human T-cell leukemia virus II. The target cell can likewise be infected by a bacterium, a protozoan, a fungus, a prion, or any other intracellular parasite, examples of which are *Chlamydia, Listeria, Salmonella, Legionella, Brucella, Coxiella, Rickettsia, Mycobacterium, Leishmania, Trypanasoma, Toxoplasma,* and *Plasmodium.*

The construct typically includes a first promoter sequence operably linked to the first coding region. The promoter can be, for example, cytomegalovirus (CMV), SV40 and retroviral long terminal repeat (LTR). The promoter can be a bidirectional promoter, and/or a second promoter sequence can be operably linked to a second coding region. The nucleic acid construct can further include a poly-A sequence operably linked to the first coding region, the second coding region, or both. The nucleic acid construct can also include an internal ribosome entry site (IRES) sequence, a ubiquitin sequence, an autocatalytic peptide sequence, enhancers, nuclear import sequences, immunostimulatory sequences, and expression cassettes for cytokines, selection markers, reporter molecules, and the like. The first polypeptide can be about 7 to 15 amino acids in length, and is preferably 9 or 10 amino acids in length. The second polypeptide can be 9 or 10 amino acids in length, or it can be an epitope cluster between about 10 and about 75 amino acids in length. The first epitope and second epitopes can bind the same or different alleles of MHC.

Other embodiments of the invention include a vaccine that includes any of the foregoing nucleic acid construct embodiments; a method of treating an animal by administering such a vaccine; and a method of making the vaccine.

### Identification of Epitope Clusters

Other embodiments of the invention disclosed herein relate to the identification of epitope cluster regions that are used to generate pharmaceutical compositions capable of inducing an immune response from a subject to whom the compositions have been administered. One embodiment of the disclosed invention relates to an epitope cluster, the cluster being derived from an antigen associated with a target, the cluster including or encoding at least two sequences having a known or predicted affinity for an MHC receptor peptide binding cleft, wherein the cluster is a fragment of the antigen.

In one aspect of the invention, the target is a neoplastic cell. Alternatively, the target may be a cell infected by an intracellular parasite. The intracellular parasite can be a virus, a bacterium or a protozoan. Optionally, the target is a pathogenic agent. The pathogenic agent can include a virus, a bacterium, a fungus, a protozoan, a prion, a toxin, or a venom.

In another aspect of the invention, the MHC receptor may be a class I HLA receptor. Similarly, the MHC receptor can be a class II HLA receptor.

In yet another aspect of the invention, the cluster includes or encodes a polypeptide having a length, wherein the length is at least 10 amino acids. Advantageously, the length of the polypeptide may be less than about 75 amino acids.

In still another aspect of the invention, there is provided an antigen having a length, wherein the cluster consists of or encodes a polypeptide having a length, wherein the length of the polypeptide is less than about 80% of the length of the antigen. Preferably, the length of the polypeptide is less than about 50% of the length of the antigen. Most preferably, the length of the polypeptide is less than about 20% of the length of the antigen.

Another embodiment of the disclosed invention relates to a method of identifying an epitope cluster including the steps of: providing a sequence of an antigen associated with a target cell; scoring candidate peptides within the sequence, based on known or predicted affinity for an MHC receptor peptide binding cleft to identify putative MHC epitopes; and identifying a region within the antigen, wherein the region includes at least two of the putative MHC epitopes, and wherein the region comprises a higher density of putative MHC epitopes than a density of putative MHC epitopes in the antigen as a whole.

### Methods of Treatment

A method of treating an animal by administering to an animal a vaccine including a first housekeeping epitope, wherein the housekeeping epitope is derived from a first antigen associated with a first target cell is similarly contemplated by the present invention. Preferably, the administering step includes a mode of delivery that is transdermal, intranodal, perinodal, oral, intravenous, intradermal, intramuscular, intraperitoneal, or mucosal.

The method of treating an animal may additionally include an assaying step to determine a characteristic indicative of a state of the target cells. Advantageously, the assaying step may further include a first assaying step and a second assaying step, wherein the first assaying step precedes the administering step, and the second assaying step follows the administering step. Preferably, the characteristic determined in the first assaying step is compared with the characteristic determined in the second assaying step to obtain a result. The result can be a diminution in number of target cells, a loss of mass or size of a tumor comprising target cells, or a decrease in number or concentration of an intracellular parasite infecting target cells.

Preferably, the target cell is a neoplastic cell. The neoplastic cell can be any transformed cell associated with solid tumors or lymphomas such as leukemia, carcinoma, lymphoma, astrocytoma, sarcoma, glioma, retinoblastoma, melanoma, Wilm's tumor, bladder cancer, breast cancer, colon cancer, hepatocellular cancer, pancreatic cancer, prostate cancer, Lung cancer, liver cancer, stomach cancer, cervical cancer, testicular cancer, renal cell cancer, and brain cancer. Alternatively, the target cell is infected by an intracellular parasite. The intracellular parasite may be a virus. The virus can be adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, or human T cell leukemia virus II. The intracellular parasite may be a bacterium, protozoan, fungus, or a prion. Advantageously, the intracellular parasite is *Chlamydia, Listeria, Salmonella, Legionella, Brucella, Coxiella, Rickettsia, Mycobacterium, Leishmania, Trypanasoma, Toxoplasma,* and *Plasmodium.*

In another aspect of the invention, the antigen is MelanA (MART-I), gp100 (Pmel 17), tyrosinase, TRP-1, TRP-2, MAGE-1, MAGE-3, BAGE, GAGE-1, GAGE-2, CEA, RAGE, NY-ESO, SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CAM 17.1, NuMa, K-ras, β-Catenin, CDK4, Mum-1, and p16. Alternatively, the antigen is associated with a virus or viral infection. In still another aspect, the antigen is an antigen associated with non-viral intracellular parasites.

The housekeeping epitope may include or encode for a polypeptide of about 6 to about 23 amino acids in length. Preferably, the polypeptide is 9 or 10 amino acids in length. The polypeptide may be synthetic. The vaccine may additionally include buffers, detergents, surfactants, anti-oxidants, or reducing agents. The housekeeping epitope may advantageously include a nucleic acid. Preferably, the housekeeping epitope is specific for at least one allele of MHC. The allele can encode types A1, A2, A3, A11, A24, A26, A29, B7, B8, B14, B18, B27, B35, B44, B62, B60, or B51.

In yet another aspect of the invention, the method of treating an animal further includes an immune epitope. The immune epitope may be derived from a second antigen associated with the target cell. Optionally, the first antigen and the second antigen are the same. The housekeeping epitope can be specific for a first allele of MHC, and the immune epitope can be specific for a second allele of MHC. The first allele and the second allele may be the same or different.

Advantageously, the vaccine includes an epitope cluster that includes the immune epitope. The epitope cluster may be derived from a second antigen associated with the target cell. Optionally, the first antigen and the second antigen are the same. The epitope cluster may include or encode a polypeptide having a length of at least 10 amino acids and less than about 60 amino acids.

Preferably, the epitope cluster includes or encodes a polypeptide having a length less than about 80% of the length of the second antigen. The length of the polypeptide can be less than about 50% of the length of the second antigen. In still another aspect, the length of the polypeptide can be less than about 20% of the length of the second antigen.

The method of treating an animal may further include a second housekeeping epitope, wherein the second housekeeping epitope is derived from a second antigen associated with a second target cell. The first antigen and the second antigen may be the same or different. Similarly, the first target cell and the second target cell may be the same or different.

A method of treating an animal including administering to an animal a vaccine comprising a nucleic acid construct is also contemplated by the present invention. The nucleic acid construct advantageously encodes a housekeeping epitope. The housekeeping epitope may be derived from a first antigen associated with a first target cell.

In another aspect of the invention there is provided a method of making a vaccine. The method includes the steps of selecting a housekeeping epitope by identifying epitopes that are or could be produced from a particular antigen source by housekeeping proteasomes wherein the housekeeping epitope is derived from a first antigen associated with a first target cell, making a vaccine including the housekeeping epitope, and preparing a vaccine composition that includes or encodes the selected housekeeping epitope.

The vaccine made in accordance with the aforementioned method is likewise provided by the present invention. The vaccine can be administered to treat an animal. Thus, a method of treating an animal with the vaccine is similarly contemplated.

### Discovery of Housekeeping and Other Epitopes

Other embodiments of the invention disclosed herein are directed to the identification of epitopes that are useful for generating vaccines capable of inducing an immune response from a subject to whom the compositions have been administered, particularly those epitopes most useful in the vaccine embodiments of the invention. One embodiment of the invention relates to a method of epitope discovery comprising the step of selecting an epitope from a population of peptide fragments of an antigen associated with a target cell, wherein the fragments have a known or predicted affinity for a major histocompatibility complex class I receptor peptide binding cleft, wherein the epitope selected corresponds to a proteasome cleavage product of the target cell.

Another embodiment of the invention relates to a method of discovering an epitope comprising the steps of: providing a sequence from a target cell, wherein the sequence encodes or comprises a protein expressed in the target cell; identifying a population of peptide fragments of the protein, wherein members of the population of peptide fragments have a known or predicted affinity for a major histocompatibility complex class I receptor peptide binding cleft; selecting the epitope from the population of peptide fragments, wherein the epitope corresponds to a product of a proteasome active in the target cell.

One aspect of this embodiment relates an epitope discovered by the aforementioned method. Another aspect of this embodiment relates to a vaccine comprising the discovered epitope. Still another aspect of the invention relates to a method of treating an animal, comprising administering to the animal the aforementioned vaccine.

One embodiment of the disclosed invention relates to a method of epitope discovery comprising the steps of: providing a neoplastic cell and a sequence, wherein the sequence comprises or encodes an antigen associated with the neoplastic cell; identifying a population of peptide fragments of the antigen, wherein the population of peptide fragments is predicted to have an affinity for a major histocompatibility complex class I receptor peptide binding cleft; selecting an epitope from the population of peptide fragments, wherein the epitope is determine by *in vitro* analysis to be a proteasome cleavage reaction product of a proteasome active in the neoplastic cell.

One aspect of this embodiment relates an epitope discovered by the aforementioned method. Another aspect of this embodiment relates to a vaccine comprising the discovered epitope. Still another aspect of the invention relates to a method of treating an animal, comprising administering to the animal the aforementioned vaccine.

Another embodiment of the disclosed invention relates to a method of epitope discovery comprising the step of selecting an epitope from a population of peptide fragments of an antigen associated with a target in a host, wherein the fragments have a known or predicted affinity for a major histocompatibility complex class I or II receptor peptide binding cleft of the host, wherein the epitope selected corresponds to a product of proteolytic cleavage of the antigen in a cell of the host.

One aspect of this embodiment relates an epitope discovered by the aforementioned method. Another aspect of this embodiment relates to a vaccine comprising the discovered epitope. Still another aspect of the invention relates to a method of treating an animal, comprising administering to the animal the aforementioned vaccine.

### Brief Description of the Drawings

Figure 1 depicts schematically the parts of a cell involved in protein processing by the proteasome and epitope presentation.
Figure 2 is a comparison of the housekeeping proteasome and the immune proteasome.
Figure 3 depicts schematically epitope synchronization between infected cells and pAPCs.
Figure 4 shows presentation of different epitopes by pAPCs and tumor cells.
Figure 5 shows presentation of different epitopes by pAPCs and infected cells.
Figure 6 depicts presentation by tumor cells of both housekeeping and immune epitopes due to induction by IFN-gamma.
Figure 7 shows an attack of virally infected cells by T cells induced to recognize a housekeeping epitope.
Figure 8 shows a dual attack against both housekeeping and immune epitopes.
Figure 9 is a depiction of the components of plasmid pVAX-EP1-IRES-EP2-ISS-NIS.
Figure 10 is a depiction of the components of plasmid pVAX-EP2-UB-EP1.
Figure 11 is a depiction of the components of plasmid pVAX-EP2-2A-EP1.
Figure 12 is a depiction of the components of plasmid pVAX-EP1-IRES-EP2.
Figure 13 depicts the results of a flow cytometry assay verifying HLA binding by Melan-A epitopes.
Figure 14 depicts the results of a flow cytometry assay verifying HLA binding by Tyrosinase peptide 207-216.
Figure 15 depicts the sequence of Melan-A (SEQ ID NO: 1), showing clustering of class I HLA epitopes.
Figure 16 depicts the sequence of SSX-2 (SEQ ID NO: 2), showing clustering of class I HLA epitopes.
Figure 17 depicts the sequence ofNY-ESO (SEQ ID NO: 3), showing clustering of class I HLA epitopes.
Figure 18 depicts the sequence of Tyrosinase (SEQ ID NO: 4), showing clustering of class I HLA epitopes predicted by the BIMAS-NIH/Parker algorithm above the line of sequence and by the SYFPEITHI/Rammensee algorithm below.

### Detailed Description of the Preferred Embodiment

Embodiments of the present invention provide epitopes, vaccines, and therapeutic methods for directing an effective immune response against a target cell. A primary basis of the invention is the novel and unexpected discovery that many target cells display epitopes that are different from the epitopes displayed by professional antigen presenting cells (pAPCs). Because of this difference, the pAPCs direct T cells against epitopes that are not present on the target cells, and the T cells therefore fail to recognize the target cells. The methods and medicaments of the present invention can cause pAPCs to display the same epitopes that are present on target cells, resulting in T cells that are correctly able to recognize and destroy the target cells.

Embodiments of the invention disclosed herein further provide methods for identifying epitopes of target antigens that can be used to generate immunologically effective vaccines. Such vaccines can stimulate the immune system to recognize and destroy target cells displaying the selected epitopes. Embodiments of the invention are particularly useful in the treatment and prevention of cancers and of infections of cells by intracellular parasites, as well as in the treatment or prevention of conditions associated with other pathogens, toxins, and allergens.

Certain kinds of targets are particularly elusive to the immune system. Among these are many kinds of cancer, as well as cells infected by intracellular parasites, such as, for example, viruses, bacteria, and protozoans. A great deal of research has been done to identify useful antigens and epitopes for generating an effective immune response against such targets, with little success. This disclosure provides a basis for the efficient discovery of a new generation of effective epitopes effective against such elusive targets.

The invention disclosed herein makes it possible to select epitope sequences with true biological relevance. For an epitope to have biological significance, e.g., to function in stimulating an immune response, it must have an affinity for the binding cleft of a major histocompatibility complex (MHC) receptor peptide. There are various means, known in the art, of predicting whether an oligopeptide sequence will have an MHC binding affinity. However, most of the sequences predicted to have MHC binding affinity are not biologically relevant, because they are not actually presented on the surface of a target cell or a pAPC.

The methods of the disclosed invention permit the vaccine designer to ignore peptides that, despite predicted high binding affinity for MHC, will never be useful because they cannot be presented by target cells. Accordingly, methods and teachings disclosed herein provide a major advance in vaccine design, one that combines the power of antigen sequence analysis with the fundamental realities of immunology. The methods taught herein allow for the simple and effective selection of meaningful epitopes for creation of MHC class I or class II vaccines using any polypeptide sequence corresponding to a desired target.

Further embodiments of the invention disclosed herein provide epitope cluster regions (ECRs) for use in vaccines and in vaccine design and epitope discovery. Specifically, embodiments of the invention relate to identifying epitope clusters for use in generating immunologically active compositions directed against target cell populations, and for use in the discovery of discrete housekeeping epitopes and immune epitopes. In many cases, numerous putative class I MHC epitopes may exist in a single target-associated antigen (TAA). Such putative epitopes are often found in clusters (ECRs), MHC epitopes distributed at a relatively high density within certain regions in the amino acid sequence of the parent TAA. Since these ECRs include multiple putative epitopes with potential useful biological activity in inducing an immune response, they represent an excellent material for *in vitro* or *in vivo* analysis to identify particularly useful epitopes for vaccine design. And, since the epitope clusters can themselves be processed inside a cell to produce active MHC epitopes, the clusters can be used directly in vaccines, with one or more putative epitopes in the cluster actually being processed into an active MHC epitope.

The use of ECRs in vaccines offers important technological advances in the manufacture of recombinant vaccines, and further offers crucial advantages in safety over existing nucleic acid vaccines that encode whole protein sequences. Recombinant vaccines generally rely on expensive and technically challenging production of whole proteins in microbial fermentors. ECRs offer the option of using chemically synthesized polypeptides, greatly simplifying development and manufacture, and obviating a variety of safety concerns. Similarly, the ability to use nucleic acid sequences encoding ECRs, which are typically relatively short regions of an entire sequence, allows the use of synthetic oligonucleotide chemistry processes in the development and manipulation of nucleic acid based vaccines, rather than the more expensive, time consuming, and potentially difficult molecular biology procedures involved with using whole gene sequences.

Since an ECR is encoded by a nucleic acid sequence that is relatively short compared to that which encodes the whole protein from which the ECR is found, this can greatly improve the safety of nucleic acid vaccines. An important issue in the field of nucleic acid vaccines is the fact that the extent of sequence homology of the vaccine with sequences in the animal to which it is administered determines the probability of integration of the vaccine sequence into the genome of the animal. A fundamental safety concern of nucleic acid vaccines is their potential to integrate into genomic sequences, which can cause deregulation of gene expression and tumor transformation. The Food and Drug Administration has advised that nucleic acid and recombinant vaccines should contain as little sequence homology with human sequences as possible. In the case of vaccines delivering tumor-associated antigens, it is inevitable that the vaccines contain nucleic acid sequences that are homologous to those which encode proteins that are expressed in the tumor cells of patients. It is, however, highly desirable to limit the extent of those sequences to that which is minimally essential to facilitate the expression of epitopes for inducing therapeutic immune responses. The use of ECRs thus offers the dual benefit of providing a minimal region of homology, while incorporating multiple epitopes that have potential therapeutic value.

Aspects of the present invention provide nucleic acid constructs that encode a housekeeping epitope. A housekeeping epitope, as will be described in greater detail below, includes peptide fragments produced by the active proteasome of a peripheral cell. A basis for the present invention is the discovery that any antigen associated with a target cell can be processed differentially into two distinguishable sets of epitopes for presentation by the class I major histocompatibility complex (MHC) molecules of the body. "Immune epitopes" are presented by pAPCs and, also generally in peripheral cells that are acutely infected or under active immunological attack by interferon (IFN) secreting cells. In contrast, "housekeeping epitopes" are presented by all other peripheral cells including, generally, neoplastic (cancerous) cells and chronically infected cells. This mismatch, or asynchrony, in presented epitopes underlies the persistence and advance of cancers and chronic infections, despite the presence of a functioning immune system in the host. It is thus essential to bring about synchronization of epitope presentation between the pAPC and the target cell in order to provoke an effective, cytolytic T lymphocyte (CTL)- mediated immune response.

Synchronization can be accomplished most reliably by providing the pAPC with a housekeeping epitope. Often a more robust response can be achieved by providing more than a single epitope. Additionally, once an effective immune response against the target cells has been established, secretion of IFN may lead to expression of the immune proteasome, thereby switching epitope presentation to immune epitopes. For this reason, among others, it can also be advantageous to include immune epitopes, in addition to housekeeping epitopes, in vaccines developed according to the above referenced disclosure. It can be of further utility to provide immune epitopes in the form of an ECR. Embodiments of the invention provide expression vectors encoding housekeeping epitopes and/or immune epitopes in a variety of combinations. Preferred expression constructs encode at least one epitope capable of stimulating a cellular immune response directed against a target cell. In one embodiment of the invention, target cells are neoplastic cells. In another embodiment, target cells are any intracellularly infected host cell. Intracellular infective agents include persistent viruses and any other infectious organism that has an intracellular stage of infection.

The nucleic acid constructs of some embodiments are directed to enhancing a subject's immune system and sensitizing it to the presence of neoplastic cells within the host. In other embodiments, the nucleic acid constructs facilitate the eradication of persistent viral infections as well as cells infected with intracellular parasites.

### Definitions

Unless otherwise clear from the context of the use of a term herein, the following listed terms shall generally have the indicated meanings for purposes of this description.

PROFESSIONAL ANTIGEN-PRESENTING CELL (pAPC) - a cell that possesses T cell costimulatory molecules and is able to induce a T cell response. Well characterized pAPCs are dendritic cells, B cells, and macrophages.

PERIPHERAL CELL - a cell that is not a pAPC.

HOUSEKEEPING PROTEASOME - a proteasome normally active in peripheral cells, and generally not present or not strongly active in pAPCs.

IMMUNE PROTEASOME - a proteasome normally active in pAPCs; the immune proteasome is also active in some peripheral cells in infected tissues.

EPITOPE - a molecule or substance capable of stimulating an immune response. In preferred embodiments, epitopes according to this definition include but are not necessarily limited to a polypeptide and a nucleic acid encoding a polypeptide, wherein the polypeptide is capable of stimulating an immune response. In other preferred embodiments, epitopes according to this definition include but are not necessarily limited to peptides presented on the surface of cells non-covalently bound to the pocket of class I MHC, such that they can interact with T cell receptors.

MHC EPITOPE - a polypeptide having a known or predicted affinity for a mammalian class I major histocompatibility complex (MHC) molecule.

HLA EPITOPE - a polypeptide having a known or predicted affinity for a human class I major histocompatibility complex (MHC) molecule.

HOUSEKEEPING EPITOPE - In a preferred embodiment, a housekeeping epitope is defined as a polypeptide fragment that is an MHC epitope, and that is displayed on a cell in which housekeeping proteasomes are predominantly active. In another preferred embodiment, a housekeeping epitope is defined as a polypeptide containing a housekeeping epitope according to the foregoing definition, that is flanked by one to several additional amino acids. In another preferred embodiment, a housekeeping epitope is defined as a nucleic acid that encodes a housekeeping epitope according to either of the foregoing definitions.

IMMUNE EPITOPE - In a preferred embodiment, an immune epitope is defined as a polypeptide fragment that is an MHC epitope, and that is displayed on a cell in which immune proteasomes are predominantly active. In another preferred embodiment, an immune epitope is defined as a polypeptide containing an immune epitope according to the foregoing definition, that is flanked by one to several additional amino acids. In another preferred embodiment, an immune epitope is defined as a polypeptide including an epitope cluster sequence, having at least two polypeptide sequences having a known or predicted affinity for a class I MHC. In yet another preferred embodiment, an immune epitope is defined as a nucleic acid that encodes an immune epitope according to any of the foregoing definitions.

TARGET CELL - a cell to be targeted by the vaccines and methods of the invention. Examples of target cells according to this definition include but are not necessarily limited to: a neoplastic cell and a cell harboring an intracellular parasite, such as, for example, a virus, a bacterium, or a protozoan.

TARGET-ASSOCIATED ANTIGEN (TAA) - a protein or polypeptide present in a target cell.

TUMOR-ASSOCIATED ANTIGEN (TuAA) - a TAA, wherein the target cell is a neoplastic cell.

Note that the following discussion sets forth the inventors' understanding of the operation of the invention. However, it is not intended that this discussion limit the patent to any particular theory of operation not set forth in the claims.

### Different Proteasomes Yield Different Epitopes

Epitopes presented by class I MHC on the surface of either pAPCs or peripheral cells are produced by digestion of proteins within those cells by proteasomes. While it has been reported that the proteasomes of pAPCs are not identical to the proteasomes of peripheral cells, the significance of this difference has been heretofore unappreciated. This invention is based on the fact that when pAPCs and peripheral cells process a given TAA, the proteasomes active in the pAPCs generate epitope fragments that are different from the epitope fragments generated by the proteasomes that are active in the peripheral cells. For convenience of reference, and as defined above, the proteasomes that are predominantly active in pAPCs are referred to herein as "immune proteasomes" while the proteasomes that are normally active in peripheral cells are referred to herein as "housekeeping proteasomes."

The significance of the differential processing of TAAs by pAPCs and peripheral cells cannot be overstated. This differential processing provides a unified explanation for why certain target cells are resistant to recognition and attack by the immune system. Although pAPCs can take-up TAAs shed from target cells and present them on their surface, the pAPCs will consequently stimulate the production of CTLs to recognize an "immune epitope" (the epitope resulting from processing of the TAA by the immune proteasome), whereas the target cells display "housekeeping epitopes" (distinct fragments of the TAA generated by the housekeeping proteasome). As a consequence, the CTL response under physiological conditions is misdirected away from epitopes on the target cells.

Since CTL responses are induced by pAPCs, by definition they target immune epitopes rather than housekeeping epitopes and thus fail to recognize target cells, which are therefore able to persist in the body. This fundamental "epitope compartmentalization" of the cellular immune response is the reason that some neoplastic cells can persist to form tumors; it is also the reason that some viruses and intracellular parasites can chronically infect cells without being eradicated by the immune system. With regard to infectious agents, normally they cause the expression of immune proteasomes in the cells they infect. This results in the production of epitopes on the cell surface that are identical to those being presented by pAPCs to the immune system. Infection thus results in "epitope synchronization" between the immune system and the infected cell, subsequent destruction of the infected cells, and clearance of infectious agent from the body. In the case of some infectious agents, notably those that are capable of establishing chronic infections, they have evolved a means of preventing expression of immune proteasomes in the cells they infect. The proteasome in these cells are maintained in a housekeeping mode, thereby preventing epitope synchronization and attack by CTL. There is substantial evidence that this is a common mechanism used by virtually all chronic infectious agents.

One way to overcome this failure on the part of CTLs to recognize and eradicate certain target cells is to provide vaccines and treatment methods that are capable of "synchronizing" epitope presentation. Epitope synchronization in this context means that the pAPCs are made to present housekeeping epitopes, resulting in CTLs that can recognize the housekeeping epitopes displayed on target cells, and thereby attack and eliminate the target cells.

Accordingly, embodiments of the invention are useful for treating neoplastic diseases including solid tumors and lymphomas. Additional embodiments of the invention have application in treating persistent viral infections as well as parasitic infections in which the infective agent has an intracellular stage of infection. Appropriate administration of housekeeping epitopes corresponding to such target cells can activate a specific, cytotoxic T cell response against the target cells.

### The Role of Epitope Differences in Cancer

In some embodiments, the present invention is directed to treating neoplastic diseases. Cancers are caused by the progressive, unregulated growth of the progeny of a single abnormal cell. The term "cancer" as used herein includes neoplastic diseases, neoplastic cells, tumors, tumor cells, malignancies and any transformed cell, including both solid tumors and diffuse neoplastic disease. Historically, cancer cells generally have been thought to escape detection and destruction by the immune system because cancer cells contain the same genetic material as other non-cancerous cells of the body. The genetic identity or similarity of cancer cells and healthy cells in the body supposedly causes the difficulty of distinguishing cancer cells from normal cells, and the immune system is therefore unable to mount an effective immune response, as evidenced by the persistence of cancer cells in the body.

To the contrary, a variety of tumor associated antigens (TuAAs) have been described which could, and indeed do, provoke immune responses. Numerous studies have described tumor infiltrating lymphocytes (TILs) which can kill target cells presenting peptides derived from various TuAAs *in vitro.* As is described in further detail below, however, the failure of TILs to control cancer results from a difference in the epitopes produced and presented by the cells which induce CTL activity, the pAPC, and the desired target cells, i.e., those of the tumor. To understand the difference, it is necessary to understand the functions and dynamics of proteasomes.

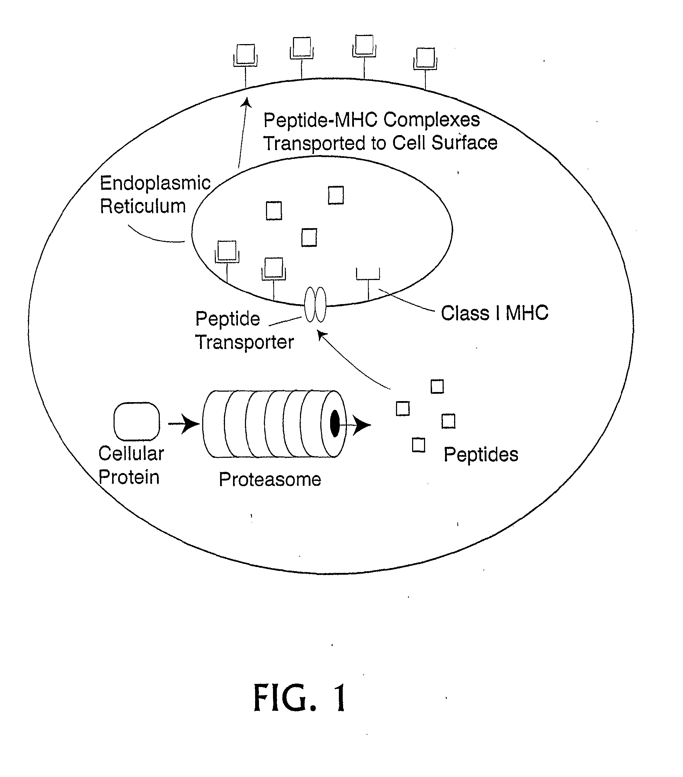

All cells contain proteasomes to degrade proteins. These proteasomes, which comprise about 1 % of the total protein content of the cell, serve to regulate protein half-life in the cell. In the course of protein degradation, proteasomes generate the vast majority of peptide fragments involved in Class I antigen presentation, and the proteasome cleavage patterns affect the availability of antigenic epitopes for presentation on Class I molecules (Figure 1). Thus MHC epitopes are produced by the proteasomal activity of cells. However, the proteolytic activity in pAPCs, as compared to peripheral cells, is markedly different. The pAPCs contain a proteasome that constitutively incorporates subunits that are typically only expressed in peripheral cells during infection or after exposure to various cytokines, particularly interferon (IFN), as part of a cellular immune response. As set forth above, the different proteasomal activities of pAPCs and peripheral cells are referred to herein as immune and housekeeping proteasomes, respectively.

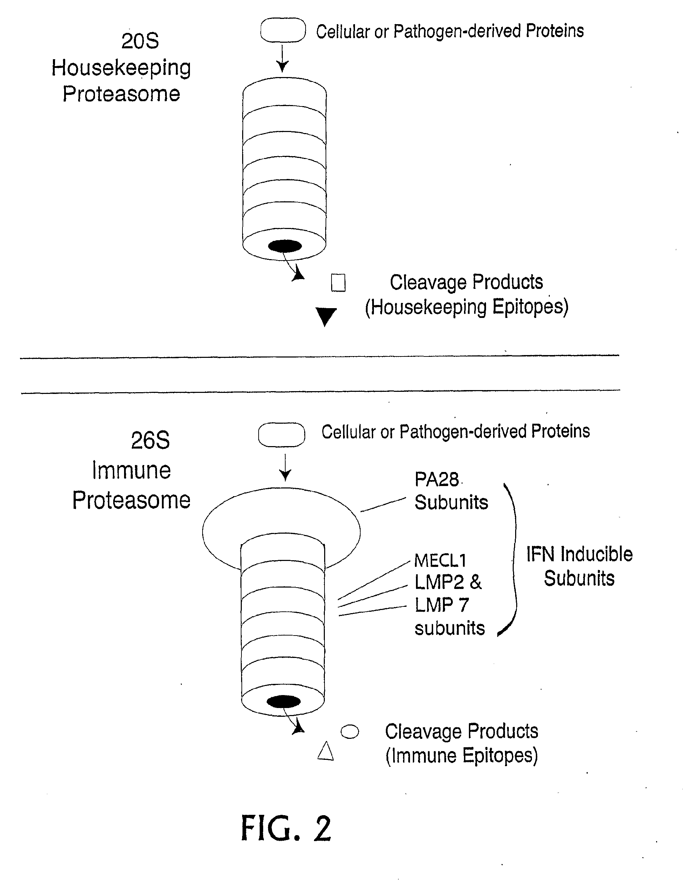

The immune and housekeeping proteasomes have the capacity to cleave proteins at similar but distinct locations. The immune proteasome incorporates several subunits that distinguish it from its housekeeping counterpart. These immune subunits include LMP2, LMP7, and MECL1, which replace the catalytic subunits of the housekeeping proteasome, and PA28α and PA28β, which serve a regulatory function (Figure 2). Collectively, incorporation of these subunits results in activity from the immune proteasome that is qualitatively and quantitatively different from the activity of the housekeeping proteasome. Although evidence has existed that there are differences between housekeeping and immune proteasomes with respect to the MHC epitopes they produce, until now these differences have been rationalized in quantitative terms. It has been suggested by others that the ultimate effect mediated by the immune proteasome is to facilitate the production of more peptides, rather than different ones.

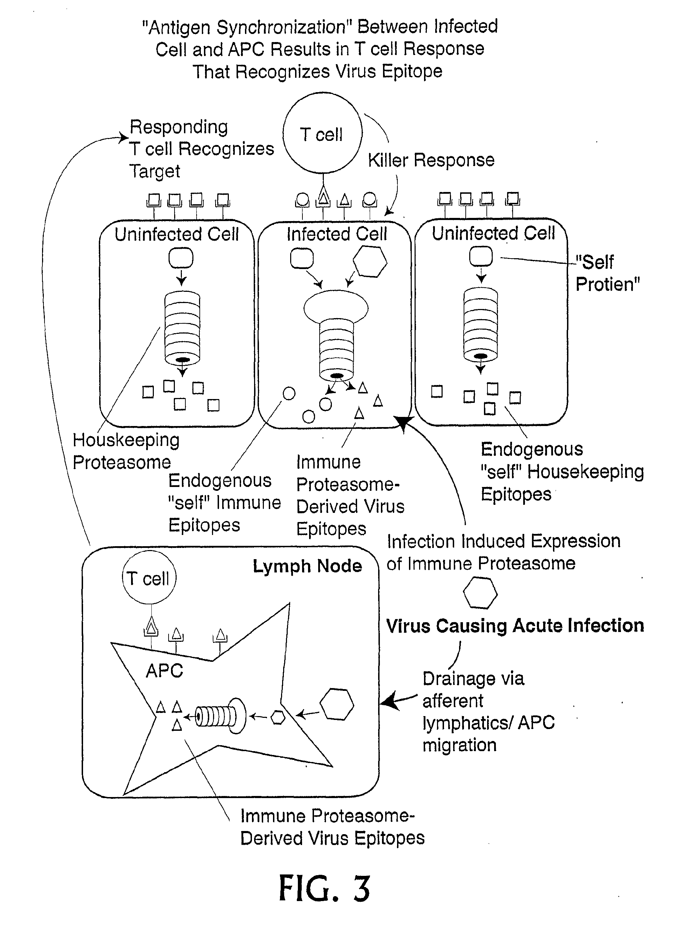

Qualitative differences in antigen processing between immune and housekeeping proteasomes have serious implications for vaccine design. IFN-γ is produced by T lymphocytes, where it is involved in promoting the induction of cellular immune responses and, as noted above, induces expression of the immune proteasome. Notably, IFN is also produced by virtually any other cell under one condition: in the event that the cell becomes infected by a pathogen. In nature, viral infection typically causes IFN production by the infected cell, which in turn induces the cell to convert from a housekeeping proteasome configuration to an immune proteasome configuration. One explanation for this phenomenon is that the infection and subsequent IFN up-regulation serves to align the infected cell, in terms of the displayed antigen repertoire, with that of the pAPCs involved in stimulating the immune response against the virus. This results in the processing of both its endogenous "self" proteins, expressed normally by the cell, and those proteins related to the infectious agent ("non-self") in an identical manner to antigen processing occurring in the pAPCs. The conversion of the infected cell's proteasome from a housekeeping configuration to an immune configuration results in "epitope synchronization" between infected cells and the pAPC. (Figure 3).

MHC class-I-restricted CTLs specific for TuAAs are an important component of the immune response against cancer. TuAAs are useful targets of a tumor-specific T cell response to the extent that they are not displayed on the surface of normal cells, or are overexpressed by the tumor cells, or are otherwise strongly characteristic of tumor cells. Numerous TuAAs are known and are readily available to those of skill in the art in the literature or commercially.

Indeed some tumors have been found to be defective in IFN-γ induction of the immune proteasomes. In these situations, it is likely that the CTL are targeting immune epitopes from TuAAs that have been processed by pAPC. Despite the high numbers of CTL in these patients specifically activated against these immune epitopes, the CTL fail to find the epitope on the cancer cells. The disease progresses and eventually the accumulating CTL, unable to locate the target, become dysfunctional (Lee, et al. Nature Medicine (1999) 5[6]:677-685). By providing the pAPC with housekeeping epitopes, one can synchronize the epitope presentation by pAPCs with the epitope presentation by the tumor, and activate a CTL population that recognizes those housekeeping epitopes present on the tumor.

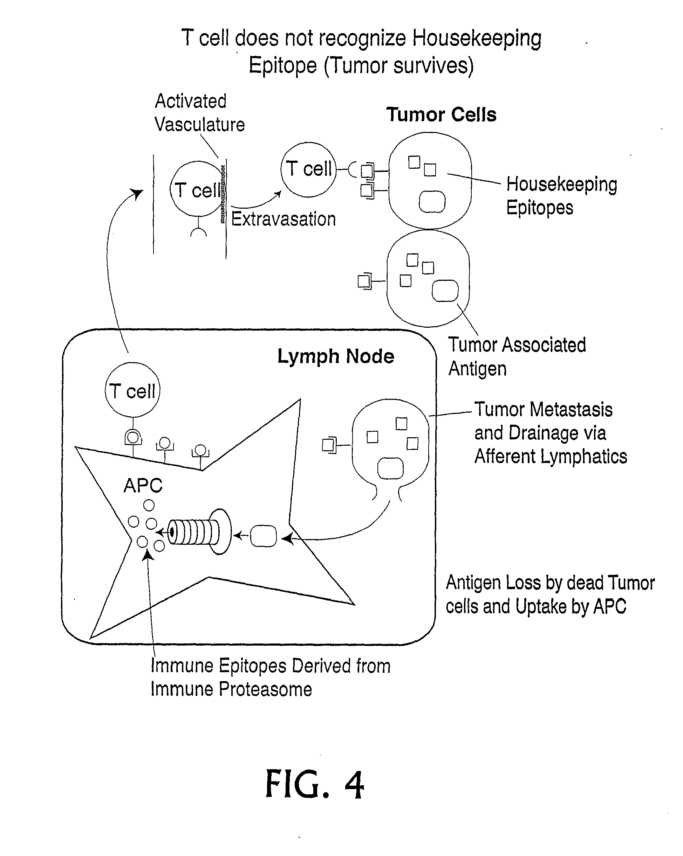

Thus, the discovery that the immune proteasomes in pAPCs produce *qualitatively* different epitopes than do the housekeeping proteasomes in peripheral cells provides an explanation for why TILs do not eradicate tumor cells. The processing mechanism described above explains how T lymphocytes find their way into tumor masses, and yet are relatively ineffective against the tumor cells themselves. Differential antigen processing between the immune proteasome of pAPCs and the housekeeping proteasome of tumor cells can explain the observation of high frequencies of T lymphocytes specific against TuAAs in patients with progressive cancer. Lee, et al. Nature Medicine (1999) 5[6]:677-685. (Figure 4).

Due to differences in proteasome activity, peripheral target cells, including tumor cells, and some cells infected by a virus or other intracellular parasite (all of which express the housekeeping proteasome), necessarily display different epitope signals than the epitope signals that T cells are conditioned by pAPCs to recognize. In view of this discovery, a compelling immunoregulatory role for the proteasome emerges. This discovery provides a key to manipulating the immune system, particularly the pAPCs, in order to induce an effective and lethal cell-mediated attack of target cells.

Differential antigen processing explains why CTLs specific for TuAAs are often found among TILs without eradication of the disease. T lymphocyte responses are primed against TuAA that have been processed by the pAPC. CTLs found among TILs are hopelessly targeting class I TuAAs that were present on the pAPC, but not on the tumor cells (Figure 4).

The behavior of tumor cells in the body, namely migration, antigen shedding, induction of inflammatory responses, etc., results in strong immune responses. Unfortunately, the natural mechanism of differential antigen processing between tumor cells and pAPCs results in epitope isolation of the tumor-that is, the tumor has a different epitope signature than that of the pAPCs that process the TuAA, and thus the tumor epitopes are "isolated" from the epitopes that the CTLs are induced by the pAPCs to recognize. The ability to predict and counter this epitope isolation effect is crucial for development of a new generation of therapeutic cancer vaccines. Overcoming epitope isolation results in epitope synchronization.

### The Role of Epitope Differences in Infections by Viruses and other Intracellular Parasites

A wide variety of mechanisms are employed by persistent pathogens in order to establish chronic infections in the host organism. A common hallmark is reduced or altered antigen expression. In some embodiments, the present invention is directed to the treatment and prevention of intracellular infection by various pathogens. Examples of such pathogens include, but are not limited to: any viruses, bacteria, protozoa, prions or other organisms that have an intracellular stage of infection in the host.

Viral antigen presentation by the pAPCs begins with the digestion of viral antigens into peptides by the proteasome. After the proteasome digests the protein into peptides, some of the peptides are loaded onto the class I complex in the endoplasmic reticulum and transported to the cell surface. At the cell surface, the class I-peptide complex is recognized by T cell receptors on the surface of CTLs and the infected cells are killed.

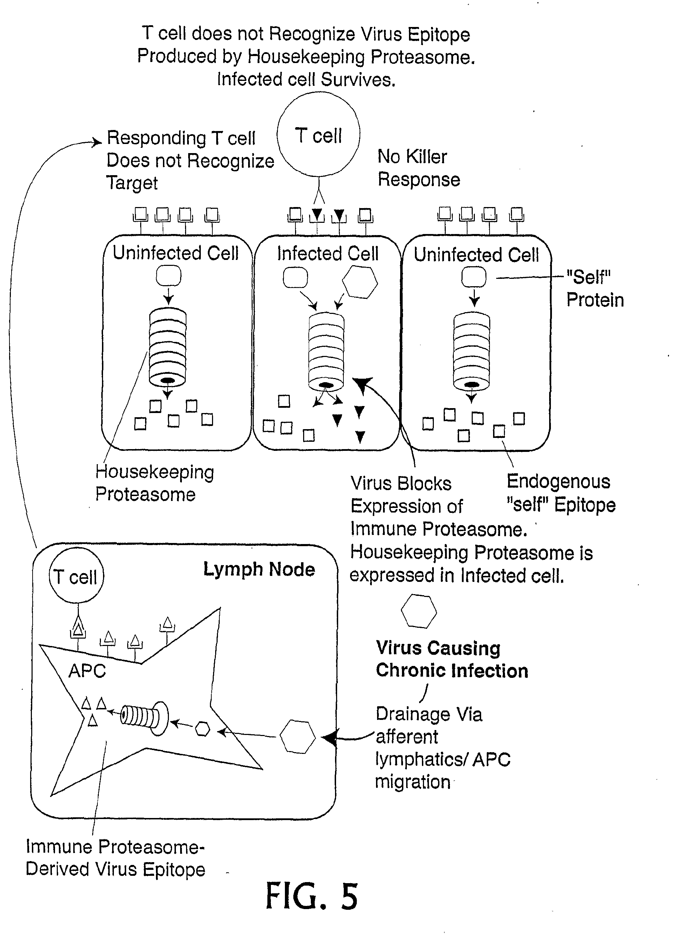

Herpes viruses and retroviruses escape detection and subsequent eradication by the host's immune system via restricted viral gene expression. Other mechanisms by which certain viruses may elude the immune system have also been proposed, including "immunologically privileged" sites of viral infection and antigenic variation in key viral peptides. While these models may explain the persistence of certain viruses, the concept of epitope synchronization, or conversely, epitope compartmentalization, provides a solution. Namely, this concept provides a basis for vaccines to direct an effective cellular immune response against any virus or other intracellular parasite that eludes the immune system by blocking immune proteasome expression in the host cells, or otherwise preventing effective epitope synchronization between infected cells and the pAPCs. (Figure 5).

Since infection of any cell by a pathogen usually causes the infected cell to produce IFN, the proteasome in infected tissue typically switches from the housekeeping configuration to an immune configuration. Infection thus has the effect of aligning the infected cell, in terms of the antigen repertoire it displays on its surface, with that of the pAPCs involved in stimulating the immune response against the virus or other intracellular pathogen. When virally infected cells or parasitically infected cells are induced to express an immune proteasome, rather than the housekeeping proteasome, the result is "epitope synchronization" between the infected cells and the pAPCs, and subsequent eradication of the infected cells by CTL.

However, certain viruses and other intracellular parasites may escape T cell recognition by down-regulating the expression of host molecules necessary for efficient T cell recognition of infected cells. There is evidence that suggests that many chronic viral infections interfere with the IFN cascade. (See Table 1). Therefore, because of the role of housekeeping proteasomes, immune proteasomes, and epitope compartmentalization, in many chronic infections, some embodiments of the invention are also applicable to the design of vaccines for any relevant intracellular parasite, including but not limited to viruses, bacteria, and protozoa. All intracellular parasites are targets for such vaccine design. These include but are not limited to: viruses such as adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, and human T cell leukemia virus II; bacteria such as *Chlamydia, Listeria, Salmonella, Legionella, Brucella, Coxiella, Rickettsia, Mycobacterium;* and protozoa such as *Leishmania, Trypanasoma, Toxoplasma,* and *Plasmodium.*

**Table 1**

| Virus | Viral Gene Product | Mechanism of IFN (Immune Proteasome) Inhibition |
|---|---|---|
| Adenovirus | EIA protein | Blocks signal transduction |
| | VA RNA | Blocks activation of PKr |
| Cowpox virus | crmA protein | Serpin, protease inhibitor blocks activation of I1-1β |
| Epstein-Barr virus | EBNA-2 protein | Blocks signal transduction |
| | EBER RNA | Blocks activation of Pkr |
| | BCRFL (VIRAL IL-10) | IL-10 HOMOLOG (inhibits Ifn-γ, I1-1, I1-2, Tnf synthesis) |
| Hepatitis B virus | Terminal protein | Blocks signal transduction |
| Human immunoDeficiency virus | Tat protein | Reduces activity of Pkr |
| | TAR RNA | Blocks activation of Pkr |
| Herpes simplex Virus | Unknown | Blocks activation of RNase L |
| Influenza virus | NSI | Binds dsRNA, blocking Pkr Activation |
| | Unknown | Activates cellular inhibitor of Pkr called p58 |
| Myxomavirus | M-T7 protein | Soluble Ifn-γ receptor (decoy) |
| | T2 protein | Soluble Tnf receptor (decoy) |
| Poliovirus | Unknown | Activates cellular inhibitor of Pkr |
| Reovirus | Sigma 3 protein | Binds dsRNA and blocks activation of Pkr |
| | K31 protein | Pkr pseudosubstrate |
| | B15R protein | Soluble I1-1 receptor (decoy) |
| | A18R protein | Regulates dsRNA production |

### Vaccines and Methods for Achieving Epitope Synchronization

As has been discussed herein, effective cellular immunity is based on synchronized epitope presentation between the pAPCs and the infected peripheral cells. In the absence of epitope synchronization, target cells are not recognized by T cells, even if those T cells are directed against TAAs. Cancer cells and cells harboring persistent intracellular parasites elude the cellular immune response because they avoid epitope synchronization. "Natural" epitope synchronization involves activation of immune proteasomes in infected cells so that the infected cells display immune epitopes and are thus recognized by T cells induced by pAPCs. Yet cancers and cells infected by persistent intracellular parasites do not have active immune proteasomes and thus go unrecognized by the normal array of induced T cells.

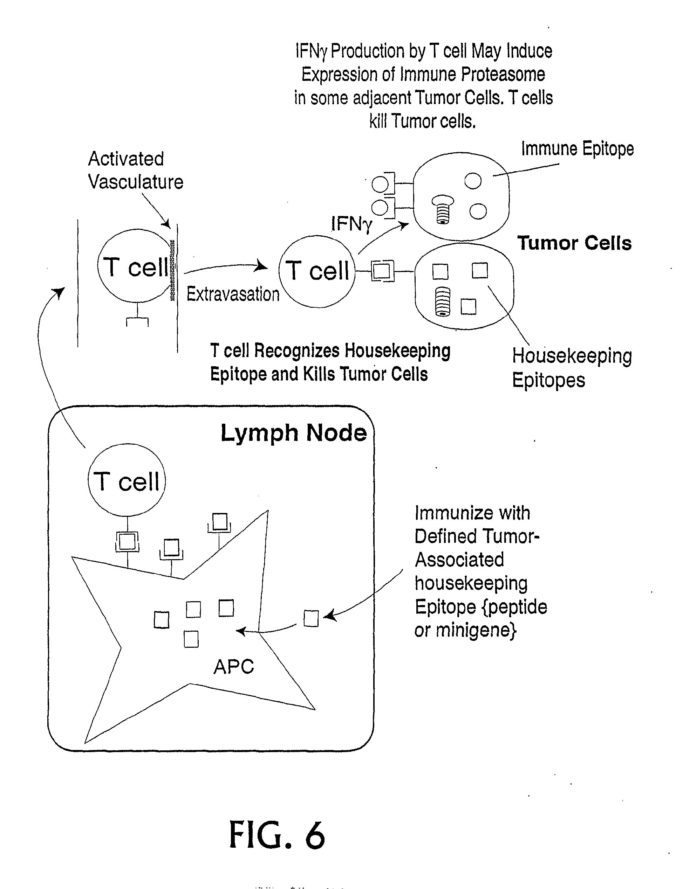

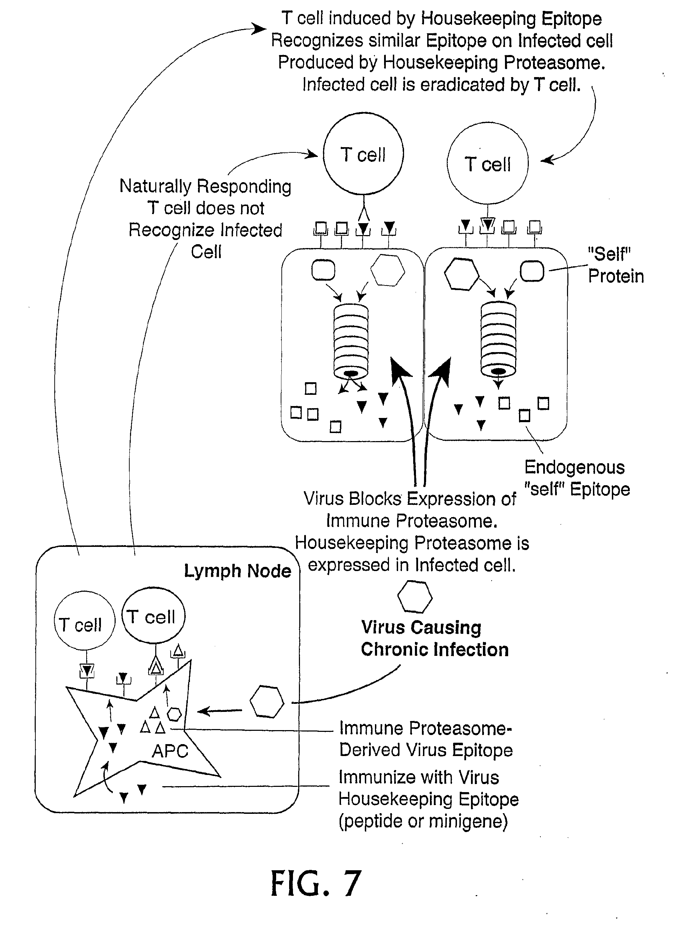

The vaccines and methods of preferred embodiments of the present invention thus represent, essentially, a "reverse" epitope synchronization, causing the pAPCs to display housekeeping epitopes to address situations in which target cells do not display immune epitopes. (Figures 6 and 7). Certain embodiments also provide a second wave of epitope synchronization by inducing pAPCs to display both housekeeping epitopes and immune epitopes corresponding to a selected target cell. Thus, in these dual epitope embodiments, once the target cells are effectively attacked by T cells that recognize housekeeping epitopes, a switch by the target cells to immune proteasome processing does not result in a loss of immune recognition. This is because of the presence of the immune epitope in the vaccine, which acts to induce a population of T cells that recognize immune epitopes.

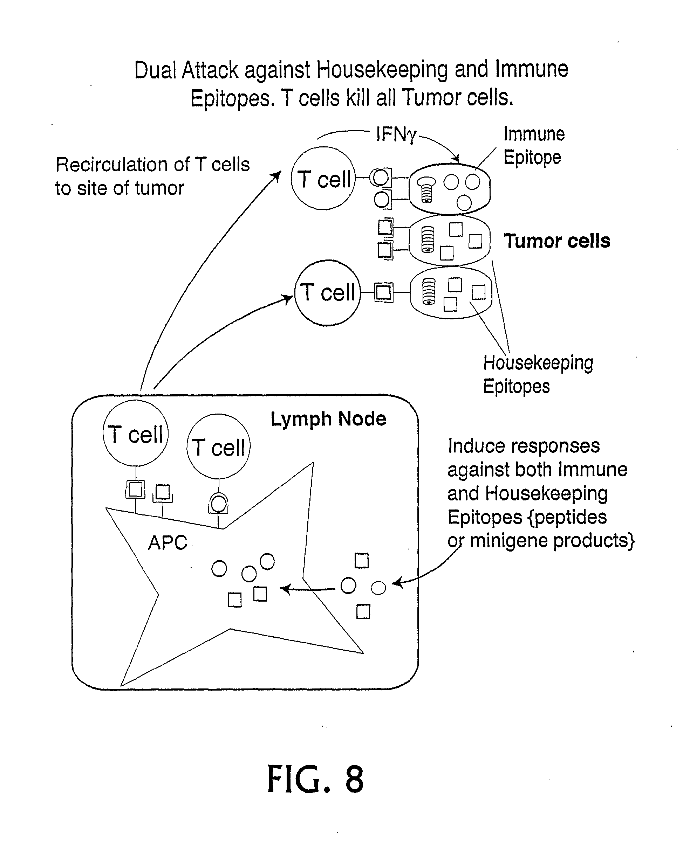

Preferred embodiments of the present invention are directed to vaccines and methods for causing a pAPC or population of pAPCs to present housekeeping epitopes that correspond to the epitopes displayed on a particular target cell. In one embodiment, the housekeeping epitope is a TuAA epitope processed by the housekeeping proteasome of a particular tumor type. In another embodiment, the housekeeping epitope is a virus-associated epitope processed by the housekeeping proteasome of a cell infected with a virus. This facilitates a specific T cell response to the target cells. Concurrent expression by the pAPCs of multiple epitopes, corresponding to different induction states (pre- and post-attack), can drive a CTL response effective against target cells as they display either housekeeping epitopes or immune epitopes. (Figure 8).

By having both housekeeping and immune epitopes present on the pAPC, this embodiment can optimize the cytotoxic T cell response to a target cell. With dual epitope expression, the pAPCs can continue to sustain a CTL response to the immune-type epitope when the tumor cell switches from the housekeeping proteasome to the immune proteasome with induction by IFN, which, for example, may be produced by tumor-infiltrating CTLs.

In a preferred embodiment, immunization of a patient is with a vaccine that includes a housekeeping epitope. Many preferred TAAs are associated exclusively with a target cell, particularly in the case of infected cells. In another embodiment, many preferred TAAs are the result of deregulated gene expression in transformed cells, but are found also in tissues of the testis, ovaries and fetus. In another embodiment, useful TAAs are expressed at higher levels in the target cell than in other cells. In still other embodiments, TAAs are not differentially expressed in the target cell compared to other cells, but are still useful since they are involved in a particular function of the cell and differentiate the target cell from most other peripheral cells; in such embodiments, healthy cells also displaying the TAA may be collaterally attacked by the induced T cell response, but such collateral damage is considered to be far preferable to the condition caused by the target cell.

When neoplastic cells are the target, preferred antigens include TuAAs. Examples of protein antigens suitable for use include differentiation antigens such as MelanA (MART-I), gp100 (Pmel 17), tyrosinase, TRP-1, TRP-2, and tumor-specific multilineage antigens such as MAGE-1, MAGE-3, BAGE, GAGE-1, GAGE-2, CEA, RAGE, NY-ESO, SCP-1, Hom/Mel-40 and FRAME. Similarly, TuAAs include overexpressed oncogenes, and mutated tumor-suppressor genes such as p53, H-Ras and HER-2/neu. Additionally, unique TuAAs resulting from chromosomal translocations such as BCR-ABL, E2A-PRL, H4-RET, IGH-IGK, MYL-RAR and viral antigens such as Epstein Barr virus antigens EBNA, and the human papillomavirus (HPV) antigens E6 and E7 are included. Other useful protein antigens include but are not limited to TSP-180, MAGE-4, MAGE-5, MAGE-6, p185erbB2, p180erbB-3, c-met, nm-23H1, PSA, TAG-72-4, CAM 17.1, NuMa, K-ras, β-Catenin, CDK4, Mum-1, and p16. These and other TuAAs and pathogen-related antigens are known and available to those of skill in the art in the literature or commercially.

In a further embodiment, the TAA is an antigen specific for a virus. See Table 2. In yet another embodiment of the present invention, the TAA is an antigen specific for a non-viral intracellular parasite. Examples of parasite-specific antigens include nucleotides, proteins, or other gene products associated with the intracellular parasite. Suitable nucleotides or proteins can be found at the NCBI Taxonomy Database located at http://www.ncbi.nlm.nih.gov/Taxonomy/tax.html/. More detailed descriptions of gene products for parasites and other pathogens are provided at this web site.

**Table 2**

| Virus | Candidate Gene Products |
|---|---|
| Herpes Simplex I | ICP4, VP16, ICPO, γ1^{34.5}, g13 |
| EBV | ZTA, EBNA-2, EBNA-1, LMP-1, LMP-2, LMP-2a, LMP-2b EBNA-3, EBNA-4, EBNA-LP, EBNA-3A, 3C, BZLF-1 |
| Poxvirus | VeTF, K3L, p37, A14L, A13L, A17L, A18R |
| SV40 | Large T antigen, Small T antigen, VPZ |
| Adenovirus | E1A, E3L, E1B, E4 (OEF6), E4 (ORF1), gp19K, ADP, RIDα, RIDβ |
| Hepatitis B | pX, L(pre-Si) |
| Htlv-1 | Tax |
| HIV | TAT, GAG, MA, ENV, TM, NEF, VIF, VPR, REV, VPX |
| Hepatis B | NS5A |
| Reovirus | δ-3 |
| Rous Sarcoma Virus | pp60^{src} |
| Harvey Sarcoma Virus | p21^{ras} |
| HPV | E6, E7, E5 |
| Polyomavirus | LT, mT, sT |

The compounds and methods described herein are effective in any context wherein a target cell displays housekeeping epitopes. Methods of discovering effective epitopes for use in connection with the vaccine and treatment embodiments of this invention are disclosed herein.

### Proteolytic Processing of Antigens

Epitopes that are displayed by MHC on target cells or on pAPCs are cleavage products of larger protein antigen precursors. For MHC I epitopes, protein antigens are digested by proteasomes resident in the cell. See Figure 1. Intracellular proteasomal digestion typically produces peptide fragments of about 3 to 23 amino acids in length. Additional proteolytic activities within the cell, or in the extracellular milieu, can trim and process these fragments further. Processing of MHC II epitopes occurs via intracellular proteases from the lysosomal/endosomal compartment.

Presumably, most products of protein processing by proteasomes or other protease activities have little or no affinity for the binding cleft of a particular MHC receptor peptide. However, the processing products that do have such an affinity are likely to be presented, at some level of abundance, by MHC at the cell surface. Conversely, if a given oligopeptide sequence does not emerge intact from the antigen processing activities of the cell, it cannot be presented at the cell surface, regardless of the predicted affinity of the sequence for MHC.

Vaccine design that focuses entirely on MHC affinity is fundamentally flawed. The mere fact that a peptide has MHC binding affinity does not ensure that such a peptide will make for a functional immunogen. To provide an epitope capable of eliciting an effective immune response against a TAA, the peptide must have MHC binding affinity and be the product of cellular peptide generating systems. The methods of the disclosed invention utilize both MHC binding affinity analysis and antigen processing analysis protocols to identify new epitopes of interest.

### Correlating Predicted or Known MHC Binding with Proteolytic Processing of Antigens

To identify epitopes potentially effective as immunogenic compounds, predictions of MHC binding alone generally are disadvantageous, because many fragments with predicted binding are never actually formed in the cell. Embodiments of the invention combine an analysis of MHC binding with an analysis of proteolytic processing to identify epitopes that have both of the essential properties of a useful epitope: MHC affinity and correct proteolytic processing. Peptides having both of these properties are strong candidates for vaccines and immunotherapies. Peptides lacking either of these properties are unlikely to have any significant opportunity to function as effective epitopes.

Embodiments of the invention are capable of identifying epitopes derived from TAAs for use in vaccines. The target antigens can be derived from neoplastic cells, cells infected with a virus or other intracellular parasite, or cells infected with other pathogenic agents such as bacteria, fungi, protozoans, viruses, prions, toxins, venoms, allergens, and the like. In short, embodiments of the method can be applied to virtually any protein sequence, to identify therein epitopes capable of generation by proteolysis and capable of binding to MHC. Accordingly, the invention is not limited to any particular target or medical condition, but instead encompasses discovery of biologically relevant MHC epitopes from any useful source.

In a preferred embodiment, the TAA is characteristic of a neoplastic cell and is thus defined as a tumor-associated antigen (TuAA). Preferred TuAAs include: differentiation antigens such as MelanA (MART-1), gp100 (Pmel 17), tyrosinase, TRP-1, TRP-2, MAGE-1, MAGE-3, BAGE, GAGE-1, GAGE-2, p15; overexpressed embryonic antigens generally; overexpressed oncogenes and mutated tumor-suppressor genes such as p53, Ras, HER-2/neu; unique tumor antigens resulting from chromosomal translocations such as BCR-ABL, E2A-PRL, H4-RET, IGH-IGK, MYL-RAR1 and viral antigens, such as Epstein Barr virus antigens (EBVA) and the human papillomavirus (HPV) antigens E6 and E7. Other antigens of interest include prostate specific antigen (PSA), prostate stem cell antigen (PSCA), MAAT-1, GP-100, TSP-180, MAGE-4, MAGE-5, MAGE-6, RAGE, p185erbB-2, p185erbB-3, c-met, nm-23H1, TAG-72, CA 19-9, CA 72-4, CAM 17.1, NuMa, K-ras, -Catenin, CDK4, Mum-1, p15, and p16. Other target antigens are also contemplated.

A variety of methods are available and well known in the art to identify TuAAs. Examples of these techniques include differential hybridization, including the use of microarrays; subtractive hybridization cloning; differential display, either at the level of mRNA or protein expression; EST sequencing; and SAGE (sequential analysis of gene expression). These nucleic acid techniques have been reviewed by Carulli, J.P. et al J. Cellular Biochem Suppl. 30/31:286-296, 1998. Differential display of proteins involves, for example, comparison of two-dimensional polyacrylamide gel electrophoresis of cell lysates from tumor and normal tissue, location of protein spots unique or overexpressed in the tumor, recovery of the protein from the gel, and identification of the protein using traditional biochemical or mass spectrometry sequencing techniques. An additional technique for identification of TuAAs is the SEREX technique, discussed in Türeci, Ö., Sahin, U., and Pfreundschuh, M., "Serological analysis of human tumor antigens: molecular definition and implications", Molecular Medicine Today, 3:342, 1997. Use of these and other methods provides one of skill in the art the techniques necessary to identify useful antigens for generating housekeeping and immune class I epitopes, as well as class II epitopes for a vaccines. However, it is not necessary, in practicing the invention, to identify a novel TuAA or TAA. Rather, embodiments of the invention make it possible to identify useful epitopes from any relevant protein sequence, whether the sequence is already known or novel.

### Analysis of TAA Fragments for MHC Binding

In order to identify biologically relevant epitopes, fragments within the TAA with a known or predicted affinity for MHC are identified. The amino acid sequence of a TAA can be analyzed by a number of different techniques with which to identify peptide fragments having a known or predicted affinity for the MHC peptide binding cleft. In one embodiment of the invention, TAA fragments are analyzed for their predicted ability to bind to the MHC peptide binding cleft using a computer algorithm. Each allele of MHC specifies a particular epitope binding domain. Thus, for any given MHC allele, the candidate peptides can be screened for predicted affinity thereto. Examples of suitable computer algorithms for this purpose include that found at the world wide web page of Hans-Georg Rammensee, Jutta Bachmann, Niels Emmerich, Stefan Stevanovic: SYFPEITHI: An Internet Database for MHC Ligands and Peptide Motifs (http://access via: syfpeithi.bmi-heidelberg.com/scripts/MHCServer.dll/home.htm). Results obtained from this method are discussed in Rammensee, et al., "MHC Ligands and Peptide Motifs," Landes Bioscience Austin, TX, 224-227, 1997. Another http:// site of interest is "bimas.dcrt.nih.gov/molbio/hla_bind," which also contains a suitable algorithm. The methods of this web site are discussed in Parker, et al., "Scheme for ranking potential HLA-A2 binding peptides based on independent binding of individual peptide side-chains," J. Immunol. 152:163-175.

Using the NIH (Parker) algorithm with the methods of the invention would select peptides using a number of possible retention times to indicate a binding sequence. In one embodiment, peptides with an infinite retention time would be selected. In another embodiment, peptides with a retention time of 25 minutes or more would be selected to indicate a binding sequence. In still another embodiment, a retention time of 15 minutes or more would be selected to indicate a binding sequence. In still another embodiment, a retention time of 10 minutes or more would be selected to indicate a binding sequence. Retention times of 9, 8, 7, 6, 5, 4, 3, 2, and 1 minute are also contemplated.

As an alternative to predictive algorithms, a number of standard *in vitro* receptor binding affinity assays are available to identify peptides having an affinity for a particular allele of MHC. Accordingly, by the method of this aspect of the invention, the initial population of peptide fragments can be narrowed to include only those peptides having an actual or predicted affinity for the selected allele of MHC.

Initially, peptide candidates for this analysis can include every possible sequence of about 6 to 24 contiguous amino acids from the entire protein sequence of the TAA. In a preferred embodiment, the sequences can be from about 7 to 20 amino acids in length. In a more preferred embodiment, the sequences can be from about 8 to 15 amino acids in length. For sequence analysis to identify fragments with predicted affinity for MHC I, a most preferred embodiment analyzes all possible sequences of 9 or 10 contiguous amino acid fragments of the TAA. Analysis of the MHC affinity of the fragments can be conducted *in vitro* or via computer analysis of the fragments.

Selected common alleles of MHC I, and their approximate frequencies, are reported in the tables 3-5 below.

**Table 3. Estimated gene frequencies of HLA-A antigens**

| Antigen | CAU | | AFR | | ASI | | LAT | | NAT | |
|---|---|---|---|---|---|---|---|---|---|---|
| | Gf^{a} | SE^{b} | Gf | SE | Gf | SE | Gf | SE | Gf | SE |
| A1 | 15.1843 | 0.0489 | 5.7256 | 0.0771 | 4.4818 | 0.0846 | 7.4007 | 0.0978 | 12.0316 | 0.2533 |
| A2 | 28.6535 | 0.0619 | 18.8849 | 0.1317 | 24.6352 | 0.1794 | 28.1198 | 0.1700 | 29.3408 | 0.3585 |
| A3 | 13.3890 | 0.0463 | 8.4406 | 0.0925 | 2.6454 | 0.0655 | 8.0789 | 0.1019 | 11.0293 | 0.2437 |
| A28 | 4.4652 | 0.0280 | 9.9269 | 0.0997 | 1.7657 | 0.0537 | 8.9446 | 0.1067 | 5.3856 | 0.1750 |
| A36 | 0.0221 | 0.0020 | 1.8836 | 0.0448 | 0.0148 | 0.0049 | 0.1584 | 0.0148 | 0.1545 | 0.0303 |
| A23 | 1.8287 | 0.0181 | 10.2086 | 0.1010 | 0.3256 | 0.0231 | 2.9269 | 0.0628 | 1.9903 | 0.1080 |
| A24 | 9.3251 | 0.0395 | 2.9668 | 0.0560 | 22.0391 | 0.1722 | 13.2610 | 0.1271 | 12.6613 | 0.2590 |
| A9 unsplit | 0.0809 | 0.0038 | 0.0367 | 0.0063 | 0.0858 | 0.0119 | 0.0537 | 0.0086 | 0.0356 | 0.0145 |
| A9 total | 11.2347 | 0.0429 | 13.2121 | 0.1128 | 22.4505 | 0.1733 | 16.2416 | 0.1382 | 14.6872 | 0.2756 |
| A25 | 2.1157 | 0.0195 | 0.4329 | 0.0216 | 0.0990 | 0.0128 | 1.1937 | 0.0404 | 1.4520 | 0.0924 |
| A26 | 3.8795 | 0.0262 | 2.8284 | 0.0547 | 4.6628 | 0.0862 | 3.2612 | 0.0662 | 2.4292 | 0.1191 |
| A34 | 0.1508 | 0.0052 | 3.5228 | 0.0610 | 1.3529 | 0.0470 | 0.4928 | 0.0260 | 0.3150 | 0.0432 |
| A43 | 0.0018 | 0.0006 | 0.0334 | 0.0060 | 0.0231 | 0.0062 | 0.0055 | 0.0028 | 0.0059 | 0.0059 |
| A66 | 0.0173 | 0.0018 | 0.2233 | 0.0155 | 0.0478 | 0.0089 | 0.0399 | 0.0074 | 0.0534 | 0.0178 |
| A10 unsplit | 0.0790 | 0.0038 | 0.0939 | 0.0101 | 0.1255 | 0.0144 | 0.0647 | 0.0094 | 0.0298 | 0.0133 |
| A10 total | 6.2441 | 0.0328 | 7.1348 | 0.0850 | 6.3111 | 0.0993 | 5.0578 | 0.0816 | 4.2853 | 0.1565 |
| A29 | 3.5796 | 0.0252 | 3.2071 | 0.0582 | 1.1233 | 0.0429 | 4.5156 | 0.0774 | 3.4345 | 0.1410 |
| A30 | 2.5067 | 0.0212 | 13.0969 | 0.1129 | 2.2025 | 0.0598 | 4.4873 | 0.0772 | 2.5314 | 0.1215 |
| A31 | 2.7386 | 0.0221 | 1.6556 | 0.0420 | 3.6005 | 0.0761 | 4.8328 | 0.0800 | 6.0881 | 0.1855 |
| A32 | 3.6956 | 0.0256 | 1.5384 | 0.0405 | 1.0331 | 0.0411 | 2.7064 | 0.0604 | 2.5521 | 0.1220 |
| A33 | 1.2080 | 0.0148 | 6.5607 | 0.0822 | 9.2701 | 0.1191 | 2.6593 | 0.0599 | 1.0754 | 0.0796 |
| A74 | 0.0277 | 0.0022 | 1.9949 | 0.0461 | 0.0561 | 0.0096 | 0.2027 | 0.0167 | 0.1068 | 0.0252 |
| A19 unsplit | 0.0567 | 0.0032 | 0.2057 | 0.0149 | 0.0990 | 0.0128 | 0.1211 | 0.0129 | 0.0475 | 0.0168 |
| A19 total | 13.8129 | 0.0468 | 28.2593 | 0.1504 | 17.3846 | 0.1555 | 19.5252 | 0.1481 | 15.8358 | 0.2832 |
| AX | 0.8204 | 0.0297 | 4.9506 | 0.0963 | 2.9916 | 0.1177 | 1.6332 | 0.0878 | 1.8454 | 0.1925 |

| | | | | | | | | | | |
|---|---|---|---|---|---|---|---|---|---|---|
| ^{a}Gene frequency. ^{b}Standard error. | | | | | | | | | | |

**Table 4. Estimated gene frequencies for HLA-B antigens**

| Antigen | CAU | | AFR | | ASI | | LAT | | NAT | |
|---|---|---|---|---|---|---|---|---|---|---|
| | Gf^{a} | SE^{b} | Gf | SE | Gf | SE | Gf | SE | Gf | SE |
| B7 | 12.1782 | 0.0445 | 10.5960 | 0.1024 | 4.2691 | 0.0827 | 6.4477 | 0.0918 | 10.9845 | 0.2432 |
| B8 | 9.4077 | 0.0397 | 3.8315 | 0.0634 | 1.3322 | 0.0467 | 3.8225 | 0.0715 | 8.5789 | 0.2176 |
| B13 | 2.3061 | 0.0203 | 0.8103 | 0.0295 | 4.9222 | 0.0886 | 1.2699 | 0.0416 | 1.7495 | 0.1013 |
| B14 | 4.3481 | 0.0277 | 3.0331 | 0.0566 | 0.5004 | 0.0287 | 5.4166 | 0.0846 | 2.9823 | 0.1316 |
| B18 | 4.7980 | 0.0290 | 3.2057 | 0.0582 | 1.1246 | 0.0429 | 4.2349 | 0.0752 | 3.3422 | 0.1391 |
| B27 | 4.3831 | 0.0278 | 1.2918 | 0.0372 | 2.2355 | 0.0603 | 2.3724 | 0.0567 | 5.1970 | 0.1721 |
| B35 | 9.6614 | 0.0402 | 8.5172 | 0.0927 | 8.1203 | 0.1122 | 14.6516 | 0.1329 | 10.1198 | 0.2345 |
| B37 | 1.4032 | 0.0159 | 0.5916 | 0.0252 | 1.2327 | 0.0449 | 0.7807 | 0.0327 | 0.9755 | 0.0759 |
| B41 | 0.9211 | 0.0129 | 0.8183 | 0.0296 | 0.1303 | 0.0147 | 1.2818 | 0.0418 | 0.4766 | 0.0531 |
| B42 | 0.0608 | 0.0033 | 5.6991 | 0.0768 | 0.0841 | 0.0118 | 0.5866 | 0.0284 | 0.2856 | 0.0411 |
| B46 | 0.0099 | 0.0013 | 0.0151 | 0.0040 | 4.9292 | 0.0886 | 0.0234 | 0.0057 | 0.0238 | 0.0119 |
| B47 | 0.2069 | 0.0061 | 0.1305 | 0.0119 | 0.0956 | 0.0126 | 0.1832 | 0.0159 | 0.2139 | 0.0356 |
| B48 | 0.0865 | 0.0040 | 0.1316 | 0.0119 | 2.0276 | 0.0575 | 1.5915 | 0.0466 | 1.0267 | 0.0778 |
| B53 | 0.4620 | 0.0092 | 10.9529 | 0.1039 | 0.4315 | 0.0266 | 1.6982 | 0.0481 | 1.0804 | 0.0798 |
| B59 | 0.0020 | 0.0006 | 0.0032 | 0.0019 | 0.4277 | 0.0265 | 0.0055 | 0.0028 | 0^{c} | - |
| B67 | 0.0040 | 0.0009 | 0.0086 | 0.0030 | 0.2276 | 0.0194 | 0.0055 | 0.0028 | 0.0059 | 0.0059 |
| B70 | 0.3270 | 0.0077 | 7.3571 | 0.0866 | 0.8901 | 0.0382 | 1.9266 | 0.0512 | 0.6901 | 0.0639 |
| B73 | 0.0108 | 0.0014 | 0.0032 | 0.0019 | 0.0132 | 0.0047 | 0.0261 | 0.0060 | 0° | - |
| B51 | 5.4215 | 0.0307 | 2.5980 | 0.0525 | 7.4751 | 0.1080 | 6.8147 | 0.0943 | 6.9077 | 0.1968 |
| B52 | 0.9658 | 0.0132 | 1.3712 | 0.0383 | 3.5121 | 0.0752 | 2.2447 | 0.0552 | 0.6960 | 0.0641 |
| B5 unsplit | 0.1565 | 0.0053 | 0.1522 | 0.0128 | 0.1288 | 0.0146 | 0.1546 | 0.0146 | 0.1307 | 0.0278 |
| B5 total | 6.5438 | 0.0435 | 4.1214 | 0.0747 | 11.1160 | 0.1504 | 9.2141 | 0.1324 | 7.7344 | 0.2784 |
| B44 | 13.4838 | 0.0465 | 7.0137 | 0.0847 | 5.6807 | 0.0948 | 9.9253 | 0.1121 | 11.8024 | 0.2511 |
| B45 | 0.5771 | 0.0102 | 4.8069 | 0.0708 | 0.1816 | 0.0173 | 1.8812 | 0.0506 | 0.7603 | 0.0670 |
| B12 unsplit | 0.0788 | 0.0038 | 0.0280 | 0.0055 | 0.0049 | 0.0029 | 0.0193 | 0.0051 | 0.0654 | 0.0197 |
| B12 total | 14.1440 | 0.0474 | 11.8486 | 0.1072 | 5.8673 | 0.0963 | 11.8258 | 0.1210 | 12.6281 | 0.2584 |
| B62 | 5.9117 | 0.0320 | 1.5267 | 0.0404 | 9.2249 | 0.1190 | 4.1825 | 0.0747 | 6.9421 | 0.1973 |
| B63 | 0.4302 | 0.0088 | 1.8865 | 0.0448 | 0.4438 | 0.0270 | 0.8083 | 0.0333 | 0.3738 | 0.0471 |
| B75 | 0.0104 | 0.0014 | 0.0226 | 0.0049 | 1.9673 | 0.0566 | 0.1101 | 0.0123 | 0.0356 0 | 0.0145 |
| B76 | 0.0026 | 0.0007 | 0.0065 | 0.0026 | 0.0874 | 0.0120 | 0.0055 | 0.0028 | 0^{c} | - |
| B77 | 0.0057 | 0.0010 | 0.0119 | 0.0036 | 0.0577 | 0.0098 | 0.0083 | 0.0034 | 0.0059 | 0.0059 |
| B15 unsplit | 0.1305 | 0.0049 | 0.0691 | 0.0086 | 0.4301 | 0.0266 | 0.1820 | 0.0158 | 0.0715 | 0.0206 |
| B15 total | 6.4910 | 0.0334 | 3.5232 | 0.0608 | 12.2112 | 0.1344 | 5.2967 | 0.0835 | 7.4290 | 0.2035 |
| B38 | 2.4413 | 0.0209 | 0.3323 | 0.0189 | 3.2818 | 0.0728 | 1.9652 | 0.0517 | 1.1017 | 0.0806 |
| B39 | 1.9614 | 0.0188 | 1.2893 | 0.0371 | 2.0352 | 0.0576 | 6.3040 | 0.0909 | 4.5527 | 0.1615 |
| B16 unsplit | 0.0638 | 0.0034 | 0.0237 | 0.0051 | 0.0644 | 0.0103 | 0.1226 | 0.0130 | 0.0593 | 0.0188 |
| B16 total | 4.4667 | 0.0280 | 1.6453 | 0.0419 | 5.3814 | 0.0921 | 8.3917 | 0.1036 | 5.7137 | 0.1797 |
| B57 | 3.5955 | 0.0252 | 5.6746 | 0.0766 | 2.5782 | 0.0647 | 2.1800 | 0.0544 | 2.7265 | 0.1260 |
| B58 | 0.7152 | 0.0114 | 5.9546 | 0.0784 | 4.0189 | 0.0803 | 1.2481 | 0.0413 | 0.9398 | 0.0745 |
| B17 unsplit | 0.2845 | 0.0072 | 0.3248 | 0.0187 | 0.3751 | 0.0248 | 0.1446 | 0.0141 | 0.2674 | 0.0398 |
| B17 total | 4.5952 | 0.0284 | 11.9540 | 0.1076 | 6.9722 | 0.1041 | 3.5727 | 0.0691 | 3.9338 | 0.1503 |
| B49 | 1.6452 | 0.0172 | 2.6286 | 0.0528 | 0.2440 | 0.0200 | 2.3353 | 0.0562 | 1.5462 | 0.0953 |
| B50 | 1.0580 | 0.0138 | 0.8636 | 0.0304 | 0.4421 | 0.0270 | 1.8883 | 0.0507 | 0.7862 | 0.0681 |
| B21 unsplit | 0.0702 | 0.0036 | 0.0270 | 0.0054 | 0.0132 | 0.0047 | 0.0771 | 0.0103 | 0.0356 | 0.0145 |
| B21 total | 2.7733 | 0.0222 | 3.5192 | 0.0608 | 0.6993 | 0.0339 | 4.3007 | 0.0755 | 2.3680 | 0.1174 |
| B54 | 0.0124 | 0.0015 | 0.0183 | 0.0044 | 2.6873 | 0.0660 | 0.0289 | 0.0063 | 0.0534 | 0.0178 |
| B55 | 1.9046 | 0.0185 | 0.4895 | 0.0229 | 2.2444 | 0.0604 | 0.9515 | 0.0361 | 1.4054 | 0.0909 |
| B56 | 0.5527 | 0.0100 | 0.2686 | 0.0170 | 0.8260 | 0.0368 | 0.3596 | 0.0222 | 0.3387 | 0.0448 |
| B22 unsplit | 0.1682 | 0.0055 | 0.0496 | 0.0073 | 0.2730 | 0.0212 | 0.0372 | 0.0071 | 0.1246 | 0.0272 |
| B22 total | 2.0852 | 0.0217 | 0.8261 | 0.0297 | 6.0307 | 0.0971 | 1.3771 | 0.0433 | 1.9221 | 0.1060 |
| B60 | 5.2222 | 0.0302 | 1.5299 | 0.0404 | 8.3254 | 0.1135 | 2.2538 | 0.0553 | 5.7218 | 0.1801 |
| B61 | 1.1916 | 0.0147 | 0.4709 | 0.0225 | 6.2072 | 0.0989 | 4.6691 | 0.0788 | 2.6023 | 0.1231 |
| B40 unsplit | 0.2696 | 0.0070 | 0.0388 | 0.0065 | 0.3205 | 0.0230 | 0.2473 | 0.0184 | 0.2271 | 0.0367 |
| B40 total | 6.6834 | 0.0338 | 2.0396 | 0.0465 | 14.8531 | 0.1462 | 7.1702 | 0.0963 | 8.5512 | 0.2168 |
| BX | 1.0922 | 0.0252 | 3.5258 | 0.0802 | 3.8749 | 0.0988 | 2.5266 | 0.0807 | 1.9867 | 0.1634 |

| | | | | | | | | | | |
|---|---|---|---|---|---|---|---|---|---|---|
| ^{a}Gene frequency. ^{b}Standard error. ^{c}The observed gene count was zero. | | | | | | | | | | |

**Table 5. Estimated gene frequencies of HLA-DR antigens**

| Antigen | CAU | | AFR | | ASI | | LAT | | NAT | |
|---|---|---|---|---|---|---|---|---|---|---|
| | Gf^{a} | SE^{b} | Gf | SE | Gf | SE | SE | SE | Gf | SE |
| DR1 | 10.2279 | 0.0413 | 6.8200 | 0.0832 | 3.4628 | 0.0747 | 7.9859 | 0.1013 | 8.2512 | 0.2139 |
| DR2 | 15.2408 | 0.0491 | 16.2373 | 0.1222 | 18.6162 | 0.1608 | 11.2389 | 0.1182 | 15.3932 | 0.2818 |
| DR3 | 10.8708 | 0.0424 | 13.3080 | 0.1124 | 4.7223 | 0.0867 | 7.8998 | 0.1008 | 10.2549 | 0.2361 |
| DR4 | 16.7589 | 0.0511 | 5.7084 | 0.0765 | 15.4623 | 0.1490 | 20.5373 | 0.1520 | 19.8264 | 0.3123 |
| DR6 | 14.3937 | 0.0479 | 18.6117 | 0.1291 | 13.4471 | 0.1404 | 17.0265 | 0.1411 | 14.8021 | 0.2772 |
| DR7 | 13.2807 | 0.0463 | 10.1317 | 0.0997 | 6.9270 | 0.1040 | 10.6726 | 0.1155 | 10.4219 | 0.2378 |
| DR8 | 2.8820 | 0.0227 | 6.2673 | 0.0800 | 6.5413 | 0.1013 | 9.7731 | 0.1110 | 6.0059 | 0.1844 |
| DR9 | 1.0616 | 0.0139 | 2.9646 | 0.0559 | 9.7527 | 0.1218 | 1.0712 | 0.0383 | 2.8662 | 0.1291 |
| DR10 | 1.4790 | 0.0163 | 2.0397 | 0.0465 | 2.2304 | 0.0602 | 1.8044 | 0.0495 | 1.0896 | 0.0801 |
| DR11 | 9.3180 | 0.0396 | 10.6151 | 0.1018 | 4.7375 | 0.0869 | 7.0411 | 0.0955 | 5.3152 | 0.1740 |
| DR12 | 1.9070 | 0.0185 | 4.1152 | 0.0655 | 10.1365 | 0.1239 | 1.7244 | 0.0484 | 2.0132 | 0.1086 |
| DR5 unsplit | 1.2199 | 0.0149 | 2.2957 | 0.0493 | 1.4118 | 0.0480 | 1.8225 | 0.0498 | 1.6769 | 0.0992 |
| DR5 total | 12.4449 | 0.0045 | 17.0260 | 0.1243 | 16.2858 | 0.1516 | 10.5880 | 0.1148 | 9.0052 | 0.2218 |
| DRX | 1.3598 | 0.0342 | 0.8853 | 0.0760 | 2.5521 | 0.1089 | 1.4023 | 0.0930 | 2.0834 | 0.2037 |

| | | | | | | | | | | |
|---|---|---|---|---|---|---|---|---|---|---|
| ^{a}Gene frequency. ^{b}Standard error. | | | | | | | | | | |

Tables 3, 4, and 5 derived from HLA Gene and Haplotype Frequencies in the North American Population: The National Marrow Donor Program Donor Registry, Mori, M. et al.

### Determining Whether a Fragment with MHC Affinity is a Useful Epitope

As discussed above, a preliminary step of the disclosed method is to select from among the original population of peptide fragments a subpopulation of peptides with an actual or predicted MHC affinity. The selected fragments are analyzed further to determine which can be produced by a cell under *in vivo* conditions that could result in binding of the peptide to the selected MHC allele. All peptides that meet both criteria of MHC affinity and correct proteolytic processing are designated as "discovered epitopes." A variety of methods are available for determining which peptide fragments can be produced by proteolytic processing *in vivo.* These methods include elution of peptides from solubilized MHC and intact cells, computer sequence analysis of the proteolytic cleavage motifs, and *in vitro* analysis of actual peptide fragments produced by cellular proteolytic machinery.

In a preferred embodiment, a series of synthetic peptides centrally containing either individual or clustered candidate peptide sequences can be generated. Such peptides typically range in length from about 10 to about 75 amino acids. In a preferred embodiment, the synthetic peptide is between about 20 and 60 amino acids in length. In a more preferred embodiment, the cluster is between about 30 and 40 amino acids in length. Using standard peptide synthesis chemistry, including t-Boc protection chemistry, Fmoc protection chemistry, and the like, one of ordinary skill in art the can produce a population of candidate peptides for subsequent screening.

Alternatively, peptide fragments containing candidate peptides can be generated *in vitro* through protease digestion or chemical cleavage of the TAA or fragments thereof. Protease digestion to prepare such fragments of TAAs can employ a wide variety of known proteases, including but not limited to proteasome proteases, trypsin, -chymotrypsin, bromelain, clostripain, elastase, endoproteinases, exoproteinases, proteinase K, ficin, papain, pepsin, plasmin, thermolysin, thrombin, trypsin, cathepsins, and others. Chemical methods can also be used to generate peptide candidates. Suitable chemicals or chemical reactions for cleaving peptide bonds include mild acid cleavage, cyanogen bromide, hydroxylamine, iodosobenzoic acid, 2-Nitro-5-thiocyanobenzoate, and the like. In one embodiment, the unfragmented TAA can be used, although the use of a particularly large initial sequence can complicate the analysis.

Regardless of how the fragments containing candidate peptides are created, determining which epitopes are produced by the cellular machinery is important. In one embodiment of the invention, proteasome digestion is used to estimate cellular epitope generation. In this embodiment, immune and housekeeping proteasomes are purified for *in vitro* use in order to assess the antigenic repertoire generated naturally from the two kinds of proteasomes.

Generally, proteasomes are prepared by affinity purification from cell extracts. In a preferred embodiment, a cell lysate is prepared using standard techniques. The lysate is cleared by ultracentrifugation if erythrocytes are not the original source material. The prepared cell lysate is then purified from other cellular components using any one of a number of purification techniques including various forms of chromatography.

In one embodiment affinity chromatography is used to purify the proteasomes. The cell lysate is applied to an affinity column containing a monoclonal antibody (mAb) against one of the proteasomal subunits. The column is then washed to purify the bound proteasomes from other cellular material. Following washing, the bound proteasomes are then eluted from the column. The eluate is characterized in terms of protein content and proteolytic activity on a standard substrate.

Cleavage analysis using both housekeeping and immune proteasomes yields class I epitopes from various TAA. The epitopes that are presented by pAPCs correspond to cleavage products of the immune proteasome, while the epitopes presented by tumors and by many cells chronically infected with intracellular parasites correspond to cleavage products of the housekeeping proteasome. Once the digest is performed, the particular molecular species produced are identified. In a preferred embodiment, this is accomplished by mass spectrometry. This allows the rapid identification of natural peptide fragments that are produced by either of the two kinds of proteasomes. In another embodiment, cleavage of the target antigen or fragments thereof by immune and housekeeping proteasomes, or by endosomal/lysosomal proteases (see below), is predicted by computer modeling based on cleavage motifs of the relevant proteolytic activities.

Whereas class I MHC is loaded primarily with proteasomally derived peptides as it initially folds in the endoplasmic reticulum, the binding cleft of class II MHC is blocked by the so-called invariant chain (Ii) in this compartment. Loading of peptide for class II MHC takes place primarily in the endosomal compartment, utilizing peptides generated by endosomal and lysosomal proteases. Thus if *in vitro* identification of MHC class II epitopes is desired, preparations of proteases from endosomal and/or lysosomal fractions can be substituted for the proteasomes. A variety of methods to accomplish this substitution are described in the literature. For example, Kido & Ohshita, Anal. Biochem., 230:41-7 (1995); Yamada, et al., J. Biochem. (Tokyo), 95:1155-60 (1984); Kawashima, et al., Kidney Int., 54:275-8 (1998); Nakabayshi & Ikezawa, Biochem. Int. 16:1119-25 (1988); Kanaseki & Ohkuma, J. Biochem. (Tokyo), 110:541-7 (1991); Wattiaux, et al., J. Cell Biol., 78:349-68 (1978); Lisman, et al., Biochem. J. 178:79-87 (1979); Dean, B., Arch. Biochem. Biophys., 227:154-63 (1983); Overdijk, et al., Adv. Exp. Med. Biol., 101:601-10 (1978); Stromhaug, et al., Biochem. J., Biochem. J., 335:217-24 (1998); Escola, et al., J. Biol. Chem. 271:27360-5 (1996); Hammond, et al., Am. J. Physiol., 267:F516-27 (1994); Williams & Smith, Arch. Biochem. Biophys. 305:298-306 (1993); Marsh, M., Methods Cell Biol., 31:319-34 (1989); and Schmid & and Mellman, Prog. Clin. Biol. Res., 270:35-49 (1988) all disclose methods to prepare suitable proteolytic preparations.

In another embodiment, the digestion to determine which epitopes the cellular machinery produces, takes place within a cell expressing the TAA or a fragment thereof. For class I epitopes it is preferred that the type of proteasome expressed by the cell be determined, for example, by western blotting. The MHC epitopes produced can then be eluted from either solubilized and purified MHC as described in Falk, K. et al. Nature 351:290, 1991, or directly from the intact cell as described in U.S. Patent 5,989,565. Eluted fragments are then identified by mass spectrometry.

### Analysis of Target Protein Fragments

The molecular species detected by mass spectrometry are compared with the candidate peptides predicted above. For the case of class I epitopes, species that are as long as, or longer than, a candidate peptide and share its C-terminus are desired; N-terminal trimming of at least up to 25 amino acids can occur independently of the proteasome (Craiu, A. et al. Proc. Natl. Acad. Sci. USA 94:10850-55, 1997). Class II MHC is highly tolerant in terms of the length of the peptides it will bind, so the absence of cleavage in the middle of the epitope becomes the primary criterion, rather than generation of a correct end.

A selected digestion product is then synthesized and used as a standard in an analytic method such as HPLC versus an aliquot of the digest. This provides a further check on the identity of the digestion product and allows its yield to be determined. In rare cases more than one potential product may have similar enough masses and chemical characteristics that they may not be reliably differentiated by these methods. In such cases the HPLC peak can be collected and subjected to direct sequencing to confirm identity.

### Analysis of Peptides for MHC Binding

The epitope is synthesized and tested for its ability to bind a MHC receptor. For example, in one preferred assay, cells displaying the MHC I receptor can be used to measure the binding affinity of candidate peptides labeled with a radionuclide. Another preferred approach measures the ability of a peptide to bind to an MHC I receptor using a cell culture-based assay. In this assay, cells lacking transporters associated with antigen processing (TAP) are used to determine whether or not a candidate peptide has the ability to bind to the MHC I receptor. TAP⁻ cells have the phenotype in which class I MHC proteins do not always fold properly, and surface expression of MHC I is thus reduced or abolished. When the cell is flooded with exogenous peptide that can bind to the MHC I cleft, expression of the receptor is restored. This can be monitored by several means such as RIA, FACS, and the like. Using TAP⁻ cells, one of skill in the art can screen large numbers of potential candidate peptides for receptor binding without having to perform detailed binding affinity analysis.

The analysis methods of the various embodiments of the invention are useful in examining candidate peptides generated in a variety of ways. For example, the described analysis can be used in evaluating multiple candidate peptides generated through *in vitro* methods or by computational analysis, to identify those candidate sequences that have MHC receptor binding characteristics. Preferred candidate peptides in this embodiment of the invention are those that are already known to be products of proteolytic production by housekeeping and/or immune proteasomes. Both *in vivo* cleavage products and *in vitro* cleavage products that are shown or predicted to bind to MHC are properly designated as "discovered epitopes." Epitope clusters for use in connection with this invention are disclosed herein.

### ECRs are Processed into MHC-Binding Epitopes in pAPCs

The immune system constantly surveys the body for the presence of foreign antigens, in part through the activity of pAPCs. The pAPCs endocytose matter found in the extracellular milieu, process that matter from a polypeptide form into shorter oligopeptides of about 3 to 23 amino acids in length, and display some of the resulting peptides to T cells via the MHC complex of the pAPCs. For example, a tumor cell upon lysis releases its cellular contents, including various proteins, into the extracellular milieu. Those released proteins can be endocytosed by pAPCs and processed into discrete peptides that are then displayed on the surface of the pAPCs via the MHC. By this mechanism, it is not the entire target protein that is presented on the surface of the pAPCs, but rather only one or more discrete fragments of that protein that are presented as MHC-binding epitopes. If a presented epitope is recognized by a T cell, that T cell is activated and an immune response results.

Similarly, the scavenger receptors on pAPC can take-up naked nucleic acid sequences or recombinant organisms containing target nucleic acid sequences. Uptake of the nucleic acid sequences into the pAPC subsequently results in the expression of the encoded products. As above, when an ECR can be processed into one or more useful epitopes, these products can be presented as MHC epitopes for recognition by T cells.

MHC-binding epitopes are often distributed unevenly throughout a protein sequence in clusters. Embodiments of the invention are directed to identifying epitope cluster regions (ECRs) in a particular region of a target protein. Candidate ECRs are likely to be natural substrates for various proteolytic enzymes and are likely to be processed into one or more epitopes for MHC display on the surface of an pAPC. In contrast to more traditional vaccines that deliver whole proteins or biological agents, ECRs can be administered as vaccines, resulting in a high probability that at least one epitope will be presented on MHC without requiring the use of a full length sequence.

### The Use of ECRs in Identifying Discrete MHC-Binding Epitopes

Identifying putative MHC epitopes for use in vaccines often includes the use of available predictive algorithms that analyze the sequences of proteins or genes to predict binding affinity of peptide fragments for MHC. These algorithms rank putative epitopes according to predicted affinity or other characteristics associated with MHC binding. Exemplary algorithms for this kind of analysis include the Rammensee and NIH (Parker) algorithms. However, identifying epitopes that are naturally present on the surface of cells from among putative epitopes predicted using these algorithms has proven to be a difficult and laborious process. The use of ECRs in an epitope identification process can enormously simplify the task of identifying discrete MHC binding epitopes.

In a preferred embodiment, ECR polypeptides are synthesized on an automated peptide synthesizer and these ECRs are then subjected to *in vitro* digests using proteolytic enzymes involved in processing proteins for presentation of the epitopes. Mass spectrometry and/or analytical HPLC are then used to identify the digest products and *in vitro* MHC binding studies are used to assess the ability of these products to actually bind to MHC. Once epitopes contained in ECRs have been shown to bind MHC, they can be incorporated into vaccines or used as diagnostics, either as discrete epitopes or in the context of ECRs.

The use of an ECR (which because of its relatively short sequence can be produced through chemical synthesis) in this preferred embodiment is a significant improvement over what otherwise would require the use of whole protein. This is because whole proteins have to be produced using recombinant expression vector systems and/or complex purification procedures. The simplicity of using chemically synthesized ECRs enables the analysis and identification of large numbers of epitopes, while greatly reducing the time and expense of the process as compared to other currently used methods. The use of a defined ECR also greatly simplifies mass spectrum analysis of the digest, since the products of an ECR digest are a small fraction of the digest products of a whole protein.

In another embodiment, nucleic acid sequences encoding ECRs are used to express the polypeptides in cells or cell lines to assess which epitopes are presented on the surface. A variety of means can be used to detect the epitope on the surface. Preferred embodiments involve the lysis of the cells and affinity purification of the MHC, and subsequent elution and analysis of peptides from the MHC; or elution of epitopes from intact cells; (Falk, K. et al. Nature 351:290, 1991, and U.S. Patent 5,989,565, respectively). A sensitive method for analyzing peptides eluted in this way from the MHC employs capillary or nanocapillary HPLC ESI mass spectrometry and on-line sequencing.

### Target-Associated Antigens that Contain ECRs

TAAs from which ECRs may be defined include those from TuAAs, including oncofetal, cancer-testis, deregulated genes, fusion genes from errant translocations, differentiation antigens, embryonic antigens, cell cycle proteins, mutated tumor suppressor genes, and overexpressed gene products, including oncogenes. In addition, ECRs may be derived from virus gene products, particularly those associated with viruses that cause chronic diseases or are oncogenic, such as the herpes viruses, human papilloma viruses, human immunodeficiency virus, and human T cell leukemia virus. Also ECRs may be derived from gene products of parasitic organisms, such as *Trypanosoma, Leishmania,* and other intracellular or parasitic organisms.

Some of these TuAA include α-fetoprotein, carcinoembryonic antigen (CEA), esophageal cancer derived NY-ESO-1, and SSX genes, SCP-1, FRAME, MART-1/MelanA (MART-1), gp100 (Pmel 17), tyrosinase, TRP-1, TRP-2, MAGE-1, MAGE-2, MAGE-3, BAGE, GAGE-1, GAGE-2, p15; overexpressed oncogenes and mutated tumor-suppressor genes such as p53, Ras, HER-2/neu; unique tumor antigens resulting from chromosomal translocations such as BCR-ABL, E2A-PRL, H4-RET, IGH-IGK, MYL-RAR1 and viral antigens, EBNA1, EBNA2, HPV-E6, -E7; prostate specific antigen (PSA), prostate stem cell antigen (PSCA), MAAT-1, GP-100, TSP-180, MAGE-4, MAGE-5, MAGE-6, RAGE, p185erbB-2, p185erbB-3, c-met, nm-23H1, TAG-72, CA 19-9, CA 72-4, CAM 17.1, NuMa, K-ras, -Catenin, CDK4, Mum-1, p15, and p16.

Numerous other TAAs are also contemplated for both pathogens and tumors. In terms of TuAAs, a variety of methods are available and well known in the art to identify genes and gene products that are differentially expressed in neoplastic cells as compared to normal cells. Examples of these techniques include differential hybridization, including the use of microarrays; subtractive hybridization cloning; differential display, either at the level of mRNA or protein expression; EST sequencing; and SAGE (sequential analysis of gene expression). These nucleic acid techniques have been reviewed by Carulli, J.P. et al., J. Cellular Biochem Suppl. 30/31:286-296, 1998. Differential display of proteins involves, for example, comparison of two-dimensional poly-acrylamide gel electrophoresis of cell lysates from tumor and normal tissue, location of protein spots unique or overexpressed in the tumor, recovery of the protein from the gel, and identification of the protein using traditional biochemical- or mass spectrometry-based sequencing. An additional technique for identification of TAAs is the Serex technique, discussed in Türeci, Ö., Sahin, U., and Pfreundschuh, M., "Serological analysis of human tumor antigens: molecular definition and implications", Molecular Medicine Today, 3:342, 1997.

Use of these and other methods provides one of skill in the art the techniques necessary to identify genes and gene products contained within a target cell that may be used as potential candidate proteins for generating the epitopes of the invention disclosed. However, it is not necessary, in practicing the invention, to identify a novel TuAA or TAA. Rather, embodiments of the invention make it possible to identify ECRs from any relevant protein sequence, whether the sequence is already known or is new.

### Protein Sequence Analysis to Identify Epitope Clusters

In preferred embodiments of the invention, identification of ECRs involves two main steps: (1) identifying good putative epitopes; and (2) defining the limits of any clusters in which these putative epitopes are located. There are various preferred embodiments of each of these two steps, and a selected embodiment for the first step can be freely combined with a selected embodiment for the second step. The methods and embodiments that are disclosed herein for each of these steps are merely exemplary, and are not intended to limit the scope of the invention in any way. Persons of skill in the art will appreciate the specific tools that can be applied to the analysis of a specific TAA, and such analysis can be conducted in numerous ways in accordance with the invention.

Preferred embodiments for identifying good putative epitopes include the use of any available predictive algorithm that analyzes the sequences of proteins or genes to predict binding affinity of peptide fragments for MHC, or to rank putative epitopes according to predicted affinity or other characteristics associated with MHC binding. As described above, available exemplary algorithms for this kind of analysis include the Rammensee and NIH (Parker) algorithms. Likewise, good putative epitopes can be identified by direct or indirect assays of MHC binding. To choose "good" putative epitopes, it is necessary to set a cutoff point in terms of the score reported by the prediction software or in terms of the assayed binding affinity. In some embodiments, such a cutoff is absolute. For example, the cutoff can be based on the measured or predicted half time of dissociation between an epitope and a selected MHC allele. In such cases, embodiments of the cutoff can be any half time of dissociation longer than, for example, 0.5 minutes; in a preferred embodiment longer than 2.5 minutes; in a more preferred embodiment longer than 5 minutes; and in a highly stringent embodiment can be longer than 10, or 20, or 25 minutes. In these embodiments, the good putative epitopes are those that are predicted or identified to have good MHC binding characteristics, defined as being on the desirable side of the designated cutoff point. Likewise, the cutoff can be based on the measured or predicted binding affinity between an epitope and a selected MHC allele. Additionally, the absolute cutoff can be simply a selected number of putative epitopes.

In other embodiments, the cutoff is relative. For example, a selected percentage of the total number of putative epitopes can be used to establish the cutoff for defining a candidate sequence as a good putative epitope. Again the properties for ranking the epitopes are derived from measured or predicted MHC binding; the property used for such a determination can be any that is relevant to or indicative of binding. In preferred embodiments, identification of good putative epitopes can combine multiple methods of ranking candidate sequences. In such embodiments, the good epitopes are typically those that either represent a consensus of the good epitopes based on different methods and parameters, or that are particularly highly ranked by at least one of the methods.

When several good putative epitopes have been identified, their positions relative to each other can be analyzed to determine the optimal clusters for use in vaccines or in vaccine design. This analysis is based on the density of a selected epitope characteristic within the sequence of the TAA. The regions with the highest density of the characteristic, or with a density above a certain selected cutoff, are designated as ECRs. Various embodiments of the invention employ different characteristics for the density analysis. For example, one preferred characteristic is simply the presence of any good putative epitope (as defined by any appropriate method). In this embodiment, all putative epitopes above the cutoff are treated equally in the density analysis, and the best clusters are those with the highest density of good putative epitopes per amino acid residue. In another embodiment, the preferred characteristic is based on the parameter(s) previously used to score or rank the putative epitopes. In this embodiment, a putative epitope with a score that is twice as high as another putative epitope is doubly weighted in the density analysis, relative to the other putative epitope. Still other embodiments take the score or rank into account, but on a diminished scale, such as, for example, by using the log or the square root of the score to give more weight to some putative epitopes than to others in the density analysis.

Depending on the length of the TAA to be analyzed, the number of possible candidate epitopes, the number of good putative epitopes, the variability of the scoring of the good putative epitopes, and other factors that become evident in any given analysis, the various embodiments of the invention can be used alone or in combination to identify those ECRs that are most useful for a given application. Iterative or parallel analyses employing multiple approaches can be beneficial in many cases. ECRs are tools for increased efficiency of identifying true MHC epitopes, and for efficient "packaging" of MHC epitopes into vaccines. Accordingly, any of the embodiments described herein, or other embodiments that are evident to those of skill in the art based on this disclosure, are useful in enhancing the efficiency of these efforts by using ECRs instead of using complete TAAs in vaccines and vaccine design.

Since many or most TAAs have regions with low density of predicted MHC epitopes, using ECRs provides a valuable methodology that avoids the inefficiencies of including regions of low epitope density in vaccines and in epitope identification protocols. Thus, useful ECRs can also be defined as any portion of a TAA that is not the whole TAA, wherein the portion has a higher density of putative epitopes than the whole TAA, or than any regions of the TAA that have a particularly low density of putative epitopes. In this aspect of the invention, therefore, an ECR can be any fragment of a TAA with elevated epitope density. In some embodiments, an ECR can include a region up to about 80% of the length of the TAA. In a preferred embodiment, an ECR can include a region up to about 50% of the length of the TAA. In a more preferred embodiment, an ECR can include a region up to about 30 % of the length of the TAA. And in a most preferred embodiment, an ECR can include a region of between 5 and 15% of the length of the TAA.

In another aspect of the invention, the ECR can be defined in terms of its absolute length. Accordingly, by this definition, the minimal cluster for 9-mer epitopes includes 10 amino acid residues and has two overlapping 9-mers with 8 amino acids in common. In a preferred embodiment, the cluster is between about 15 and 75 amino acids in length. In a more preferred embodiment, the cluster is between about 20 and 60 amino acids in length. In a most preferred embodiment, the cluster is between about 30 and 40 amino acids in length.

In practice, as described above, ECR identification can employ a simple density function such as the number of epitopes divided by the number of amino acids spanned by the those epitopes. It is not necessarily required that the epitopes overlap, but the value for a single epitope is not significant. If only a single value for a percentage cutoff is used and an absolute cutoff in the epitope prediction is not used, it is possible to set a single threshold at this step to define a cluster. However, using both an absolute cutoff and carrying out the first step using different percentage cutoffs, can produce variations in the global density of candidate epitopes. Such variations can require further accounting or manipulation. For example, an overlap of 2 epitopes is more significant if only 3 candidate epitopes were considered, than if 30 candidates were considered for any particular length protein. To take this feature into consideration, the weight given to a particular cluster can further be divided by the fraction of possible peptides actually being considered, in order to increase the significance of the calculation. This scales the result to the average density of predicted epitopes in the parent protein.

Similarly, some embodiments base the scoring of good putative epitopes on the average number of peptides considered per amino acid in the protein. The resulting ratio represents the factor by which the density of predicted epitopes in the putative cluster differs from the average density in the protein. Accordingly, an ECR is defined in one embodiment as any region containing two or more predicted epitopes for which this ratio exceeds 2, that is, any region with twice the average density of epitopes. In other embodiments, the region is defined as an ECR if the ratio exceeds 1.5, 3, 4, or 5, or more.

Considering the average number of peptides per amino acid in a target protein to calculate the presence of an ECR highlights densely populated ECRs without regard to the score/affinity of the individual constituents. This is most appropriate for use of score-based cutoffs. However, an ECR with only a small number of highly ranked candidates can be of more biological significance than a cluster with several densely packed but lower ranking candidates, particularly if only a small percentage of the total number of candidate peptides were designated as good putative epitopes. Thus in some embodiments it is appropriate to take into consideration the scores of the individual peptides. This is most readily accomplished by substituting the sum of the scores of the peptides in the putative cluster for the number of peptides in the putative cluster in the calculation described above.

This sum of scores method is more sensitive to sparsely populated clusters containing high scoring epitopes. Because the wide range of scores (i.e. half times of dissociation) produced by the BIMAS-NIH/Parker algorithm can lead to a single high scoring peptide dwarfing the contribution of other potential epitopes, the log of the score rather than the score itself is preferably used in this procedure.

Various other calculations can be devised under one or another condition. Generally speaking, the epitope density function is constructed so that it is proportional to the number of predicted epitopes, their scores, their ranks, and the like, within the putative cluster, and inversely proportional to the number of amino acids or fraction of protein contained within that putative cluster. Alternatively, the function can be evaluated for a window of a selected number of contiguous amino acids. In either case the function is also evaluated for all predicted epitopes in the whole protein. If the ratio of values for the putative cluster (or window) and the whole protein is greater than, for example, 1.5, 2, 3, 4, 5, or more, an ECR is defined.

### Analysis of Target Gene Products For MHC Binding

Once a TAA has been identified, the protein sequence can be used to identify putative epitopes with known or predicted affinity to the MHC peptide binding cleft. Tests of peptide fragments can be conducted *in vitro,* or using the sequence can be computer analyzed to determine MHC receptor binding of the peptide fragments. In one embodiment of the invention, peptide fragments based on the amino acid sequence of the target protein are analyzed for their predicted ability to bind to the MHC peptide binding cleft. Examples of suitable computer algorithms for this purpose include the Rammensee/SYFPEITHI and the NIH (Parker) sites referenced in the discussion of epitope discovery above.

As an alternative to predictive algorithms, a number of standard *in vitro* receptor binding affinity assays are available to identify peptides having an affinity for a particular allele of MHC. Accordingly, by the method of this aspect of the invention, the initial population of peptide fragments can be narrowed to include only putative epitopes having an actual or predicted affinity for the selected allele of MHC. Selected common alleles of MHC, and their approximate frequencies, are reported in the tables 3-5 above.

It has been observed that predicted epitopes often cluster at one or more particular regions within the amino acid sequence of a TAA. The identification of such ECRs offers a simple and practicable solution to the problem of designing effective vaccines for stimulating cellular immunity. For vaccines in which immune epitopes are desired, an ECR is directly useful as a vaccine. This is because the immune proteasomes of the pAPCs can correctly process the cluster, liberating one or more of the contained MHC-binding peptides, in the same way a cell having immune proteasomes processes and presents peptides derived from the complete TAA. The cluster is also a useful a starting material for identification of housekeeping epitopes produced by the housekeeping proteasomes active in peripheral cells.

### Additional Considerations for Vaccine Design

There exist numerous alleles of MHC I in the human population. Thus, in a preferred embodiment, vaccine design can take into account the MHC I genotype of the patient, so as to deliver epitopes having suitable binding affinities for a particular patient's MHC allele(s). Since a patient may be homozygous or heterozygous for the relevant locus, in some embodiments of the invention, epitopes optimal for a single MHC I allele are preferred, while in other embodiments, epitopes corresponding to different MHC alleles may be preferred. A partial list of major class I MHC types, each generally encoded by multiple alleles, and their approximate frequencies, are reported in Table 6.

**Table 6**

| **HLA type** | **Frequency in mixed-race population** |
|---|---|
| **A1** | **30%** |
| **A2** | **47%** |
| **A3** | **23%** |
| **A11** | **15%** |
| **A24** | **14%** |
| **A29** | **7%** |
| **A26** | **6%** |
| **B7** | **22%** |
| **B8** | **21%** |
| **B14** | **9%** |
| **B18** | **10%** |
| **B27** | **8%** |
| **B35** | **20%** |
| **B44** | **26%** |
| **B62** | **14%** |
| **B60** | **10%** |
| **B51** | **10%** |

In yet another embodiment of the present invention, the pAPCs are provided with a housekeeping epitope and an epitope cluster. The epitope cluster is a peptide or nucleic acid sequence that contains or encodes at least two sequences having a known or predicted affinity to MHC I. While it is preferable that the housekeeping epitope be provided to the pAPCs in a state that is fully processed or as a precursor that is engineered in such a way so that it can be processed in the pAPC to be an effective housekeeping epitope, the immune epitope can be processed from a larger precursor by the pAPCs. This is because the immune proteasome is constitutively active in the pAPC, and is fully competent to process an appropriate precursor of presumably any length into a "correct" immune epitope.

Potential epitopes are commonly but not always found in clusters in discrete segments of a TAA containing multiple epitopes for the purpose of providing an immune epitope. Simply providing the pAPC with a polypeptide containing a cluster of potential epitopes, or a nucleic acid encoding a cluster, or a recombinant organism expressing the cluster enables the pAPC to produce at least one appropriate immune epitope. Since epitope clusters generally contain potential epitopes for more than one class I MHC allele, in many embodiments a single cluster can be used to produce immune epitopes useful with more than one class I MHC allele.

In a preferred embodiment, a patient is inoculated with a vaccine that includes housekeeping epitopes derived from a selected TAA. The housekeeping epitope can be a polypeptide or a nucleic acid encoding a polypeptide, or a recombinant organism engineered to express the discrete epitope. Beyond this "minimal" vaccine containing a housekeeping epitope (whether as a polypeptide, a nucleic acid, or recombinant organism), embodiments of the invention include vaccines that additionally have one or more other housekeeping epitopes, or one or more immune epitopes, or any combination thereof. Such epitopes can be derived from the same TAA, or they can be derived from different TAAs.

A preferred embodiment of the present invention includes a method of administering a vaccine including a housekeeping epitope to induce a therapeutic immune response. The vaccine is administered to a patient in a manner consistent with the standard vaccine delivery protocols that are well known in the art. Methods of administering epitopes of TAAs include, without limitation, transdermal, intranodal, perinodal, oral, intravenous, intradermal, intramuscular, intraperitoneal, and mucosal administration. A particularly useful method of vaccine delivery to elicit a CTL response is disclosed in PCT Publication No. WO 99/01283, entitled "A METHOD OF INDUCING A CTL RESPONSE," filed on July 10, 1998.

Because the epitope synchronization system has utility in inducing a cell-mediated immune response, a vaccine to induce a specific T cell response to a target cell is likewise included in a preferred embodiment of the present invention. The vaccine contains a housekeeping epitope in a concentration effective to cause a pAPC or populations of pAPCs to display housekeeping epitopes. Advantageously, the vaccine can include a plurality of housekeeping epitopes or one or more housekeeping epitopes optionally in combination with one or more immune epitopes. Formulations of the vaccine contain peptides and/or nucleic acids in a concentration sufficient to cause pAPCs to present the epitopes. The formulations preferably contain epitopes in a total concentration of about 1µg-1mg/100µl of vaccine preparation. Conventional dosages and dosing for peptide vaccines and/or nucleic acid vaccines can be used with the present invention, and such dosing regimens are well understood in the art. In one embodiment, a single dosage for an adult human may advantageously be from about 1 to about 5000 µl of such a composition, administered one time or multiple times, e.g., in 2, 3, 4 or more dosages separated by 1 week, 2 weeks, 1 month, or more. In a particularly preferred embodiment, such a composition is administered continuously, directly into a lymph node, through the use of an insulin pump, at a rate of at least 1 µl per hour over several days. Such administration can be repeated periodically to maintain the CTL response as is more fully described in PCT Publication No. WO 99/01283.

The compositions and methods of the invention disclosed herein further contemplate incorporating adjuvants into the formulations in order to enhance the performance of the vaccines. Specifically, the addition of adjuvants to the formulations is designed to enhance the delivery or uptake of the epitopes by the pAPCs. The adjuvants contemplated by the present invention are known by those of skill in the art and include, for example, GMCSF, GCSF, IL-2, IL-12, BCG, tetanus toxoid, and osteopontin/ETA-1.

In a further embodiment, housekeeping epitope-reactive T cells can be administered to a patient as an adoptive immunotherapy. Such T cells can be most readily obtained by in vitro immunization, using cells from a naïve donor, though using the patient as donor can also be feasible. Techniques for in vitro immunization are known in the field, for example, Stauss et al., Proc. Natl. Acad. Sci. USA 89:7871-7875, 1992; Salgaller et al. Cancer Res. 55:4972-4979, 1995; Tsai et al., J. Immunol. 158:1796-1802, 1997; and Chung et al., J. Immunother. 22:279-287, 1999. Use of immunized donors, or patients themselves, as initial sources of T cells is also contemplated. (See for example Oelke, M. et al. Clin Cancer Res. 6:1997-2005, 2000; Gervois, N. et al. Clin Cancer Res. 6:1459-1467, 2000; Valmori, D. et al. Cancer Res. 59:2167-3173, 1999; Tsai, V. et al. Crit. Rev. Immunol. 18:65-75, 1998; Matsunaga, K. et al. Jpn. J. Cancer Res. 90:1007-1015, 1999; van Elsas, A. et al. Eur J. Immunol. 26:1683-1689, 1996; and Alters, S.E. et al. Adv. Exp. Med. Biol. 417:519-524, 1997). Once generated, sufficient numbers of such T cells can be obtained by expansion in vitro through stimulation with the vaccines of this invention and/or cytokines (see for example Kurokawa, T. et al., Int. J. Cancer 91:749-746, 2001). These T cells can constitute a clone or a polyclonal population recognizing one or more epitopes. Typically, on the order of 10⁵ to 10⁸ cells are transferred in mice and 10⁸ to 10¹¹ in humans. (See for example Drobyski, W.R. et al. Blood 97:2506-2513, 2001; Seeley B.M. et al. Otolazyngol. Head Neck Surg. 124:436-441, 2001; Kanwar, J.R. et al. Cancer Res. 61:1948-1956, 2001; Plautz, G.E. et al. Clin. Cancer Res. 6:2209-2218, 2000; Plautz, G.E. et al., J. Neurosurg. 89:42-51, 1998; and Plautz, G.E. et al., Urology 54:617-623, 1999). Clones and otherwise more enriched populations generally require the transfer of fewer cells. The epitopes recognized can be housekeeping epitopes or a combination of housekeeping and immune epitopes. It is also envisioned that genetic engineering can be used to express cloned TCRs in a cell line suitable for use in adoptive immunotherapy. Examples of sources from which useful TCRs can be cloned include the T cells described above, and HLA-transgenic mice immunized with the vaccines of this invention. Additional variations will be apparent to one of skill in the art.

In some embodiments of the invention, the vaccines can include a recombinant organism, such as a virus, bacterium or parasite, genetically engineered to express an epitope in a host. For example, *Listeria monocytogenes,* a gram-positive, facultative intracellular bacterium, is a potent vector for targeting TuAAs to the immune system. In a preferred embodiment, this vector can be engineered to express a housekeeping epitope to induce therapeutic responses. The normal route of infection of this organism is through the gut and can be delivered orally. In another embodiment, an adenovirus (Ad) vector encoding a housekeeping epitope for a TuAA can be used to induce anti-virus or anti-tumor responses. Bone marrow-derived dendritic cells can be transduced with the virus construct and then injected, or the virus can be delivered directly via subcutaneous injection into an animal to induce potent T-cell responses. Another embodiment employs a recombinant vaccinia virus engineered to encode amino acid sequences corresponding to a housekeeping epitope for a TAA. Vaccinia viruses carrying constructs with the appropriate nucleotide substitutions in the form of a minigene construct can direct the expression of a housekeeping epitope, leading to a therapeutic T cell response against the epitope.

Particularly useful nucleic acid constructs useful as vaccines in accordance with the present invention are disclosed herein.

### Epitope-Encoding Vector Constructs

The present invention provides nucleic acid constructs for use as therapeutic vaccines. The constructs include a coding region having a sequence that encodes a polypeptide. The polypeptide is an epitope of a TAA. In one embodiment, the target cell is a neoplastic cell and the polypeptide is an epitope or precursor of an epitope of a TuAA. In another embodiment, the target cell is any cell infected with an intracellular parasite. The term "parasite" as used herein includes any organism or infective agent such as a virus that has an intracellular stage of infection within the host. These include but are not limited to: viruses such as adenovirus, cytomegalovirus, Epstein-Barr virus, herpes simplex virus 1, herpes simplex virus 2, human herpesvirus 6, yaricella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, and human T cell leukemia virus II; bacteria such as *Chlamydia, Listeria, Salmonella, Legionella, Brucella, Coxiella, Rickettsia, Mycobacterium;* and protozoa such as *Leishmania, Trypanasoma, Toxoplasma,* and *Plasmodium.*

The polypeptide(s) encoded by the nucleic acid construct can include a housekeeping epitope of a TAA. In preferred embodiments, the nucleic acid construct encodes a plurality of housekeeping epitopes. When the construct encodes such a plurality, the multiple epitopes can all correspond to different segments of a single TAA, or they can correspond to different TAAs. In a preferred embodiment, the nucleic acid construct contains a housekeeping epitope and an immune epitope. In another preferred embodiment, the nucleic acid construct contains a housekeeping epitope and an epitope cluster region.

In preferred embodiments, wherein the construct of the vaccine encodes both a housekeeping epitope and an immune epitope, the vaccine can stimulate a cellular immune response against target cells presenting either epitope--that is, the immune response can recognize the housekeeping epitopes displayed initially by the target cells, and then can also recognize the immune epitopes presented by the target cells after induction by IFN.

Advantageously, the nucleic acid construct can further include a third or fourth sequence, or more, with such sequences encoding a third or fourth epitope, or additional epitopes, respectively. Such epitopes can be derived from a single TAA or from two or more different TAAs, and can be housekeeping or immune epitopes on any combination. The constructs can be designed to encode epitopes corresponding to any other proteasome activities that may play a role in processing antigens in any target cell or pAPC.

The encoded MHC epitopes are preferably about 7-15 amino acids in length, and more preferably, 9 or 10 amino acids in length. While the generally preferred peptide size for MHC I binding is 9 amino acids, shorter and longer peptides may also in some cases bind MHC I. Likewise, many peptides much longer than 9 amino acids can be trimmed by exopeptidases or other proteases resident in the cell, to produce fragments that bind MHC I very effectively. The size of a peptide containing an immune epitope sequence is not critical, so long as the sequence includes the epitope. This is because the immune proteasome, resident in the pAPC, in combination with trimming exopeptidases and other proteases, in its normal function correctly processes full length TAAs to produce immune epitopes. Thus, the nucleic acid sequence encoding the immune epitope can actually encode a much larger precursor, including the complete TAA. Such a construct preferably also encodes a housekeeping epitope.

Examples of TuAAs and other TAAs suitable for use in the present invention include but are not limited to: differentiation antigens such as MelanA (MART-I), gp100 (Pmel 17), tyrosinase, TRP-1, TRP-2, and tumor-specific multilineage antigens such as MAGE-1, MAGE-3, BAGE, GAGE-1, GAGE-2, CEA, RAGE, NY-ESO, SCP-1, Hom/Mel-40 and PRAME. Similarly, TuAAs include overexpressed oncogenes, and mutated tumor-suppressor genes such as p53, H-Ras and HER-2/neu. Additionally, unique TuAAs resulting from chromosomal translocations such as BCR-ABL, E2A-PRL, H4-RET, IGH-IGK, MYL-RAR and viral antigens such as Epstein Barr virus antigens EBNA, and the human papillomavirus (HPV) antigens E6 and E7 are included. Other useful protein antigens include but are not limited to TSP-180, MAGE-4, MAGE-5, MAGE-6, RAGE, NY-ESO, p185erbB2, p180erbB-3, c-met, nm-23H1, PSA, TAG-72-4, CAM 17.1, NuMa, K-ras, β-Catenin, CDK4, Mum-1, and p16. These and other TuAAs and pathogen-related antigens are known and available to those of skill in the art in the literature or commercially.

In a further embodiment, the TAA is an antigen specific for a virus. See Table 2 above. In yet another embodiment of the present invention, the TAA is an antigen specific for a non-viral intracellular parasite. Examples of parasite-specific antigens include nucleotides, proteins, or other gene products associated with the intracellular parasite. Suitable nucleotides or proteins can be found at the NCBI Taxonomy Database located at http://www.ncbi.nhn.nih.gov/Taxonomy/tax.html/. More detailed descriptions of gene products for parasites and other pathogens are provided at this web site.

Particularly preferred peptides are about 7 - 15 amino acids in length. An extensive listing of peptides having MHC binding motifs is provided in Han-Georg Rammensee, Jutta Bachmann, and Stefan Stevanovic, "MHC Ligands and Peptide Motifs," Springer-Verlag, Germany, (1997) Landes Bioscience, Austin, Texas.

The epitopes encoded by the constructs have affinity to one or more MHC I alleles. In some embodiments, wherein a patient is heterozygous for MHC I, the construct can encode epitopes corresponding to different MHC I alleles.

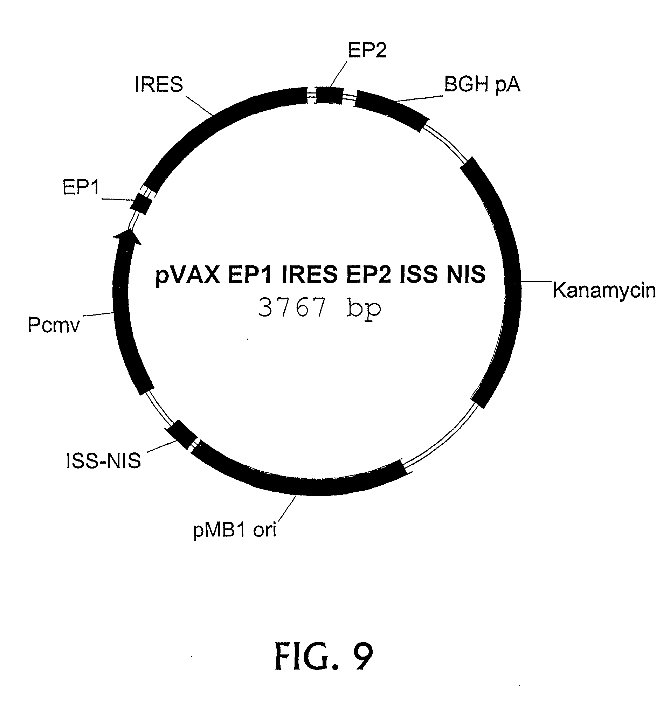

Preferred nucleic acid constructs include at least one promoter sequence that is operably linked to the 5' end of the coding region of the construct. It will be appreciated by those of skill in the art that any promoter active in mammalian cells can be employed. Preferred promoter sequences include, but are not limited to, the CMV promoter, the SV40 promoter, and retroviral LTR promoter sequences, and can also include EF-1A, UbC, β-actin promoters. In some embodiments, the constructs can include two or more promoters that are operably linked to the 5' end of different polypeptide-encoding sequences. Likewise, the constructs can employ enhancers, nuclear import sequences, immunostimulatory sequences, and expression cassettes for cytokines, selection markers, reporter molecules, and the like. Moreover, immunostimulatory, or other modulatory sequences can be attached to the vector via a stably hybridized PNA peptide nucleic acid. In preferred embodiments, the nucleic acid constructs of the present invention also include a poly-A sequence that is operably linked to a 3' end of the coding region. A nucleic acid construct that includes a nuclear import sequence and an immunostimulatory sequence is depicted in Figure 9.

In certain embodiments, the nucleic acid constructs encode an mRNA that is translated as a single polypeptide and then cleaved. In one such embodiment the polypeptide consists of a linear array of epitopes, wherein the first (N-terminal) sequence is one or more immune epitopes or epitope clusters, and the second (C-terminal) sequence is a housekeeping epitope, such that the correct C-terminus of the housekeeping epitope is specified by the termination codon, and all other HLA epitope termini are determined by proteasomal processing and exopeptidase trimming.

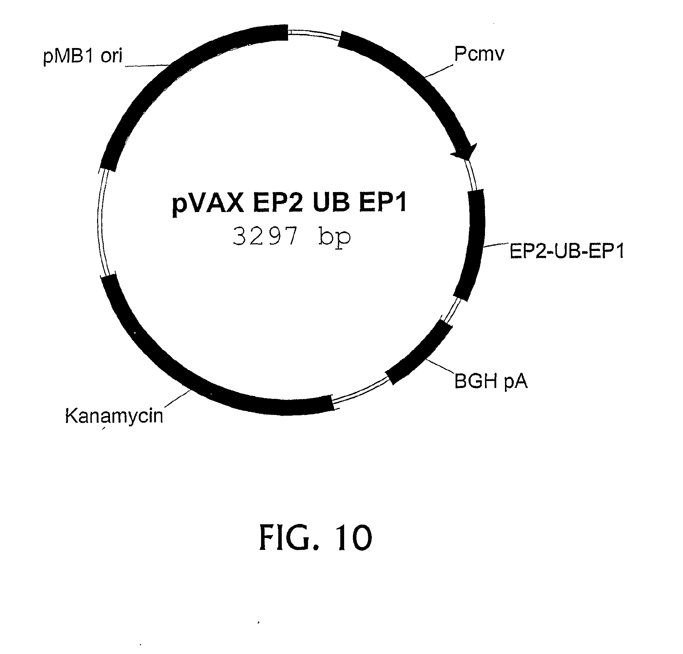

In another preferred embodiment, the nucleic acid construct encodes an amino acid sequence wherein an immune epitope or an epitope cluster is linked to a ubiquitin sequence. The ubiquitin sequence is similarly linked to a housekeeping epitope. The presence of ubiquitin between the epitopes facilitates efficient delivery of the immune epitope to the proteasome for epitope processing. The ubiquitin sequence (with or without an N-terminal spacer to ensure the integrity of the preceding peptide) is located in frame between the first and second sequence, or between any other epitope-encoding sequences. The so produced Sequence1-Ubiquitin-Sequence2 polypeptide is rapidly (co-translationally) cleaved at the Ubiquitin-Sequence2 junction by Ubiquitin-specific processing proteases, producing Sequence1-Ubiquitin and Sequence2. (See Figure 10)

Physiologically, ubiquitin serves primarily as a signal that targets protein for degradation by the proteasome. It is among the most conserved proteins in eukaryotes, with only three conservative amino acid substitutions between yeast and human. Although the precise sequence of ubiquitin may vary somewhat, the sequence of the preferred embodiment is represented by SEQ ID NO: 5. Ubiquitin is a 76 amino acid long polypeptide having two crucial features: 1) a C-terminal Gly residue, involved in the conjugation of ubiquitin to the Lys side chain of protein substrates and 2) a Lys residue, at position 48, for the formation of multi-ubiquitin chains.

Ubiquitin genes are unique in the sense that all of them are synthesized as fusions to other polypeptides, including other ubiquitins. In the yeast *S*. *cerevisiae,* four ubiquitin genes have been identified: whereas the first three (UBI1-3) are fused to ribosomal proteins, the fourth gene (UBI4) is synthesized as a fusion of five identical repeats of the ubiquitin sequence. Thus, functional free ubiquitin is naturally produced after co-translational proteolytic processing by ubiquitously expressed ubiquitin-specific hydrolases. Such a natural organization has been exploited by generating C-terminal fusions between a single ubiquitin moiety and any desired polypeptide.

Ubiquitin can exist in two conformations: the first one is described above and consists of a linear fusion of a single ubiquitin to any desired polypeptide, in which the C-terminal Gly of ubiquitin is linked, via a peptide bond to the N-terminal amino acid of the polypeptide of choice. The second involves the conjugation of a ubiquitin moiety to a protein substrate, via a Gly-Lys bond formation. In this case, the COOH group of the ubiqutin Gly is linked to the ε (epsilon) side chain of a solvent exposed Lys of the substrate (or another ubiquitin moiety). The ubiquitin signal for the degradation of the substrate is associated with the second conformation. Thus, in the Sequence1-Ubiquitin-Sequence2 construct described above, Sequence2 typically is not targeted to the proteasome. Accordingly, the Sequence2 position is preferably used for a fully processed epitope, or one needing only N-terminal trimming, typically a housekeeping epitope. The ubiquitin moiety remaining attached to Sequence 1 in the construct described above can be polyubiquitinated at Lys48, thereby targeting that fragment to the proteasome for processing, and resulting in the liberation of the epitope contained in Sequence1. It should be noted that if more than two sequences are linked together in a linear array by ubiquitin moieties, generally only the last sequence behaves in the manner of Sequence2; the processing of all of the upstream sequences resembles that of Sequence1. To the extent that the constructs described herein are expressed in pAPCs, wherein the immune proteasome is predominantly active, correct expression of housekeeping epitopes by these constructs benefits from the housekeeping epitopes being in the Sequence2 position, or a correspond position wherein the epitope does not require proteasomal processing in the pAPC.

In yet another embodiment the nucleic acid constructs of the present invention may include autoproteolytic peptide-encoding sequences. Such sequences are located between the first and second sequences or between any other epitope-encoding sequences. Examples of such autoproteolytic sequences include the inteins; also included are the 3C^{pro} and 2A^{pro} proteases of picomaviruses, including polioviruses and other enteroviruses, rhinoviruses, cardioviruses, and apthoviruses, and the equivalent cornoviridae proteases. These proteases catalyze the post-translational cleavage of the large precursor polyprotein made by this family of viruses.

In one embodiment, the autocatalytic protein sequence is inserted between two or more epitopes. In a further embodiment, the sequence is inserted after two or more epitopes, but the cleavage signal is found between the epitopes such that they are cleaved into two or more fully functional epitopes. The type of protease is not important, it is only important that the appropriate cleavage signal be available for the correct processing of the epitopes.

Because the cleavage sites and the sequences of the autocatalytic proteins are known (recently reviewed by Seipelt, J. et al., Virus Research 62:159-168, 1999) they can easily be used for construction of a vector which produces a polyprotein or biprotein. Briefly, 3C^{pro} predominantly recognizes a Q-G site as a cleavage signal although other closely adjacent positions can be important. Also the 3C^{pro} of some of these viruses adhere less closely to this general pattern, providing for a greater degree of flexibility in design. The limitation imposed by these requirements is more formal than real, particularly if the protease is placed between the epitopes to be expressed. In this arrangement an upstream immune epitope can be liberated by proteasomal processing even if the viral protease fails to cleave its N-terminus. The key residues for cleavage at the C-terminus are internal to 3C^{pro} itself, generally leaving just 1-4 residues, if any, to be removed by exopeptidase trimming from the N-terminus of a downstream housekeeping epitope. 2A^{pro} can be used much the same way with the understanding that the cleavage site, while favoring G-P, is somewhat more variable among these viruses. It must also be considered that its expression can lead to a shutdown of host cell protein synthesis with a rapidity and completeness that depend on the virus strain from which it was derived.

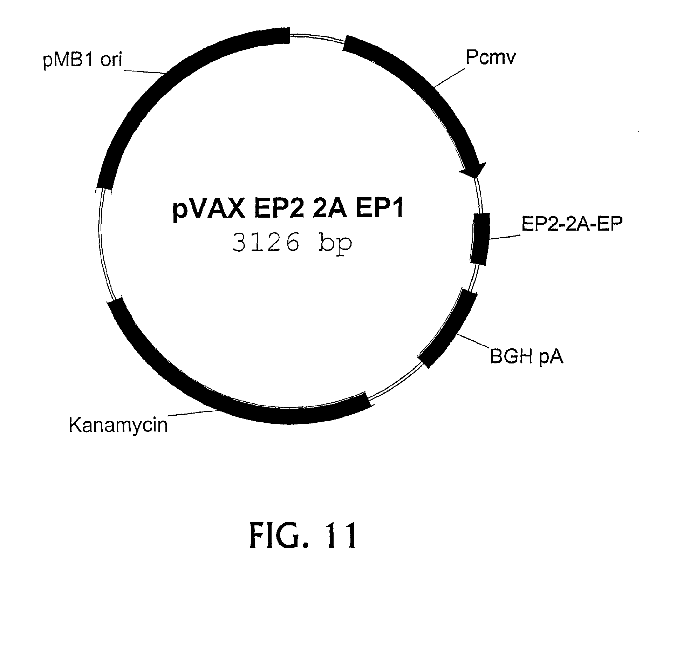

Strictly speaking, the 2A proteins from cardioviruses and apthoviruses (i.e., Foot-and-Mouth Disease Virus (FMDV)) are not proteases, but rather prevent peptide bond formation at their C-termini without causing a termination of translation (Ryan, M.D., et al., Bioorganic Chemistry 27:55-79, 1999).Thus by positioning these 2A proteins between epitopes one can cause scission within a single reading frame. The 2A protein from FMDV is very small, only 18 amino acids, making it particularly well suited to multiple epitope expression. A plasmid employing the 2A protein is depicted as Figure 11.

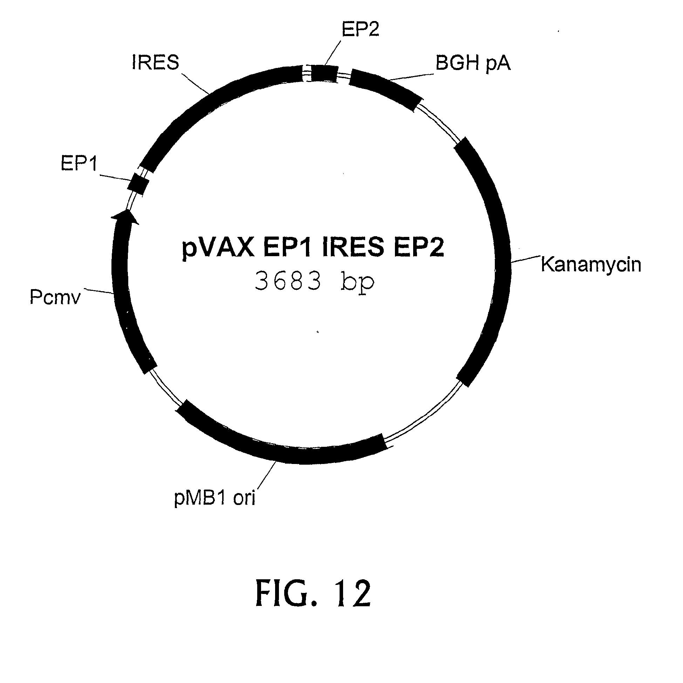

In certain other embodiments, the nucleic acid constructs encode an mRNA that is translated as two or more polypeptides. In one such embodiment the transcript can contain one or more internal ribosome entry site (IRES) sequences that are located between the first and second sequence or between any other epitope-encoding sequences. IRES sequences are naturally used by picornaviruses to direct internal cap-independent translation of mRNA. Such IRES sequences can also allow independent translation of two or more consecutive open reading frames from the same messenger RNA. Although the IRES sequences of various constructs may vary, the IRES sequence of one preferred embodiment is provided in SEQ ID NO: 6. The C-terminus of each epitope expressed is determined by termination codons. Thus the order of the sequences encoding the housekeeping epitope and the sequences encoding the immune epitope does not matter, which provides flexibility of plasmid construction. Optionally, the sequence encoding the housekeeping epitope can precede the IRES sequence and the sequence encoding the immune epitope can be linked to the other end of the IRES sequence. Such vectors can also usefully encode two or more housekeeping epitopes. They can further allow the combination of the various single polypeptide constructs described above, in order to productively express multiple epitopes. See Figure 12.

In certain other embodiments, the nucleic acid constructs encode two or more mRNA transcripts. Each of these transcripts may encode single epitopes or any of the dual or multiple epitope transcripts described in the embodiments above. Two or more transcripts can be the result of using multiple promoters. Those of skill in the art will recognize that use of more than one copy of a single promoter can lead to instability of the plasmid during propagation. Thus it will generally be preferable to use two (or more) different promoters.

Two or more transcripts can also be the result of using bidirectional promoters. Bidirectional promoters can be found in a wide variety of organisms. Examples of such promoters include PDGF-A from human, pcbAB and pcbC from *Penicillium chrysogenum,* neurotropic JC virus, and BRCA1 from mouse, dog and human. Although intensive research on bidirectional promoters began comparatively recently, there is a growing body of information on the sequence, regulation, and other intricacies of how they work. For example, the human transcobalamin II promoter requires a 69 base pair (bp) fragment containing a GC box and an E box for full transcriptional activity. The dipeptidylpeptidase IV promoter was shown to stimulate transcription from both sides with a similar efficiency. Rat mitochondrial chaperonins 60 and 10 are linked head to head and share a bidirectional promoter. Accordingly, various working bidirectional promoters have been identified, sequenced, and cloned in such a way that they can be used in a nucleic acid construct to express two genes.

Thus, in a preferred embodiment, the nucleic acid constructs contain bidirectional promoters such as, for example, those listed above, linked to a nucleic acid sequence encoding a housekeeping epitope or precursor thereof. In a particularly preferred embodiment, the nucleic acid construct contains bidirectional promoters linked to nucleic acid sequences encoding a plurality of housekeeping epitopes. In another embodiment, the nucleic acid constructs comprise bidirectional promoters linked to nucleic acid sequences encoding a housekeeping epitope and an immune epitope, or to an epitope cluster region. In addition, the bidirectional promoter may be positively or negatively regulated.

When the nucleic acid construct contains more than one epitope, the bidirectional promoter may express the plurality of epitopes in comparable amounts or some may be expressed at higher levels than the others. Alternatively, one epitope can be inducible and the other constitutive. In this way, a temporal regulation of epitope expression can be achieved, wherein one epitope is expressed early in the treatment and the other expressed later.

### Analysis of Epitope Expression

Several methods described in the literature can be used to determine if epitopes have been presented on pAPCs. An indirect but powerful method is the use of class I tetramer analysis to determine T cell frequency in an animal before and after administration of a housekeeping epitope. Clonal expansion of T cells in response to an epitope occurs preferentially only when the epitope is presented to T cells by pAPCs. Therefore, measurements of specific T cell frequency against the housekeeping epitope before and after administration of the epitope to an animal is a means of determining if the epitope is present on pAPCs. An increase in frequency of T cells specific to the epitope after administration indicates that the epitope was presented on pAPC. Other methods of determining T cell frequency such as limiting dilution analysis or ELISPOT can be used in principally the same manner to assess housekeeping epitope presentation by pAPCs. Similarly any method of determining T cell frequency in an animal may be used.

A direct method for determining housekeeping epitope presentation on pAPCs involves the purification of pAPCs from an animal after administration of an epitope. After vaccination of an animal with an housekeeping epitope, pAPCs may be harvested from PBMC, splenocytes or lymph node cells, using monoclonal antibodies against specific markers present on pAPCs and affinity purification, such as with the use of monoclonal antibodies fixed to magnetic beads. The optimal time for such harvest is variable, and can depend on the animal vaccinated, the nature of the vaccine, and other factors including dosing, site of administration, pharmacokinetics, and the like. Crude blood or splenoctye preparation can be enriched for pAPCs using this technique. The enriched pAPCs can then be used in a proliferation assay against a T cell clone that has been generated and is specific for the housekeeping epitope of interest. The pAPCs are coincubated with the T cell clone and the T cells are monitored for proliferation activity, such as by measuring the incorporation of radiolabeled thymidine by T cells. Proliferation indicates that T cells specific for the housekeeping epitope are being stimulated by that epitope on the pAPCs.

The following examples are intended for illustration purposes only, and should not be construed as limiting the scope of the invention in any way.

### Examples

### Example 1. Proteolytic characterization of an HLA epitope as a housekeeping epitope or an immune epitope

Using the procedures described below, a synthetic peptide of 13 amino acids or more is prepared, containing the candidate HLA epitope centrally. Proteasomes are prepared from cells expressing each type of proteasome, for example red blood cells and Raji cells for housekeeping and immune proteasomes, respectively. The peptide is digested with the proteasome preparations and the resultant fragments identified by mass spectrometry. If one of those fragments is co-C-terminal with the HLA epitope, and is produced in significant yield in the preparation containing a housekeeping proteasome, then the HLA epitope is a housekeeping epitope. Similarly, if one of those fragments is co-C-terminal with the HLA epitope and is produced in significant yield by the immune proteasome, and is not produced in significant yield by the housekeeping proteasome, then the HLA epitope is a immune epitope.

### A. Peptide Synthesis

Synthetic or recombinant polypeptides are constructed which encompass the HLA epitope and at least two residues proximal to its termini. These residues added to the ends of a particular HLA epitope are to ensure that the proteasome complex encounters a processing environment similar to that found within the cell, hence increasing the likelihood that it performs its proteolytic functions normally. Additional residues normally found proximal to the ends of the HLA epitope can be added if necessary to help increase the solubility of the peptides.

Some HLA epitopes present solubility difficulties due to their high hydrophobicity. Certain peptides can be extremely difficult to purify because they will not dissolve in normal chromatographic eluents, or they can be very difficult to use once purified because they will not dissolve in the digestion buffers. This problem can be avoided by carefully choosing which part of the sequence surrounding the HLA epitope to include in a particular peptide construct, or by extending the sequence as mentioned in the preceding paragraph. If there are no residues proximal to the ends of the HLA epitope that can help increase the solubility, a short hydrophilic sequence can be added instead (e.g. -EAEAE). This is added at least three to five residues past the end of the HLA epitope to maintain a natural terminal cleavage site for the proteasome.

In a preferred embodiment, peptides are synthesized on an Applied Biosystems 433A Peptide Synthesizer using standard Fmoc solid phase synthesis methodologies. The synthesizer is equipped with a conductivity feedback monitoring system which allows for increased reaction times for sequences that contain stretches of residues that are difficult to deprotect and/or difficult to couple. After synthesis, the peptides are cleaved from their support with trifluoroacetic acid in the presence of appropriate scavengers, precipitated with ether, and then lyophilized.

The crude peptides are then purified on a preparative diphenyl HPLC column after first developing a gradient using a similar analytical diphenyl HPLC system. The major HPLC fractions from the first preparative injection of the peptide are analyzed by electrospray mass spectrometry to identify the target compound. The corresponding peaks from subsequent injections are collected, pooled and lyophilized, and a sample is taken to verify retention time and chromatographic purity by analytical HPLC. These purified peptides are then ready for digestion by the proteasome preparation.

### B. Proteasome Assay

Immune or housekeeping proteasome complexes are isolated as is described in detail in Example 2 below.

The purified peptides are then dissolved in an appropriate buffer to a concentration of about 1 mM and added to approximately 2 volumes of the proteasome preparations. Replicate digests are prepared: one for mass spectrometry analysis and one for HPLC analysis, and an additional digest is prepared using a positive control peptide to verify proper functioning of the proteasome preparation used. The following peptides are suitable for use as control peptides for immune proteasome assays: MLLAVLYCLLWSFQTS (SEQ ID NO: 7); HSYTTAEEAAGITILTVILGVL (SEQ ID NO: 8); EAASSSSTLVEVTLGEVPAAESPD (SEQ ID NO: 9); EFLWGPRALVETSYVKVLHHMVKI (SEQ ID NO: 10); APEEKIWEELSVLEVFEGR (SEQ ID NO: 11); and ELMEVDPIGHLYIFAT (SEQ ID NO: 12). Underlined residues indicate proteolytic cleavage sites. Peptide FLWGPRALVETSYVK (SEQ ID NO: 13) is suitable as a control peptide for housekeeping proteasome assays. These are allowed to incubate in parallel at 37°C for a period of time and then the digestion is stopped by the addition of dilute trifluoroacetic acid and the samples frozen on dry ice. One replicate and a positive control are sent for analysis using a Lasermat 2000 (Finnigan Mat, LTD, U.K.). Matrix Assisted Laser Desorption Ionization - Time Of Flight (MALDI-TOF) mass spectrometry, and the others are set aside for HPLC.

### C. MALDI-TOF Mass Spectrometric Analysis of the Digest

Analysis of the digests is conducted employing either "Peptide" software, (Lighthouse Data), or software available from ThermoBioanalysis Ltd., U.K. This software can generate the sequence and molecular weight of all the possible fragments that satisfy both requirements of having the correct C-terminus of any predicted epitope, and containing the full length of that epitope or longer.

For example, if the HLA epitope encompassing peptide is of the sequence:
AAMLLAVLYCLLSEIAAAEEE,
where the underlined sequence is the HLA epitope, then the program would identify all of the following sequences as being potentially useful, and would assign each a molecular weight.
AAMLLAVLYCLLSEI
AMLLAVLYCLLSEI
MLLAVLYCLLSEI
LLAVLYCLLSEI
LAVLYCLLSEI
AVLYCLLSEI

If the MALDI-TOF results show that one or more of those molecular weights is represented in a digestion mixture, then the corresponding peptide is synthesized, purified, identified by mass spectrometry and then subjected to analytical HPLC to establish both a standard retention time and an approximate mass to peak area ratio. The reserve digest is then diluted in an appropriate solvent and injected using the same analytical HPLC method. If the digest gives a peak in good yield that has the same retention time as that of the standard, it is almost certain that it is due to the presence of that sequence in the digest. If there is any ambiguity due to the possible generation of other fragments that would give the same or similar mass spectrometry results, the suspect component can be collected and set aside for C-terminal sequencing to confirm identity.

### Example 2. Purification of proteasome complexes

### A. Proteasome complexes from blood cells

Concentrated erythrocyte bags were obtained from a local blood bank, (*HemaCare*, Van Nuys, CA). The contents of each bag were poured into 200 ml centrifuge tubes and washed 3 times with PBS by centrifugation at 2000 RPM for 10 minutes at room temperature in a swinging bucket rotor of a Megafuge 2.0 (Heraeus, Southplainfield, NJ). After the last wash the samples were pooled in one container, to minimize variability among tubes, and then re-divided into several centrifuge tubes. The cells were centrifuged again at 2000 RPM for 10 min. The residual PBS was aspirated. The pellet was stored at -70° C until use.

### B. Proteasome complexes from tumor cells

Raji cells, a Burkitt's lymphoma cell line, were obtained from *ATCC, (American Type Culture Collection,* Manassas, VA). The cells were grown using standard cell culture methods and stimulated with INF-Gamma (100-500U/ml) *(Pharmingen,* San Diego, CA). Expression of immune proteasome subunits was confirmed separately by immunohistochemsitry on the culture, and SDS-PAGE on a sample of the cell lysate. The cells were collected by centrifugation, washed with PBS and stored at -70° C until use.

### C. Further processing of proteasome complexes

Blood or lymphoma tumor cell pellets (frozen) were thawed in a 37°C bath and ddH₂O was added to each tube. The cell suspension was homogenized in a 40 ml Dounce homogenizer. Further, for the tumor cells, the cell homogenate was centrifuged at 2000 rpm to remove cell debris. The supernatant was centrifuged at 10,000 rpm at 4° C for 10 minutes and further centrifuged at 50,000 rpm at 4° C for 30 minutes in a T-1270 rotor (Sorval, Newtown, CT).

The homogenates were passed through filter paper to remove debris, and then pooled together. A 68% sucrose solution was added to the pooled homogenate sample. An antibody-Sepharose preparation was incubated with the homogenate for three hours at room temperature in a rotator. The suspension was centrifuged and washed 3X with TBS and further thoroughly washed over vacuum funnel 6-8 X. Proteasomes were eluted in TBS (pH 7.6) and the optical density of the eluate was measured. The proteasome preparation was dialyzed overnight at 4° C against 20mM Tris (pH 7.6) using cellulose membrane MWCO 1000. The next day the proteasome preparation was concentrated by ultrafiltration in a Millipore ULTRAFREE-15 centrifugation device (Millipore, Danbury, CT). The proteasomes, at a concentration of 4mg/ml, were then aliquotted and stored at -20° C until use. The proteasomes were tested for activity and specificity by digestion of a fluorogenic substrate or a control peptide yielding known fragments. The following peptides are suitable for use as control peptides for immune proteasome assays: MLLAVLYCLLWSFQTS (SEQ ID NO: 14); HSYTTAEEAAGITILTVILGVL (SEQ ID NO: 15); EAASSSSTLVEVTLGEVPAAESPD (SEQ ID NO: 16); EFLWGPRALVETSYVKVLHHMVKI (SEQ ID NO: 17); APEEKIWEELSVLEVFEGR (SEQ ID NO: 18); and ELMEVDPIGHLYIFAT (SEQ ID NO: 19). Underlined residues indicate proteolytic cleavage sites. Peptide FLWGPRALVETSYVK (SEQ ID NO: 20) is suitable as a control peptide for housekeeping proteasome assays.

### D. Quantitation and activity analysis of proteasome preparations

An enzyme-linked immunosorbant assay (ELISA) was used to quantitate the proteasome preparations described above. ELISA techniques are well known in the art and are discussed generally in Ausubel, et al., "Short Protocols in Molecular Biology," 3rd Ed., Unit 11.2 (1997). Hybridoma cells (MCP-21) producing a monoclonal anti-human proteasome antibody were obtained from the European Collection of Cell Culture ((ECACC), UK) and were maintained using standard cell culture techniques and equipment. Hybridoma supplement (Gibco BRL, Rockville, MD) was added to the antibody-producing cells. Upon reaching cell density of 500,000 cells/ml in an approximate volume of 2-3 liters, the cells were removed by centrifugation and the supernatant was collected. Secretion of mAb in the medium was monitored periodically by optical density (O.D.) using a Lambda 20 Spetrophotometer (Perkin Elmer, Norwalk, CT).

The supernatant was passed over a protein G sepharose column (Amersham/Pharmacia Biotech Piscataway, NJ). The column was washed with PBS and the antibody was eluted in a 0.1M glycine buffer, pH 2.2. The optical density of the eluate fractions was measured at 280 nm, and the positive factions were collected. The antibody was dialyzed against 2L of PBS for 2 days at 4° C and stored until use.

The antibody was bound to CNBr-activated Sepharose 4B (Amersham Pharmacia biotech, Piscataway, NY). The antibody-Sepharose complex was washed alternately 5-7 times with 0.1M sodium acetate saline, pH 4 and 0.1M sodium borate saline, pH 8 and finally suspended in Tris buffered saline (TBS), pH 8. The preparation was stored at 4° C until use.

### Example 3. Generation of predicted MHC I peptide cleft binding peptides using algorithmic modeling

A population of candidate MHC I binding peptides, generated from the amino acid sequence of human carcinoembryonic antigen precursor (CEA) (GENBANK ACCESSION P06731), was produced using an algorithm. The particular algorithm is available at <<http://134.2.96.221/scripts/hlaserver.dll/EpPredict.htm>>, as discussed above. Once the algorithm was accessed, the amino acid sequence for CEA was provided. Next, parameters for the length of the epitope (decamers) and the particular MHC allele (H2-Db) of interest were selected. Following this, the data were submitted for algorithmic analysis. The resulting data are shown in Table 7.

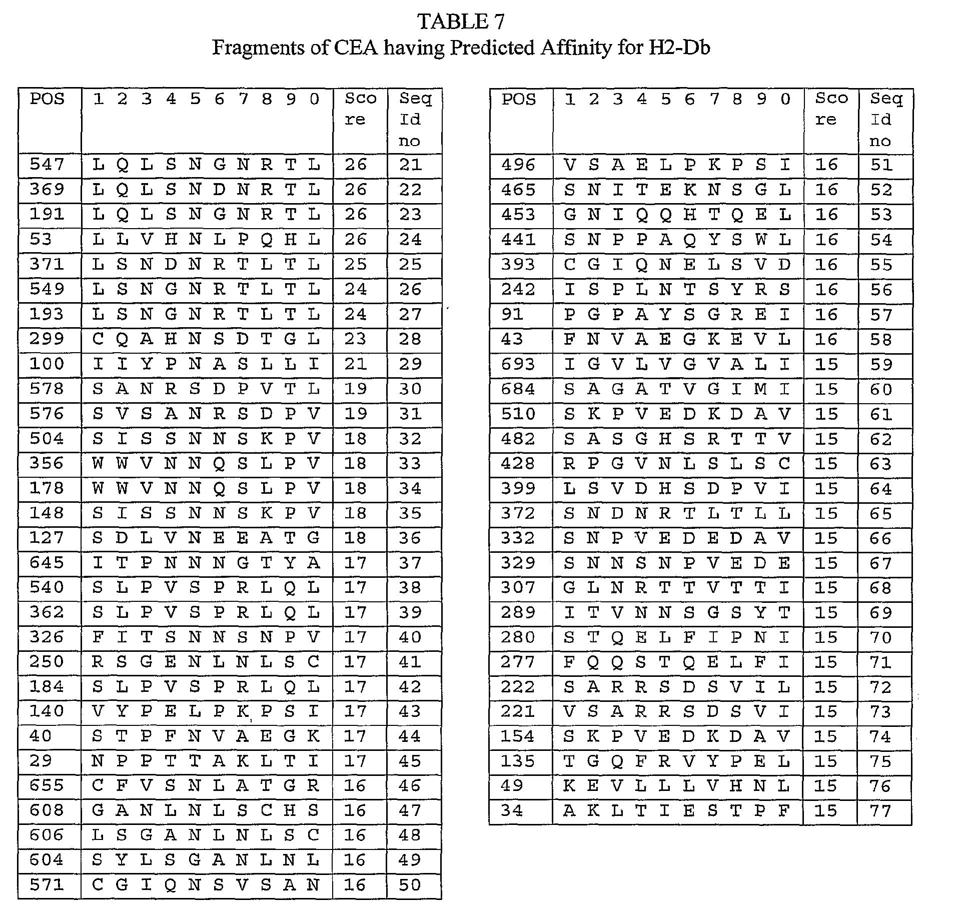

The table above arbitrarily cuts off scores below 15. The algorithm can produce scores of less than 15.

### Example 4. Digestion of peptide precursors using immune and housekeeping proteasomes to determine fragments produced by proteolytic digestion

Peptides were synthesized using a 433A ABI synthesizer. Peptides were produced in 0.25 mmole quantities using Fastmoc chemistry. The peptides were tested for solubility and once solubilized, a 2mM solution was prepared and divided into ∼ 25-30µL aliquots which were stored at -20°C for future use. Timed digest reactions, typically consisting of 2µl of peptide and 4µl of proteasome, were conducted with t=0 as a control and an incubation of the peptide with water instead of the proteasome as a further control. The reaction was carried out at 37° C and ended by the addition of 10% TFA (trifluroacetic acid) on dry ice. The frozen samples were then analyzed by MALDI-TOF mass spectroscopy (MS) as described in Example 5, below.

An optional desalting step can be performed on the digests prior to MS analysis using the ZIP-TIP method (Millipore, Boston, MA). The ZIP TIP is a specially designed pipet tip which contains a bed of spherical silica resin. The sample is bound to the tip, which is pre-equilibrated with 0.1% TFA, and then eluted with 50% Acetonitrile 0.1% TFA elution buffer.

### Example 5. Identification and quantitation of relevant proteolytic fragments by HPLC and mass spectrometry

### A. Identifying sequences of therapeutic interest

The amino acid sequence of a protein of interest is entered into a computer, and the algorithm of Rammensee, et al., is used to generate 9- or 10-amino-acid-long sequences predicted to bind a particular HLA receptor. The algorithm also ranks these predicted epitopes according to how well they match the binding motif.

Synthetic peptides containing the sequence of the identified potential epitopes are then constructed to encompass the epitope candidate sequence and at least 3-5 residues proximal to its termini. The residues added to the ends of a particular epitope candidate are to ensure that the proteasome complex encounters a processing environment similar to that found within the cell, hence increasing the likelihood that it performs its proteolytic functions normally. Additional residues normally found proximal to the ends of the epitope candidate may be added if necessary to help increase the solubility of the peptides.

Peptides are synthesized on an Applied Biosystems 433A Peptide Synthesizer (Applied Biosystems, Norwalk, CT) using standard Fmoc solid phase synthesis methodologies. The synthesizer is equipped with a conductivity feedback monitoring system which allows for increased reaction times for sequences that contain stretches of difficult to deprotect and difficult to couple residues. After synthesis, the peptides are cleaved from their support with trifluoroacetic acid in the presence of appropriate scavengers, precipitated with ether and then lyophilized.

The crude peptides are then dissolved in a suitable solvent at 0.5 mg/ml. Five microliters (5µl) of this solution is then analyzed on a Shimadzu analytical reverse phase HPLC system (Shimadzu Scientific Instruments, Columbia, MD) using a 0.1% TFA water - acetonitrile gradient. Typically, a C-18 silica column (Machery-Nagel # 720051.40, (Machery-Nagel GmbH, Germany)) is used for hydrophillic and a phenyl silica column (Vydac # 219TP5415 (The Separations Group, Inc., Hesperia, CA)) is used for hydrophobic peptides. The gradients used vary from 0-40% acetonitrile for hydrophillic to 30-70% acetonitrile for hydrophobic peptides. The peptides are subsequently purified on a Varian Prostar HPLC system (Varian, Inc., Palo Alto, CA) using similar gradients and semi-preparative versions of the above-mentioned columns (Machery Nagel # 715802, and Vydac 219TP510). The major HPLC fractions from the first preparative injection of the peptide are analyzed using a MALDI-TOF mass spectrometer to identify the desired component. The corresponding peaks from subsequent injections are collected, pooled and lyophilized, and a sample is taken to verify retention time and chromatographic purity by analytical HPLC using the system described above. These purified peptides are then ready for digestion by the proteasome preparation.

### B. Proteasome assay

Immune or housekeeping proteasome complexes are isolated by the method of Levy, (Morel, S., et al., Immunity 12:107-117 (2000), and the references cited therein) described above. The purified peptide is dissolved in an appropriate buffer to a concentration of about 1 to 2 mM and added to approximately 2 volumes of the proteasome preparation. The buffer chosen must solvate the peptide without interfering with the digestion process. An additional digest is prepared using the positive control peptide described above to verify proper functioning of the proteasome preparation used. These are incubated at 37°C for periods of up to 120 minutes and then the digestion is stopped by the addition of dilute trifluoroacetic acid; the samples are analyzed immediately by mass spectrometry, or they are frozen on dry ice until analysis. The digest reaction can also be halted by putting samples on ice for immediate analysis by mass spectrometry.

### C. MALDI-TOF mass spectrometric analysis of the digest

Approximately 0.5 µl of each digest was mixed with an equal volume of the matrix solution (10 mg/ml dihydroxybenzoic acid in 70% EtOH, pH 2-3) directly on the sample slide and allowed to air dry at about 40°C. The samples were then analyzed on a Lasermat™ MALDI-TOF mass spectrometer (Thermo Bioanalysis, Santa Fe, NM)that was calibrated with suitable molecular weight standards.

The computer programs (either "Peptide" software, (Lighthouse Data), or "Dynamo" (ThermoBioanalysis Ltd., U.K.)) developed for the proteasome assay generates the sequence and molecular weight of all the possible fragments that satisfy both requirements of having the correct C-terminus of any predicted epitope, and of containing the full length of that epitope or longer.

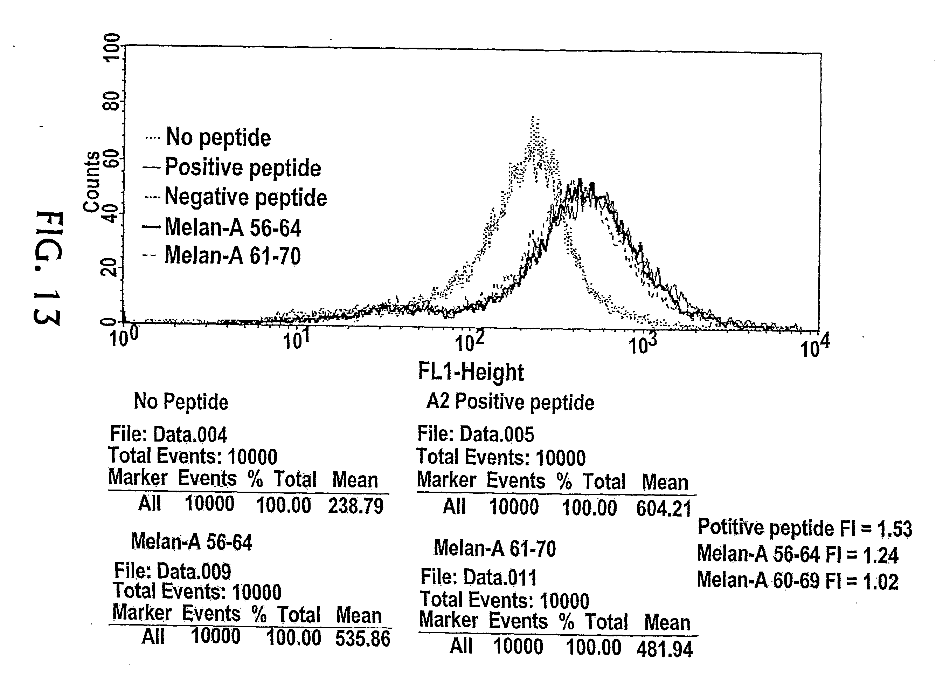

When the MALDI-TOF results showed that a particular molecular weight was represented in a digestion mixture, the corresponding peptide was synthesized, purified, identified by MALDI-TOF and then subjected to reverse phase analytical HPLC to establish a standard retention time and an approximate mass to peak area ratio. These procedures are directly analogous to those described above. A replicate proteasome digest was then diluted in an appropriate solvent and analyzed using the same analytical HPLC method. When the digest gives a peak in good yield that has the same retention time as that of the standard, it is almost certain that it is due to the presence of that sequence in the digest. When there is any ambiguity due to the possible generation of other fragments that would give rise to the same or similar mass spectrometry results, the suspect component can be collected and set aside for sequencing to confirm identity. The analytical HPLC also importantly provides relatively accurate quantitation of the peptide product in the digest, which allows determination of whether a given peptide is a minor or a major product of the digest, which indicates whether the epitope is efficiently produced by the proteasome. Using the above method, housekeeping epitopes were identified. Figure 13 shows the results of a flow cytometry assay to verify HLA binding by these epitopes. This assay is discussed in Example 6.

### Example 6. Determine the MHC binding ability of selected peptides

Binding of a candidate epitope to HLA-A2.1 was assayed according to the method of Stauss et al., (Proc Natl Acad Sci USA 89(17):7871-5 (1992)). T2 cells, which express empty or unstable MHC molecules on their surface, were washed twice and suspended at 5x10⁶ cells/ml in serum-free complete Iscove's modified Dulbecco's medium (IMDM). β₂ microglobulin (Sigma, St. Louis, MO) was added at 5µg/ml and the cells distributed to a 96-well U-bottom plate at 5x10⁵ cells/well. Peptides were added at 100, 10, 1 and 0.1µg/ml. The plate was rocked gently for 2 minutes and then incubated for 4 hours in a 5% CO₂ incubator at 37°C. After the unbound peptide was removed by washing twice with IMDM, a saturating amount of monoclonal antibody W6/32 (Sigma) was added. After incubation for 30 minutes at 4°C, cells were washed with PBS supplemented with 1% heat-inactivated FCS, 0.1%(w:v) sodium azide, pH 7.4-7.6 (staining buffer), and incubated with fluorescein isothiocyanate (FITC)-conjugated goat F(ab') antimouse-IgG (Sigma) for 30 min at 4°C and washed four times as before. The cells were resuspended in staining buffer and fixed by adding a quarter volume of 2% paraformaldehyde. The analysis of surface HLA-A2.1 molecules stabilized by peptide binding was performed by flow cytometry using a FACScan (Becton Dickinson, San Jose, CA).

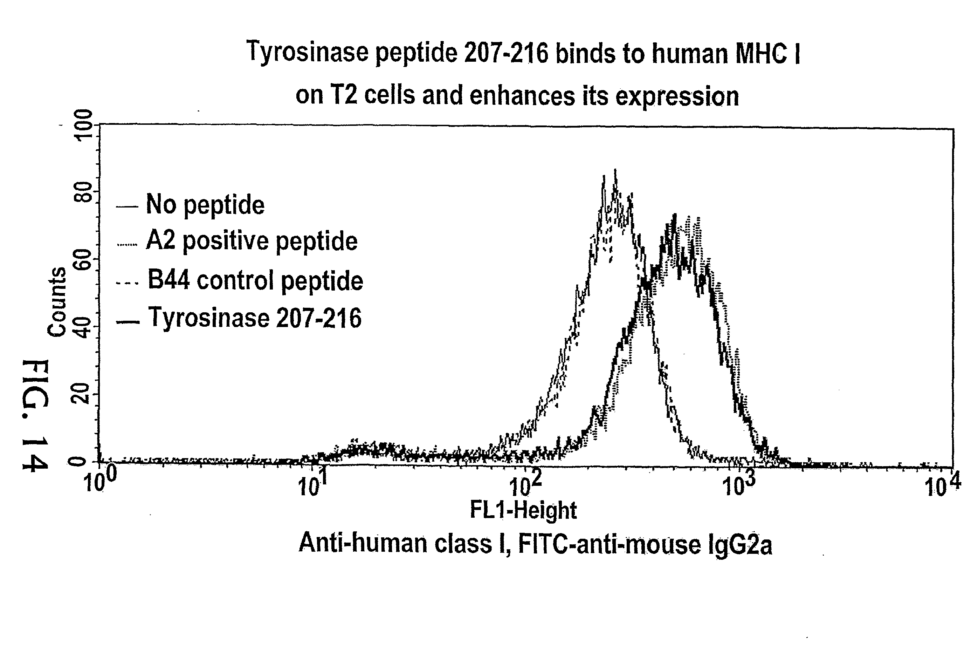

The results of the experiment are shown in Figure 14. Using the method discussed above, a candidate tyrosinase housekeeping epitope identified by proteasomal digestion, (tyrosinase 207-216, FLPWHRLFLL SEQ ID NO: 78) was found to bind HLA-A2.1 to a similar extent as the known A2.1 binder FLPSDYFPSV (SEQ ID NO: 79) (positive control). HLA-B44 binding peptide AEMGKYSFY (SEQ ID NO: 80) used as a negative control. The fluorescence obtained from the negative control was similar to the signal obtained when no peptide was used in the assay. Positive and negative control peptides were chosen from Table 18.3.1 in Current Protocols in Immunology p. 18.3.2, John Wiley and Sons, New York, 1998.

### Example 7. Elution of HLA epitopes from tumors, tissue samples, immortalized cell lines, or tumor cell lines

Rather than generating HLA epitopes with *in vitro* proteolysis, they can be identified after elution from the HLA of tumors, tissue samples, tumor cell lines or other immortalized cell lines using mass spectrometry methods. While a variety of such methods can be used, one of the most powerful methods of identifying epitopes from the surface of cells involves capillary or nanocapillary HPLC ESI mass spectrometry and on-line sequencing, as described in the published literature. Elution procedures for solubilized HLA and intact cells are also described in Falk, K. et al. Nature 351:290, 1991 and in U.S. Patent 5,989,565, respectively. Not described in the literature, however, is the need to identify the type proteasome expressed in the cells undergoing peptide elution and analysis, so as to determine if the epitopes identified are housekeeping epitopes, which are needed to make effective vaccines. To definitively identify the HLA epitope as either a housekeeping or immune epitope one generally must know which proteasome the source cells express. Proteasome expression can be assessed preferably by western blotting, which is described in detail below, and can also be assessed by RT-PCR, immunohistochemistry, or *in situ* hybridization.

### Example 8. IFN induction test

Another assay to distinguish between housekeeping epitopes and immune epitopes is to test the ability of anti-peptide CTL to kill cells expressing the TAA in question. IFN can be used to induce expression of the immune proteasome (assuming it is not already constitutively expressed) and CTL recognition of the induced and uninduced cells can be compared. As above, proteasome type should be confirmed, e.g., by western blotting. If the IFN-induced cells are killed preferentially, the peptide constitutes an immune epitope. If the non-induced cells are killed preferentially, the peptide constitutes a housekeeping epitope. Some epitopes can be produced by both proteasomes at differing efficiencies, and in such cases cytolytic activity is observed against both populations. Such epitopes are classified as housekeeping epitopes since they are present on peripheral target cells.

### Example 9. The use of human peripheral blood mononuclear cells (PBMCs) or tumor infiltrating lymphocytes (TILs) to identify housekeeping epitopes

TILs isolated from patient biopsies, or PBMCs from blood of donors or patients can be used to identify housekeeping epitopes using methods that are commonly described in the published literature. To identify housekeeping epitopes, the target cells used to test for active killing by PBMCs or TILs are confirmed to express only the housekeeping proteasomes, and not to express at significant levels the immune proteasome. PBMCs from donor blood are stimulated *in vitro* using a panel of peptide antigens with predicted affinity for the class I HLA allele expressed on the blood cells being used. Each PBMC sample is stimulated with a specific class I peptide antigen for one week, preferably with the combination of cytokines such as IL-2 or IL-12 to enhance the activity of the T cells. This stimulation is repeated at least three times to induce clonal expansion of T cells specific against the peptide. A standard chromium release assay is performed using target cells that are known to express the protein containing the epitope and exclusively the housekeeping proteasome. Evidence of killing of the target cells as measured by chromium release indicates that the peptide used to stimulate the PBMCs is present as a housekeeping epitope on the surface of the target cell. Tumors expressing this protein are thus candidate targets for a vaccine containing the epitope.

### Example 10. Identification of housekeeping and/or immune proteasomes by western blotting

Both of the following protocols start with a membrane onto which proteins extracted from cells of interest have been transferred after electrophoretic separation.

A. Chromogenic protocol:
1. Wash the membrane for 5 min in 20 ml PBS-T (phosphate buffered saline, pH 7.4 + 0.1% Tween-20) at room temperature on an orbital shaker (RT/shaker).
PBS (Sigma, Cat. No. P-3813)
(Volumes may vary with type of container throughout).
2. Incubate the membrane for 5 min in 20 ml PBS-T, 3% H₂O₂ at RT/shaker:
2 ml 30%H₂O₂+18ml PBS-T
3. Wash the membrane 3x5 min with PBS-T at RT/shaker.
4. Block overnight in 20ml PBS-T/5% nonfat dry milk at 4°C/shaker:
20ml PBS-T+1g milk
5. Rinse the membrane in PBS-T.
6. Incubate the membrane in 5ml of primary antibody (Affinity Research Products Ltd, United Kingdom) in blocking buffer for 2hrs at RT/shaker:

| | |
|---|---|
| α-LMP 2 antiserum (mouse) (Cat. No.PW8205) | 1:5000 |
| α-LMP 2 antiserum (human) (Cat. No. PW 8345) | 1:10000 |
| α-LMP 7 antiserum (Cat. No. PW 8200) | 1:20000 |
| α-20S proteasome α2 subunit monoclonal antibody (Cat. No. PW 8105) | 1:1000 |

These conditions are for the preceding antibodies only. Conditions for every antibody must be determined empirically.
7. Wash the membrane as in step 3.
8. Incubate the membrane in 5 ml of secondary antibody (Vector Laboratories, Inc., Burlingame, CA) in blocking buffer for 30 min at RT/shaker:

| | |
|---|---|
| GARB (Goat anti Rabbit) (for antisera) (Vector Labs Cat. No. BA-1000) | 1:2000 |
| Horse anti mouse (for monoclonal antibodies) (Vector Labs Cat. No. BA-2000) | 1:1000 |

9. Wash the membrane as in step 3.
10. Incubate the membrane in 5 ml of ABC (Vector Laboratories, Cat. No. PK-6100) in PBS-T for 30 min:
Make ABC at least 30 min before using as follows:
   A=5ul/1ml=25ul/5ml
   B=5ul/1ml = 25ul/5ml
   5ul A+5ul B >mix>let stand at 4 C>add 990ul PBS-T
   Dilute ABC in PBS-T just before using
11. Wash the membrane as in step 3.
12. Detection:
1) transfer 5 ml of 0.2M PB into a 1^{st} 15 ml tube
   0.4M Phosphate buffer:
   90.4 ml of Sodium Phosphate Monobasic (1M)
   619.2 ml of Sodium Phosphate Dibasic (0.5M)
   pH to 7.4
   QS to 1L
2) transfer 2.8 ml of 0.2M PB into a2^{nd} 15 ml tube
3) transfer 2 ml of 1% Glucose into a 3^{rd} 15 ml tube
4) weigh 6mg of ANS (Ammonium Nickel Sulfate) and transfer it into 1^{st} 15ml tube;
   vortex
5) add 110 1 of Glucose Oxidase (Sigma, Cat. No.G-6891) into an eppendorftube
6) add 110 1 of DAB substrate (Diaminobenzidine HCl, KPL, Maryland Cat. No.71-00-46) into another eppendorf tube
7) Mix in the hood: 5ml PB+ 2ml Glucose
   +110 1 GO
   +110 1 DAB
   + 2.8 ml 0.2M PB
13. Apply detection mixture on the membrane and set up timer. Record length of incubation in chromogen.
14. After bands became visible enough wash the membrane 3 times with 0.2M PB.
15. Shake in PBS overnight at RT.

### B. Chemiluminescence protocol:

1. Rinse the membrane twice in TBS-T (Tris-buffered saline pH7.6 + 0.1% Tween-20). Tris-buffered saline: 2.42g Tris base (20mM)
8g sodium chloride (137mM)
3.8ml 1M hydrochloric acid
2. Block overnight in 20ml of blocking buffer (TBS-T/5% nonfat dry milk) 4 °C/shaker:
20ml TBS-T + 1g milk
Volumes depend on type of container
3. Rinse the membrane twice with TBS-T.
4. Incubate the membrane in 5ml of primary antibody (Affinity Research Products Ltd, United Kingdom) in blocking buffer for 2hrs at RT/shaker:

| | |
|---|---|
| α-LMP 2 antiserum (mouse) (Cat. No.PW8205) | 1:5000 |
| α-LMP 2 antiserum (human) (Cat. No. PW 8345) | 1:10000 |
| α-LMP 7 antiserum (Cat. No. PW 8200) | 1:20000 |
| α-20S proteasome α2 subunit monoclonal antibody (Cat. No. PW 8105) | 1:1000 |

5. Wash the membrane in 20ml of TBS-T at RT/shaker:
Briefly rinse the membrane using two changes of TBS-T then wash once for 15 minutes and twice for 5 minutes with fresh changes of the washing buffer at room temperature.
6. Incubate the membrane in 5 ml of HRP labeled (Horseradish peroxidase-labeled) secondary antibody (Amersham; Cat# NIF 824 or NIF 825) 1:1000 dilution in blocking buffer for 1h at RT/shaker
7. Wash the membrane as in step 5.
8. Mix an equal volume of detection solution 1 (Amersham, Cat#RPN2109) and detection solution 2 (Amersham, Cat#RPN2109) (1ml+1ml).
9. Drain the excess buffer from the washed membrane and put it on a piece of Saran Wrap, protein side up. Add the detection reagent to cover the membrane.
10. Incubate for 1 minute at room temperature without agitation.
11. Drain off excess of detection reagent and transfer the membrane to Kodak Digital Science Image Station 440CF protein side down. Develop and quantify the signal according to the manufacturers' instructions.

The presence of housekeeping-specific subunits (in either protocol) is directly assessed using:

| | |
|---|---|
| α-β1 (Y) subunit monoclonal antibody (Cat. No. PW 8140) | 1:1000 |
| α-β2 (Z) subunit monoclonal antibody (Cat. No. PW 8145) | 1:1000 |

(Affinity Research Products Ltd, United Kingdom).

### Example 11. Preparation of a housekeeping epitope peptide vaccine

A sequence identified to be a housekeeping epitope is synthesized using a commercial peptide synthesizer. Peptides of interest are formulated in different ways and administered alone, or in combination with adjuvants, such as CFA, IFA, or melacine, or with cytokines, such as IL-2, IL-12, or GM-CSF in order to achieve the effect of stimulating T cells against the epitope in animals. Peptides are also formulated with controlled release substances, such as PLGA microspheres or other biodegradable substances, which alter the pharmacokinetics of the peptide and can also improve immunogenicity. Peptides are also formulated for oral delivery using such substances to facilitate priming of the immune response through uptake into GALT (gut-associated lymphoid tissues). Peptide are also adhered to minute gold particles so that they can be delivered using a "gene gun."

### A. Synthesis of GMP-grade peptides

Peptides are synthesized using either FMOC or tBOC solid phase synthesis methodologies. After synthesis, the peptides are cleaved from their supports with either trifluoroacetic acid or hydrogen fluoride, respectively, in the presence of appropriate protective scavengers. After removing the acid by evaporation, the peptides are extracted with ether to remove the scavengers and the crude, precipitated peptide is then lyophilized. Purity of the crude peptides is determined by HPLC, sequence analysis, amino acid analysis, counterion content analysis and other suitable means. If the crude peptides are pure enough (greater than or equal to about 90% pure), they can be used as is. If purification is required to meet drug substance specifications, the peptides are purified using one or a combination of the following: re-precipitation; reverse-phase, ion exchange, size exclusion or hydrophobic interaction chromatography; or counter-current distribution.

### B. Drug product formulation

GMP-grade peptides are formulated in a parenterally acceptable aqueous, organic, or aqueous-organic buffer or solvent system in which they remain both physically and chemically stable and biologically potent. Generally, buffers or combinations of buffers or combinations of buffers and organic solvents are appropriate. The pH range is typically between 6 and 9. Organic modifiers or other excipients can be added to help solubilize and stabilize the peptides. These include detergents, lipids, co-solvents, antioxidants, chelators and reducing agents. In the case of a lyophilized product, sucrose or mannitol or other lyophilization aids can be added. Peptide solutions are sterilized by membrane filtration into their final container-closure system and either lyophilized for dissolution in the clinic, or stored until use.

### Example 12. Delivery of a housekeeping epitope peptide vaccine

### A. Intranodal delivery

A formulation containing peptide in aqueous buffer with an antimicrobial agent, an antioxidant, and an immunomodulating cytokine, was injected continuously over several days into the inguinal lymph node using a miniature pumping system developed for insulin delivery (MiniMed; Northridge, CA). This infusion cycle was selected in order to mimic the kinetics of antigen presentation during a natural infection.

### B. Controlled release

A peptide formulation is delivered using controlled PLGA microspheres, which alter the pharmacokinetics of the peptide and improve immunogenicity. This formulation is injected or taken orally.

### C. Gene gun delivery

A peptide formulation is prepared wherein the peptide is adhered to gold microparticles. The particles are delivered in a gene gun, being accelerated at high speed so as to penetrate the skin, carrying the particles into dermal tissues that contain pAPCs.

### D. Aerosol delivery

A peptide formulation is inhaled as an aerosol, for uptake into appropriate vascular or lymphatic tissue in the lungs.

### Example 13. Preparation of a nucleic acid vaccine

A carrier plasmid vector, pVAX1 (Invitrogen, Carlsbad, CA), containing a kanamycin resistance gene and a CMV promoter, was modified to include two sequences containing the desired epitopes. In addition it contained an IRES sequence situated between two epitopes to allow their simultaneous expression using one promoter. A suitable E. *coli* strain was then transfected with the plasmid and plated out onto selective media. Several colonies were grown up in suspension culture and positive clones were identified by restriction mapping. The positive clone was then grown up and aliquotted into storage vials and stored at -70°C.

A mini-prep (QIAprep Spin Mini-prep: Qiagen, Valencia, CA) of the plasmid was then made from a sample of these cells and automated fluorescent dideoxy sequence analysis was used to confirm that the construct had the desired sequence. Further nucleic acid vaccine vectors and formulations are described in the foregoing sections of this specification, and in Examples 18-20 below.

### Example 14. Delivery of a nucleic acid vaccine

A nucleic acid vaccine is injected into a lymph node using a miniature pumping system, such as the MiniMed insulin pump. A nucleic acid constructs formulated in an aqueous buffered solution containing an antimicrobial agent, an antioxidant, and an immunomodulating cytokine, is delivered over a several day infusion cycle in order to mimic the kinetics of antigen presentation during a natural infection.

Optionally, the nucleic acid construct is delivered using controlled release substances, such as PLGA microspheres or other biodegradable substances. These substances are injected or taken orally. The nucleic acid vaccine is given using oral delivery, priming the immune response through uptake into GALT tissues. Alternatively, the nucleic acid vaccine is delivered using a gene gun, wherein the nucleic acid vaccine is adhered to minute gold particles. Nucleic acid constructs can also be inhaled as an aerosol, for uptake into appropriate vascular or lymphatic tissue in the lungs.

### Example 15. Assay for presentation of housekeeping epitope on pAPCs

### A. Tetramer analysis

Class I tetramer analysis is used to determine T cell frequency in an animal before and after administration of a housekeeping epitope. Clonal expansion of T cells in response to an epitope indicates that the epitope is presented to T cells by pAPCs. The specific T cell frequency is measured against the housekeeping epitope before and after administration of the epitope to an animal, to determine if the epitope is present on pAPCs. An increase in frequency of T cells specific to the epitope after administration indicates that the epitope was presented on pAPC.

### B. Proliferation assay

Approximately 24 hours after vaccination of an animal with an housekeeping epitope, pAPCs are harvested from PBMCs, splenocytes, or lymph node cells, using monoclonal antibodies against specific markers present on pAPCs, fixed to magnetic beads for affinity purification. Crude blood or splenoctye preparation is enriched for pAPCs using this technique. The enriched pAPCs are then used in a proliferation assay against a T cell clone that has been generated and is specific for the housekeeping epitope of interest. The pAPCs are coincubated with the T cell clone and the T cells are monitored for proliferation activity by measuring the incorporation of radiolabeled thymidine by T cells. Proliferation indicates that T cells specific for the housekeeping epitope are being stimulated by that epitope on the pAPCs.

### Example 16. Assay for effectiveness of housekeeping epitopes as anticancer treatment

Surrogate endppints or survival are used to determine the effectiveness of epitope synchronization vaccines in cancer treatment.

### A. T cell frequency analysis

A useful surrogate endpoint is the determination of T cell frequency against the housekeeping epitope used in immunization. Patients developing elevated T cell frequencies against specific TuAA epitopes used in tumor immunotherapy have significantly better survival compared to patients immunized by the same epitope but not developing increased T cell frequency to the epitope. Tetramer analysis, ELISPOT analysis, or limiting dilution analysis are used to assess T cell frequency to a housekeeping epitope before and after immunization with the epitope, indicating the anticancer effectiveness of a housekeeping epitope in a vaccine.

### B. Tumor burden/survival analysis

An animal with an existing tumor is assessed for tumor burden before and after immunization with a housekeeping epitope. Partial or complete tumor regression indicates effective therapeutic intervention, and correlates with improved survival. In a laboratory setting, several animals are inoculated in parallel with a tumor. Some of the animals are then immunized with a housekeeping epitope vaccine. Survival of animals immunized with the housekeeping epitope is compared to those which received a control epitope or placebo, to determine effectiveness of the vaccine.

### C. Chromium release assay

An animal genetically engineered to express human class I MHC is immunized using a housekeeping epitope. T cells from these animals are used in a standard chromium release assay using human tumor targets or targets engineered to express the same class I MHC. T cell killing of the targets indicates that stimulation of T cells in a patient would be effective at killing a tumor expressing a similar TuAA.

### Example 17: Identification of Unique Housekeeping Epitopes

Epitopes useful in the vaccines and methods of the present invention can be readily identified as disclosed herein. For example, three unique housekeeping epitopes that are not produced by pAPCs have been identified as follows:

### A. Isolation and Purification

Immune or housekeeping proteasome complexes are isolated. The purified peptide is dissolved in an appropriate buffer to a concentration of about 1 to 2 mM and added to approximately 2 volumes of the proteasome preparation. The buffer chosen must solvate the peptide without interfering with the digestion process. An additional digest is prepared using the positive control peptide described above to verify proper functioning of the proteasome preparation used. These are incubated at 37°C for periods of up to 120 minutes and then the digestion is stopped by the addition of dilute trifluoroacetic acid; the samples are analyzed immediately by mass spectrometry, or they are frozen on dry ice until analysis. The digest reaction can also be halted by putting samples on ice for immediate analysis by mass spectrometry.

### B. MALDI-TOF mass spectrometric analysis of the digest

Approximately 0.5 µl of each digest was mixed with an equal volume of the matrix solution (10 mg/ml dihydroxybenzoic acid in 70% EtOH, pH 2-3) directly on the sample slide and allowed to air dry at about 40°C. The samples were then analyzed on a Lasermat™ MALDI-TOF mass spectrometer that was calibrated with suitable molecular weight standards.

The computer program developed for the proteasome assay generates the sequence and molecular weight of all the possible fragments that satisfy both requirements of having the correct C-terminus of any predicted epitope, and of containing the full length of that epitope or longer.

When the MALDI-TOF results showed that a particular molecular weight was represented in a digestion mixture, the corresponding peptide was synthesized, purified, identified by MALDI-TOF and then subjected to reverse phase analytical HPLC to establish a standard retention time and an approximate mass to peak area ratio. These procedures are directly analogous to those described above. A replicate proteasome digest was then diluted in an appropriate solvent and analyzed using the same analytical HPLC method. When the digest gives a peak in good yield that has the same retention time as that of the standard, it is almost certain that it is due to the presence of that sequence in the digest. When there is any ambiguity due to the possible generation of other fragments that would give rise to the same or similar mass spectrometry results, the suspect component can be collected and set aside for sequencing to confirm identity. Using the above method, housekeeping epitopes were identified.

### C. Additional Unique Housekeeping Epitope

Binding of a candidate epitope to HLA-A2.1 was assayed according to the method of Stauss et al., (Proc Natl Acad Sci USA 89(17):7871-5 (1992)). T2 cells, which express empty or unstable MHC molecules on their surface, were washed twice and suspended at 5x10⁶ cells/ml in serum-free complete Iscove's modified Dulbecco's medium (IMDM). β₂ microglobulin (Sigma, St. Louis, MO) was added at 5µg/ml and the cells distributed to a 96-well U-bottom plate at 5x10⁵ cells/well. Peptides were added at 100, 10, 1 and 0.1µg/ml. The plate was rocked gently for 2 minutes and then incubated for 4 hours in a 5% CO₂ incubator at 37°C. After the unbound peptide was removed by washing twice with IMDM, a saturating amount of monoclonal antibody W6/32 (Sigma) was added. After incubation for 30 minutes at 4°C, cells were washed with PBS supplemented with 1% heat-inactivated FCS, 0.1%(w:v) sodium azide, pH 7.4-7.6 (staining buffer), and incubated with fluorescein isothiocyanate (FITC)-conjugated goat F(ab') antimouse-IgG (Sigma) for 30 min at 4°C and washed four times as before. The cells were resuspended in staining buffer and fixed by adding a quarter volume of 2% paraformaldehyde. The analysis of surface HLA-A2.1 molecules stabilized by peptide binding was performed by flow cytometry using a FACScan (Becton Dickinson, San Jose, CA).

Using the method discussed above, a candidate tyrosinase housekeeping epitope identified by proteasomal digestion, (tyrosinase 207-216, FLPWHRLFLL SEQ ID NO: 78) was found to bind HLA-A2.1 to a similar extent as the known A2.1 binder FLPSDYFPSV (SEQ ID NO: 79) (positive control). HLA-B44 binding peptide AEMGKYSFY (SEQ ID NO: 80) used as a negative control. The fluoresence obtained from the negative control was similar to the signal obtained when no peptide was used in the assay. Positive and negative control peptides were chosen from Table 18.3.1 in Current Protocols in Immunology p. 18.3.2, John Wiley and Sons, New York, 1998.

### Example 18. Construction of pVAX-EP1-IRES-EP2

### Overview:

The starting plasmid for this construct is pVAX1 purchased from Invitrogen.(Carlsbad, CA) Epitope EP1 and EP2 were synthesized by GIBCO BRL (Rockville, MD). IRES was cut out from pIRES purchased from Clontech (Palo Alto, CA). See Figure 12.

### Procedure:

| | |
|---|---|
| 1 | Digest pIRES with EcoRI and NotI. Separate the digested fragments with agarose gel, and purify the IRES fragment by gel purification; |
| 2 | Digest pVAX1 with EcoRI and NotI. Gel-purify the pVAX1 fragment; |
| 3 | Set up a ligation containing the purified pVAX1 and IRES fragment; |
| 4 | Transform competent DH5α with the ligation mixture; |
| 5 | Pick up 4 colonies and make a miniprep. |
| 6 | Perform restriction enzyme digestion analysis of the miniprep DNA. One recombinant colony having the IRES insert was used for further insertion of EP1 and EP2. This intermediate construct was called pVAX-IRES. |
| 7 | Synthesize EP1 and EP2; |
| 8 | Subclone EP1 into pVAX-IRES between AflII and EcoRI site, to make pVAX-EP1-IRES; |
| 9 | Subclone EP2 into pVAX-EP1-IRES between SalI and NotI, to make the final construct pVAX-EP1-IRES-EP2; |
| 10 | Sequence the EP1-IRES-EP2 insert to confirm sequence. |

### Example 19. Construction of pVAX-EP1-IRES-EP2-ISS-NIS

### Overview:

The starting plasmid for this construct was pVAX-EP1-IRES-EP2 (Example 18). ISS (immunostimulatory sequence) (SEQ ID NO: 82) introduced to this construct is AACGTT, and the NIS (standing for nuclear import sequence) (SEQ ID NO: 81) used is the SV40 72bp repeat sequence. ISS-NIS was synthesized by GIBCO BRL. See Figure 9.

### Procedure:

| | |
|---|---|
| 1 | Digest pVAX-EP1-IRES-EP2 with NruI. Gel-purify the linearized plasmid; |
| 2 | Synthesize ISS-NIS; |
| 3 | Set up a ligation reaction containing the purified linearized pVAX-EP1-IRES-EP2 and synthesized ISS-NIS; |
| 4 | Transform competent DH5α with the ligation product; |
| 5 | Pick up colonies and make a miniprep; |
| 6 | Carry out restriction enzyme digestion of the miniprep; |
| 7 | Sequence the plasmid with the insert. |

### Example 20. Construction of pVAX-EP2-UB-EP1

### Overview:

The starting plasmid for this construct is pVAX1 (Invitrogen). EP2 and EP1 were synthesized by GIBCO BRL. Wild type Ubiquitin cDNA encoding the 76 amino acids in the construct was cloned from yeast. See Figure 10.

### Procedure:

| | |
|---|---|
| 1 | Perform RT-PCR using yeast mRNA. Primers were designed to amplify the complete coding sequence of yeast Ubiquitin; |
| 2 | Analyze the RT-PCR products using agarose gel. Gel-purify the band with the predicted size; |
| 3 | Subclone the purified DNA band into pZERO1 at EcoRV site. The resulting clone was named pZERO-UB; |
| 4 | Sequence several clones of pZERO-UB. Confirm Ubiquitin sequence before further manipulations; |
| 5 | Synthesize EP1 and EP2; |
| 6 | Ligate EP2, Ubiquitin and EP1 and clone the insert into pVAX1 between BamHI and EcoRI, making it under control of the CMV promoter; |
| 7 | Confirm the sequence of the insert EP2-UB-EP1 by sequencing. |

IRES (SEQ ID NO: 6) 
AATTCCGCCCCTCTCCCTCCCCCCCCCCTAACGTTACTGGCCGAAGCCGCTTGGAATAA
GGCCGGTGTGCGTTTGTCTATATGTGATTTTCCACCATATTGCCGTCTTTTGGCAATGTG
AGGGCCCGGAAACCTGGCCCTGTCTTCTTGACGAGCATTCCTAGGGGTCTTTCCCCTCT
CGCCAAAGGAATGCAAGGTCTGTTGAATGTCGTGAAGGAAGCAGTTCCTCTGGAAGCT
TCTTGAAGACAAACAACGTCTGTAGCGACCCTTTGCAGGCAGCGGAACCCCCCACCTG
GCGACAGGTGCCTCTGCGGCCAAAAGCCACGTGTATAAGATACACCTGCAAAGGCGGC
ACAACCCCAGTGCCACGTTGTGAGTTGGATAGTTGTGGAAAGAGTCAAATGGCTCTCC
TCAAGCGTATTCAACAAGGGGCTGAAGGATGCCCAGAAGGTACCCCATTGTATGGGAT
 
CTGATCTGGGGCCTCGGTGCACATGCTTTACATGTGTTTAGTCGAGGTTAAAAAACGT
CTAGGCCCCCGAACCACGGGGACGTGGTTTTCCTTTGAAAAACACGATGATAA
 Reference: Clontech PT3266-5
UBIQUITIN (SEQ ID NO: 5) 
ATGCAGATTTTCGTCAAGACTTTGACCGGTAAAACCATAACATTGGAAGTTGAATCTTC
CGATACCATCGACAACGTTAAGTCGAAAATTCAAGACAAGGAAGGTATCCCTCCAGAT
CAACAAAGATTGATCTTTGCCGGTAAGCAGCTAGAAGACGGTAGAACGCTGTCTGATT
ACAACATTCAGAAGGAGTCCACCTTACATCTTGTGCTAAGGCTAAGAGGTGGC
 Reference: Ozkaynak, E., Finley, D., Solomon, M.J. and Varshavsky, A., The yeast ubiquitin genes: a family of natural gene fusions. EMBO J. 6 (5), 1429-1439 (1987).
NIS (SEQ ID NO: 81)
   TGGTTGCTGACTAATTGAGATGCATGCTTTGCATACTTCTGCCTGCTGGGGAGCCTGGG GACTTTCCACACC
   Reference: Dean DA, Dean BS, Muller S, Smith LC, Sequence requirements for plasmid nuclear import. Exp. Cell Res. 253 (2): 713-22 (1999).
ISS (SEQ ID NO: 82)
   AACGTT
   Reference: Sato Y, Roman M, Tighe H, Lee D, Corr M, Nguyen M, Silverman GJ, Lotz M, Carson DA and Raz E, Immunostimulatory DNA sequences necessary for effective intradermal gene immunization. Science, 273: 352-354 (1996).

Examples 21-24 all concern the prediction of 9-mer epitopes presented by HLA-A2.1, although the procedure is equally applicable to any HLA type, or epitope length, for which a predictive algorithm or MHC binding assay is available.

### Example 21. Melan-A/MART-1 (SEQ ID NO: 1)

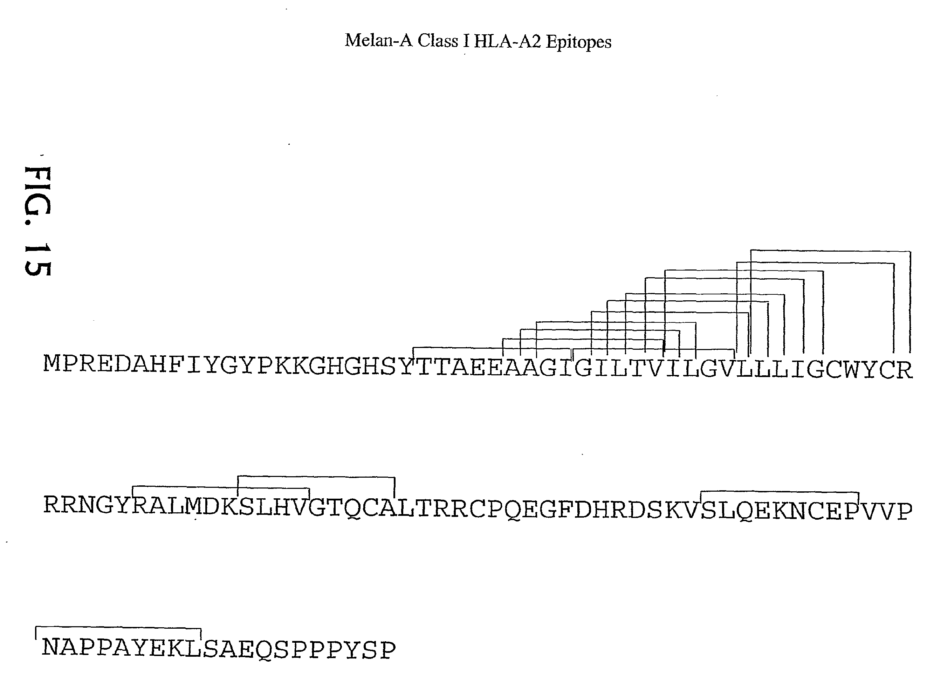

This melanoma tumor-associated antigen (TAA) is 118 amino acids in length. Of the 110 possible 9-mers, 16 are given a score 16 by the SYFPEITHI/Rammensee algorithm. (See Table 8). These represent 14.5% of the possible peptides and an average epitope density on the protein of 0.136 per amino acid. Twelve of these overlap, covering amino acids 22-49 of SEQ ID NO: 1 resulting in an epitope density for the cluster of 0.428, giving a ratio, as described above, of 3.15. Another two predicted epitopes overlap amino acids 56-69 of SEQ ID NO: 1, giving an epitope density for the cluster of 0.143, which is not appreciably different than the average, with a ratio of just 1.05. See Figure 15.

**Table 8**

| SYFPEITHI (Rammensee algorithm) Results for Melan-A/MART-1 (SEQ ID NO: 1) | | |
|---|---|---|
| **Rank** | **Start** | **Score** |
| **1** | 31 | 27 |
| **2** | 56 | 26 |
| **3** | 35 | 26 |
| **4** | 32 | 25 |
| **5** | 27 | 25 |
| **6** | 29 | 24 |
| **7** | 34 | 23 |
| **8** | 61 | 20 |
| **9** | 33 | 19 |
| **10** | 22 | 19 |
| **11** | 99 | 18 |
| **12** | 36 | 18 |
| **13** | 28 | 18 |
| **14** | 87 | 17 |
| **15** | 41 | 17 |
| **16** | 40 | 16 |

Restricting the analysis to the 9-mers predicted to have a half time of dissociation of 5 minutes by the BIMAS-NIH/Parker algorithm leaves only 5. (See Table 9). The average density of epitopes in the protein is now only 0.042 per amino acid. Three overlapping peptides cover amino acids 31-48 of SEQ ID NO: 1 and the other two cover 56-69 of SEQ ID NO: 1, as before, giving ratios of 3.93 and 3.40, respectively. (See Table 10).

**Table 9**

| BIMAS-NIH/Parker algorithm Results for Melan-A/MART-1 (SEQ ID NO: 1) | | | |
|---|---|---|---|
| **Rank** | **Start** | **Score** | **Log(Score)** |
| **1** | 40 | 1289.01 | 3.11 |
| **2** | 56 | 1055.104 | 3.02 |
| **3** | 31 | 81.385 | 1.91 |
| **4** | 35 | 20.753 | 1.32 |
| **5** | 61 | 4.968 | 0.70 |

**Table 10**

| Predicted Epitope Clusters for Melan-A/MART-1 (SEQ ID NO: 1) | | | | | |
|---|---|---|---|---|---|
| **Calculations(Epitopes/AAs)** | | | | | |
| **Cluster** | **AA** | **Peptides** | **Cluster** | **Whole protein** | **Ratio** |
| 1 2 | 31-48 | 3, 4, 1 | 0.17 | 0.042 | 3.93 |
| 2 | 56-69 | 2, 5 | 0.14 | 0.042 | 3.40 |

### Example 22. SSX-2/HOM-MEL-40 (SEQ ID NO: 2)

This melanoma tumor-associated antigen (TAA) is 188 amino acids in length. Of the 180 possible 9-mers, 11 are given a score 16 by the SYFPEITHI/Rammensee algorithm. (See Table 11). These represent 6.1% of the possible peptides and an average epitope density on the protein of 0.059 per amino acid. Three of these overlap, covering amino acids 99-114 of SEQ ID NO: 2 resulting in an epitope density for the cluster of 0.188, giving a ratio, as described above, of 3.18. There are also overlapping pairs of predicted epitopes at amino acids 16-28, 57-67, and 167-183 of SEQ ID NO: 2, giving ratios of 2.63, 3.11, and 2.01, respectively. There is an additional predicted epitope cluster covering amino acids 5-28 of SEQ ID NO: 2. Evaluating the region 5-28 of SEQ ID NO: 2 containing three epitopes gives an epitope density of 0.125 and a ratio 2.14. (See Table 12).

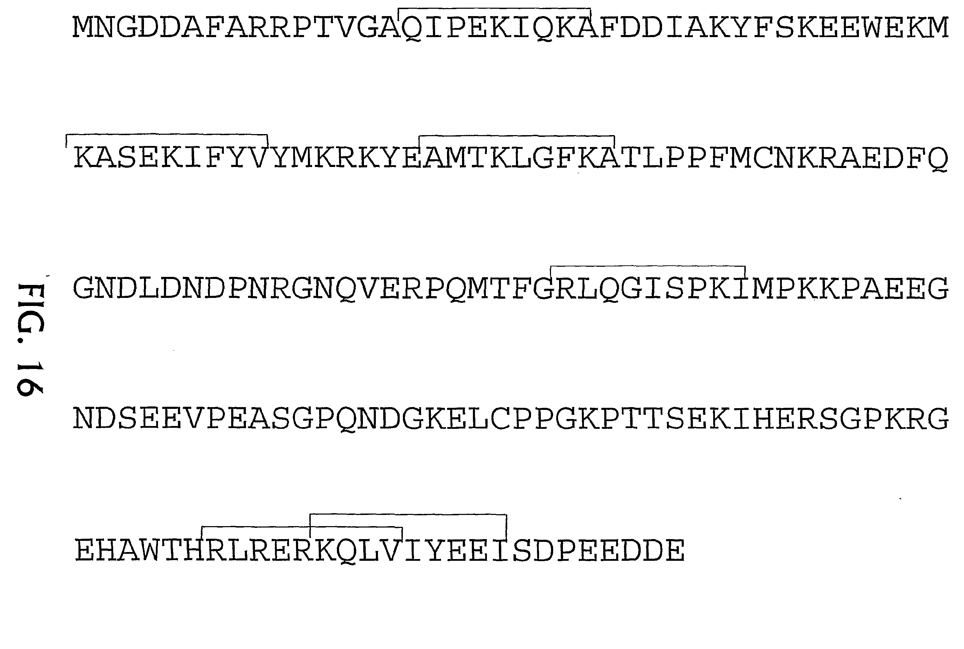

Restricting the analysis to the 9-mers predicted to have a half time of dissociation of 5 minutes by the BIMAS-NIH/Parker algorithm leaves only 6. (See Table 13). The average density of epitopes in the protein is now only 0.032 per amino acid. Only a single pair overlap, at 167-180 of SEQ ID NO: 2, with a ratio of 4.48. However the top ranked peptide is close to another single predicted epitope if that region, amino acids 41-65 of SEQ ID NO: 2, is evaluated the ratio is 2.51, representing a substantial difference from the average. (See Figure 16 and Table 14).

**Table 11**

| SYFPEITHI/Rammensee algorithm for SSX-2/HOM-MEL-40 (SEQ ID NO: 2) | | |
|---|---|---|
| **Rank** | **Start** | **Score** |
| **1** | 103 | 23 |
| **2** | 167 | 22 |
| **3** | 41 | 22 |
| **4** | 16 | 21 |
| **5** | 99 | 20 |
| **6** | 59 | 19 |
| **7** | 20 | 17 |
| **8** | 5 | 17 |
| **9** | 175 | 16 |
| **10** | 106 | 16 |
| **11** | 57 | 16 |

**Table 12**

| Calculations(Epitopes/AAs) (SEQ ID NO: 2) | | | | | |
|---|---|---|---|---|---|
| **Calculations(Epitopes/AAs)** | | | | | |
| **Cluster** | **AA** | **Peptides** | **Cluster** | **Whole protein** | **Ratio** |
| **1** | 5 to 28 | 8, 4, 7 | 0.125 | 0.059 | 2.14 |
| **2** | 16-28 | 4, 7 | 0.15 | 0.059 | 2.63 |
| **3** | 57-67 | 11,6 | 0.18 | 0.059 | 3.11 |
| **4** | 99-114 | 5, 1, 10 | 0.19 | 0.059 | 3.20 |
| **5** | 167-183 | 2,9 | 0.12 | 0.059 | 2.01 |

**Table 13**

| BIMAS-NIH/Parker algorithm (SEQ ID NO: 2) | | | |
|---|---|---|---|
| **Rank** | **Start** | **Score** | **Log(Score)** |
| **1** | 41 | 1017.062 | 3.01 |
| **2** | 167 | 21.672 | 1.34 |
| **3** | 57 | 20.81 | 1.32 |
| **4** | 103 | 10.433 | 1.02 |
| **5** | 172 | 10.068 | 1.00 |
| **6** | 16 | 6.442 | 0.81 |

**Table 14**

| Calculations(Epitopes/AAs) (SEQ ID NO: 2) | | | | | |
|---|---|---|---|---|---|
| **Cluster** | **AA** | **Peptides** | **Cluster** | **Whole protein** | **Ratio** |
| **1** | 41-65 | 1, 3 | .08 | 0.032 | 2.51 |
| **2** | 167-180 | 2, 5 | 0.14 | 0.032 | 4.48 |

### Example 23. NY-ESO (SEQ ID NO: 3)

This tumor-associated antigen (TAA) is 180 amino acids in length. Of the 172 possible 9-mers, 25 are given a score 16 by the SYFPEITHI/Rammensee algorithm. (See Table 15). Like Melan-A above, these represent 14.5% of the possible peptides and an average epitope density on the protein of 0.136 per amino acid. However the distribution is quite different. Nearly half the protein is empty with just one predicted epitope in the first 78 amino acids. Unlike Melan-A where there was a very tight cluster of highly overlapping peptides, in NY-ESO the overlaps are smaller and extend over most of the rest of the protein. One set of 19 overlapping peptides covers amino acids 108-174 of SEQ ID NO: 3, resulting in a ratio of 2.04. Another 5 predicted epitopes cover 79-104 of SEQ ID NO: 3, for a ratio of just 1.38. (See Table 16).

If instead one takes the approach of considering only the top 5% of predicted epitopes, in this case 9 peptides, one can examine whether good clusters are being obscured by peptides predicted to be less likely to bind to MHC. When just these predicted epitopes are considered we see that the region 108-140 of SEQ ID NO: 3 contains 6 overlapping peptides with a ratio of 3.64. There are also 2 nearby peptides in the region 148-167 of SEQ ID NO: 3 with a ratio of 2.00. Thus the large cluster 108-174 of SEQ ID NO: 3 can be broken into two smaller clusters covering much of the same sequence. (See Table 16).

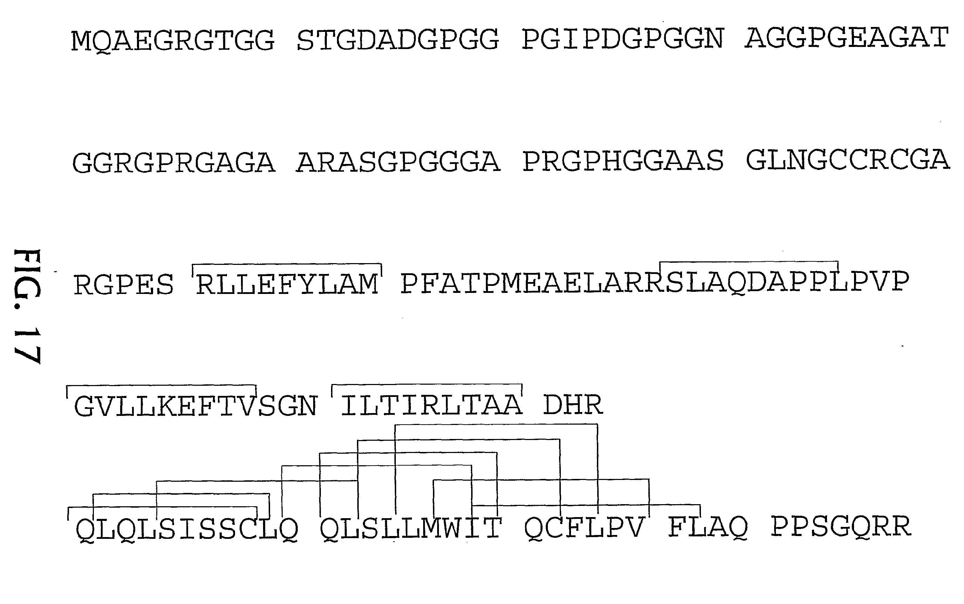

Restricting the analysis to the 9-mers predicted to have a half time of dissociation of 5 minutes by the BIMAS-NIH/Parker algorithm brings 14 peptides into consideration. (See Table 17). The average density of epitopes in the protein is now 0.078 per amino acid. A single set of 10 overlapping peptides is observed, covering amino acids 144-171 of SEQ ID NO: 3, with a ratio of 4.59. All 14 peptides fall in the region 86-171 of SEQ ID NO: 3 which is still 2.09 times the average density of epitopes in the protein. While such a large cluster is larger than we consider ideal it still offers a significant advantage over working with the whole protein. (See Figure 17 and Table 18).

**Table 15**

| SYFPEITHI (Rammensee algorithm) Results for NY-ESO (SEQ ID NO: 3) | | |
|---|---|---|
| **Rank** | **Start** | **Score** |
| **1** | 108 | 25 |
| **2** | 148 | 24 |
| **3** | 159 | 21 |
| **4** | 127 | 21 |
| **5** | 86 | 21 |
| **6** | 132 | 20 |
| **7** | 122 | 20 |
| **8** | 120 | 20 |
| **9** | 115 | 20 |
| **10** | 96 | 20 |
| **11** | 113 | 19 |
| **12** | 91 | 19 |
| **13** | 166 | 18 |
| **14** | 161 | 18 |
| **15** | 157 | 18 |
| **16** | 151 | 18 |
| **17** | 137 | 18 |
| **18** | 79 | 18 |
| **19** | 139 | 17 |
| **20** | 131 | 17 |
| **21** | 87 | 17 |
| **22** | 152 | 16 |
| **23** | 144 | 16 |
| **24** | 129 | 16 |
| **25** | 15 | 16 |

**Table 16**

| Calculations(Epitopes/AAs) (SEQ ID NO: 3) | | | | | |
|---|---|---|---|---|---|
| **Cluster** | **AA** | **Peptides** | **Cluster** | **Whole protein** | **Ratio** |
| **1** | 108-140 | 1, 9, 8, 7, 4, 6 | 0.18 | 0.05 | 3.64 |
| **2** | 148-167 | 2, 3 | 0.10 | 0.05 | 2.00 |
| **3** | 79-104 | 5 12, 10, 18, 21 | 0.19 | 0.14 | 1.38 |
| **4** | 108-174 | 1, 11, 9, 8, 7, 4, 6, 17, 2, 16, 15, 3, 14, 13, 24, 20, 19, 23, 22 | 0.28 | 0.14 | 2.04 |

**Table 17**

| BIMAS-NIH/Parker algorithm Results for NY-ESO (SEQ ID NO: 3) | | | |
|---|---|---|---|
| **Rank** | **Start** | **Score** | **Log(Score)** |
| **1** | 159 | 1197.321 | 3.08 |
| **2** | 86 | 429.578 | 2.63 |
| **3** | 120 | 130.601 | 2.12 |
| **4** | 161 | 83.584 | 1.92 |
| **5** | 155 | 52.704 | 1.72 |
| **6** | 154 | 49.509 | 1.69 |
| **7** | 157 | 42.278 | 1.63 |
| **8** | 108 | 21.362 | 1.33 |
| **9** | 132 | 19.425 | 1.29 |
| **10** | 145 | 13.624 | 1.13 |
| **11** | 163 | 11.913 | 1.08 |
| **12** | 144 | 11.426 | 1.06 |
| **13** | 148 | 6.756 | 0.83 |
| **14** | 152 | 4.968 | 0.70 |

**Table 18**

| Calculations(Epitopes/AAs) (SEQ ID NO: 3) | | | | | |
|---|---|---|---|---|---|
| **Cluster** | **AA** | **Peptides** | **Cluster** | **Whole protein** | **Ratio** |
| **1** | 86-171 | 2, 8, 3, 9, 10, 12, 13, 14, 6, 5, 7, 1, 4, 11 | 0.163 | 0.078 | 2.09 |
| **2** | 144- 171 | 10, 12, 13, 14, 6, 5, 7, 1, 4, 11 | 0.36 | 0.078 | 4.59 |

### Example 24. Tyrosinase (SEQ ID NO: 4)

This melanoma tumor-associated antigen (TAA) is 529 amino acids in length. Of the 521 possible 9-mers, 52 are given a score 16 by the SYFPEITHI/Rammensee algorithm. (See Table 19). These represent 10% of the possible peptides and an average epitope density on the protein of 0.098 per amino acid. There are 5 groups of overlapping peptides containing 2 to 13 predicted epitopes each, with ratios ranging from 2.03 to 4.41, respectively. There are an additional 7 groups of overlapping peptides, containing 2 to 4 predicted epitopes each, with ratios ranging from 1.20 to 1.85, respectively. The 17 peptides in the region 444-506 of SEQ ID NO: 4, including the 13 overlapping peptides above, constitutes a cluster with a ratio of 2.20. (See Table 20).

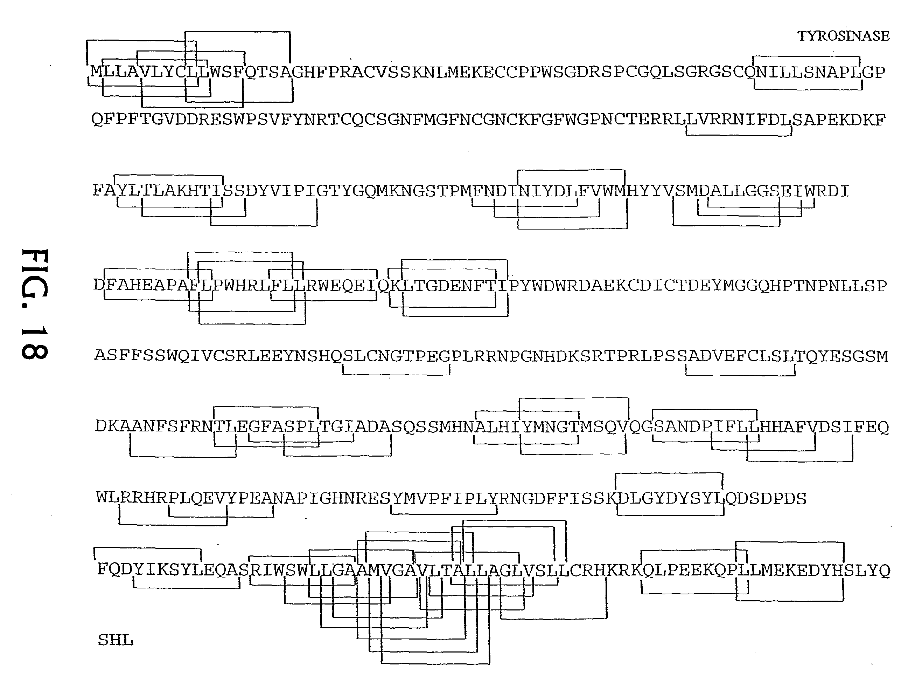

Restricting the analysis to the 9-mers predicted to have a half time of dissociation of 5 minutes by the BIMAS-NIH/Parker algorithm brings 28 peptides into consideration. (See Table 21). The average density of epitopes in the protein under this condition is 0.053 per amino acid. At this density any overlap represents more than twice the average density of epitopes. There are 5 groups of overlapping peptides containing 2 to 7 predicted epitopes each, with ratios ranging from 2.22 to 4.9, respectively. (See Table 22). Only three of these clusters are common to the two algorithms. Several, but not all, of these clusters could be enlarged by evaluating a region containing them and nearby predicted epitopes. See Figure 18.

**Table 19**

| SYFPEITHI/Rammensee algorithm Results for Tyrosinase (SEQ ID NO: 4) | | | | | | |
|---|---|---|---|---|---|---|
| **Rank** | **Start** | **Score** | | **Rank** | **Start** | **Score** |
| **1** | 490 | 34 | | **27** | 5 | 19 |
| **2** | 491 | 31 | | **28** | 484 | 18 |
| **3** | 487 | 28 | | **29** | 476 | 18 |
| **4** | 1 | 27 | | **30** | 463 | 18 |
| **5** | 2 | 25 | | **31** | 444 | 18 |
| **6** | 482 | 23 | | **32** | 425 | 18 |
| **7** | 380 | 23 | | **33** | 316 | 18 |
| **8** | 369 | 23 | | **34** | 187 | 18 |
| **9** | 214 | 23 | | **35** | 402 | 17 |
| **10** | 506 | 22 | | **36** | 388 | 17 |
| **11** | 343 | 22 | | **37** | 346 | 17 |
| **12** | 207 | 22 | | **38** | 336 | 17 |
| **13** | 137 | 22 | | **39** | 225 | 17 |
| **14** | 57 | 22 | | **40** | 224 | 17 |
| **15** | 169 | 20 | | **41** | 208 | 17 |
| **16** | 118 | 20 | | **42** | 186 | 17 |
| **17** | 9 | 20 | | **43** | 171 | 17 |
| **18** | 488 | 19 | | **44** | 514 | 16 |
| **19** | 483 | 19 | | **45** | 494 | 16 |
| **20** | 480 | 19 | | **46** | 406 | 16 |
| **21** | 479 | 19 | | **47** | 385 | 16 |
| **22** | 478 | 19 | | **48** | 349 | 16 |
| **23** | 473 | 19 | | **49** | 184 | 16 |
| **24** | 365 | 19 | | **50** | 167 | 16 |
| **25** | 287 | 19 | | **51** | 145 | 16 |
| **26** | 200 | 19 | | **52** | 139 | 16 |

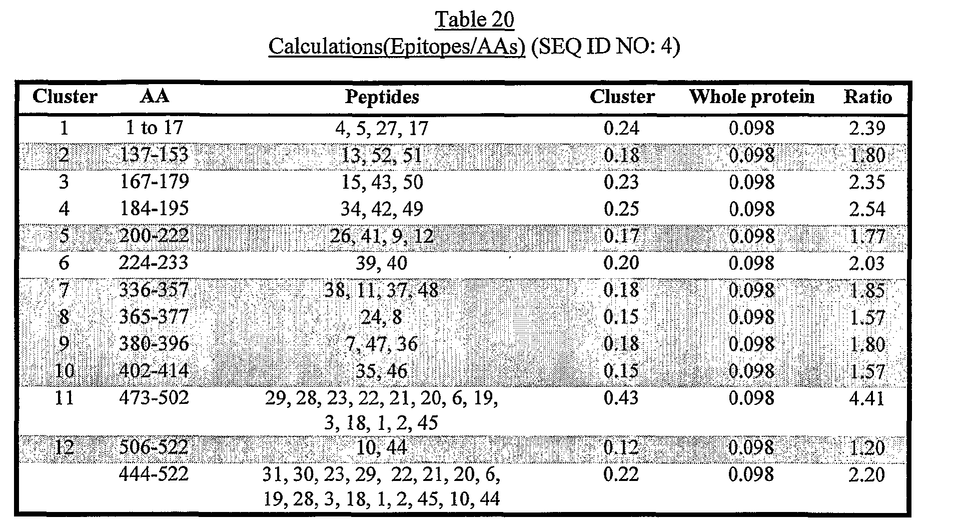

**Table 21**

| BIMAS-NIH/Parker algorithm Results (SEQ ID NO: 4) | | | |
|---|---|---|---|
| **Rank** | **Start** | **Score** | **Log(Score)** |
| **1** | 207 | 540.469 | 2.73 |
| **2** | 369 | 531.455 | 2.73 |
| **3** | 1 | 309.05 | 2.49 |
| **4** | 9 | 266.374 | 2.43 |
| **5** | 490 | 181.794 | 2.26 |
| **6** | 214 | 177.566 | 2.25 |
| **7** | 224 | 143.451 | 2.16 |
| **8** | 171 | 93.656 | 1.97 |
| **9** | 506 | 87.586 | 1.94 |
| **10** | 487 | 83.527 | 1.92 |
| **11** | 491 | 83.527 | 1.92 |
| **12** | 2 | 54.474 | 1.74 |
| **13** | 137 | 47.991 | 1.68 |
| **14** | 200 | 30.777 | 1.49 |
| **15** | 208 | 26.248 | 1.42 |
| **16** | 460 | 21.919 | 1.34 |
| **17** | 478 | 19.425 | 1.29 |
| **18** | 365 | 17.14 | 1.23 |
| **19** | 380 | 16.228 | 1.21 |
| **20** | 444 | 13.218 | 1.12 |
| **21** | 473 | 13.04 | 1.12 |
| **22** | 57 | 10.868 | 1.04 |
| **23** | 482 | 8.252 | 0.92 |
| **24** | 483 | 7.309 | 0.86 |
| **25** | 5 | 6.993 | 0.84 |
| **26** | 225 | 5.858 | 0.77 |
| **27** | 343 | 5.195 | 0.72 |
| **28** | 514 | 5.179 | 0.71 |

**Table 22**

| Calculations(Epitopes/AAs) (SEQ ID NO: 4) | | | | | |
|---|---|---|---|---|---|
| **Cluster** | **AA** | **Peptides** | **Cluster** | **Whole protein** | **Ratio** |
| 1 | 1 to 17 | 3, 12, 25, 4 | 0.24 | 0.053 | 4.45 |
| 2 | 200-222 | 14, 1, 15, 6 | 0.17 | 0.053 | 3.29 |
| 3 | 224-233 | 7,26 | 0.20 | 0.053 | 3.78 |
| 4 | 365-377 | 18,2 | 0.15 | 0.053 | 2.91 |
| 5 | 473-499 | 21, 17, 23, 24, 10, 5, 11 | 0.26 | 0.053 | 4.90 |
| 6 | 506-522 | 9,28 | 0.12 | 0.053 | 2.22 |
| 7 | 365-388 | 18, 2, 19 | 0.13 | 0.053 | 2.36 |
| 8 | 444-499 | 20, 16, 21, 17, 23, 24, 10, 5, 11 | 0.16 | 0.053 | 3.03 |
| 9 | 444-522 | 20, 16, 21, 17, 23, 24, 10, 5, 11, 9, 28 | 0.14 | 0.053 | 2.63 |
| 10 | 200-233 | 14, 1, 15, 6, 7, 26 | 0.18 | 0.053 | 3.33 |

Some embodiments of the invention are disclosed in the following items.

### Items

1. A vaccine comprising a first housekeeping epitope, wherein the housekeeping epitope is derived from a first antigen associated with a first target cell.
2. The vaccine of item 1 comprising a nucleic acid construct, wherein the construct encodes the housekeeping epitope.
3. The vaccine of item 1 or 2, wherein the target cell is a neoplastic cell.
4. The vaccine of item 3, wherein the neoplastic cell is selected from the group consisting of leukemia, carcinoma, lymphoma, astrocyloma, sarcoma, glioma, retinoblastoma, melanoma, Wilm's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, and brain cancer.
5. The vaccine of item 3, wherein the antigen is selected from the group consisting of MelanA (MART-1), gap100 (Pmel 17), tyrosinase, TRP-1, TRP-2, MAGE-1, MAGE-3, BAGE, GAGE-1, GAGE-2, CEA, RAGE. NY-HSO, SCP-1, Hom/[Mel-40, FRAME, p53, H-Ras, HER- 2/neu, BCR-ABL, E2A-PRL, H4-RET, IGH-IGK, MYL-RAR, Epstein Barr virus antigens, EBNA, human papillomavirus (HPV) antigen E6 and E7, TSP-180, MAGE-4, MAGE-5, MAGE-6, pl85erbB2, pl80erbB-3, c-met, nm-23Hl, PSA, TAG-72-4, CAM 17.1, NuMa, K-ras, beta-Catenin, CDK4, Mum-1, p16, TAGE, PSMA, PSCA, CT7, telomerase, 43-9F, 5T4, 791Tgp72, alpha-fetoprotein, beta-HCG, BCA225, BTAA, CA 125, CA 15-3 (CA 27.29\BCAA), CA 195, CA 242, CA-50, GAM43, CD68\KP1, CO-029, FGF-5, G250, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, and GA733-2\KSA.
6. The vaccine of item 1 or 2, wherein the target cell is infected by an intracellular parasite.
7. The vaccine of item 6, wherein the intracellular parasite is a virus.
8. The vaccine of item 7, wherein the virus is selected from the group consisting of: adenovirus, cytomegalo virus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, and human T cell leukemia virus II.
9. The vaccine of item 7, wherein the antigen is a virus-associated antigen.
10. The vaccine of item 6, wherein the intracellular parasite is a bacterium, a protozoan, a fungus, or a prion.
11. The vaccine of item 10, wherein the intracellular parasite is selected from the group consisting of Chlamydia, Listeria, Salmonella, Legionella, Brucella, Coxiella, Rickettsia, Mycobacterium, Leishmania, Trypanasoma, Toxoplasma, and Plasmodium.
12. The vaccine of item 10, wherein the antigen is parasite-associated antigen.
13. The vaccine of item 1 or 2, wherein the housekeeping epitope comprises or encodes a polypeptide of about 6 to about 23 amino acids in length.
14. The vaccine of item 13, the polypeptide being 9 or 10 amino acids in length.
15. The vaccine of item 1, wherein the housekeeping epitope comprises a nucleic acid.
16. The vaccine of item 1, wherein the housekeeping epitope comprises a synthetic polypeptide.
17. The vaccine of any of the preceding items wherein the vaccine further comprises at least one component selected from the group consisting of buffers, detergents, surfactants, anti- oxidants, or reducing agents.
18. The vaccine of any of the preceding items, wherein the housekeeping epitope is specific for at least one allele of MHC.
19. The vaccine of item 18, wherein the allele encodes type A2.
20. The vaccine of item 18, wherein the allele encodes a type selected from the group consisting of A1, A3, A11, and A24.
21. The vaccine of item 18, wherein the allele encodes a type selected from the group consisting of A26, A29, B7, B8, B14, B18, B27, B35, B44, B62, B60, and B51.
22. The vaccine of any of the preceding items, further comprising an immune epitope.
23. The vaccine of item 22, wherein the immune epitope is derived from a second antigen associated with the target cell.
24. The vaccine of item 21, wherein the first antigen and the second antigen are the same.
25. The vaccine of item 22, 23, or 24 wherein the housekeeping epitope is specific for a first allele of MHC, and wherein the immune epitope is specific for a second allele of MHC.
26. The vaccine of item 25, wherein the first allele and the second allele are the same.
27. The vaccine of item 25, wherein the first allele and the second allele are not the same.
28. The vaccine of any of items 22-27, wherein the vaccine comprises an epitope cluster, and wherein the epitope cluster comprises the immune epitope.
29. The vaccine of item 28, wherein the epitope cluster comprises or encodes a polypeptide having a length, wherein the length is at least 10 amino acids.
30. The vaccine of item 29, wherein the length of the polypeptide is less than about 60 amino acids.
31. The vaccine of item 28, wherein the epitope cluster is derived from a second antigen associated with the target cell.
32. The vaccine of item 31, wherein the first antigen and the second antigen are the same.
33. The vaccine of item 31 or 32, the second antigen having a length, wherein the epitope cluster consists of or encodes a polypeptide having a length, wherein the length of the polypeptide is less than about 80% of the length of the second antigen.
34. The vaccine of item 33, wherein the length of the polypeptide is less than about 50% of the length of the second antigen.
35. The vaccine of item 34, wherein the length of the polypeptide is less than about 20% of the length of the second antigen.
36. The vaccine of any of the preceding items, further comprising a second housekeeping epitope, wherein the second housekeeping epitope is derived from a second, antigen associated with a second target cell.
37. The vaccine of item 36, wherein the first antigen and the second antigen are the same.
38. The vaccine of item 36, wherein the first antigen and the second antigen are not the same.
39. The vaccine of item 36, wherein the first target cell and the second target cell are the same.
40. The vaccine of item 36, wherein the first target cell and the second target cell are not the same.
41. A method of making a vaccine, comprising the steps of: selecting a first housekeeping epitope by identifying polypeptide fragments that are or could be produced by a housekeeping proteasome from a first antigen associated with a first target cell or target pathogen; and preparing a vaccine composition comprising or encoding the selected housekeeping epitope.
42. An isolated T cell expressing a T cell receptor specific for an MHC-peptide complex comprising a first housekeeping epitope, wherein the housekeeping epitope is derived from a first antigen associated with a first target cell.
43. A T cell clone comprising the T cell of item 42.
44. A polyclonal population of T cells comprising the T ceil of item 42.
45. The T cell of item 42 produced by an in vitro immunization.
46. The T cell of item 42 isolated from an immunized animal.
47. A method of making an adoptive immunotherapeutic, comprising: the T cell of any of items 42-46 with a pharmaceutically acceptable adjuvant, carrier, diluent, or excipient.
48. The use of the T cell of any of items 42-46 in the manufacture of a medicament for use in adoptive immunotherapy.
49. A method of epitope discovery comprising the step of selecting an epitope from a population of peptide fragments of an antigen associated with a target in a host, wherein the fragments have a known or predicted affinity for a major histocompatibility complex receptor peptide binding cleft of the host, wherein the epitope selected corresponds to a product of proteolytic cleavage of the antigen in a cell of the host.
50. The method of item 49 wherein the target is a target therein the major histocompatibility complex receptor is a class I major histocompatibility complex receptor, wherein the product of proteolytic cleavage is a proteasome cleavage product, and wherein the cell of the host is the target cell.
51. The method of item 49 wherein the major histocompatibility complex receptor is a class 11 major histocompatibility complex receptor.
52. The method of item 51, the target is selected from the group consisting of a neoplastic cell, a pathogen, a toxin, a venom, and an allergen.
53. A method of discovering an epitope comprising the steps of: providing a sequence from a target cell, wherein the sequence encodes or comprises a protein expressed the target cell; identifying a population of peptide fragments of the protein, wherein members of the population of peptide fragments have a known or predicted affinity lor a major histocompatibility complex class I receptor peptide binding cleft; selecting the epitope from the population of peptide fragments, wherein the epitope corresponds to a product of a proteasome active in the target cell.
54. The method of item 50 or 53, wherein the target cell is a neoplastic cell or an antigen presenting cell.
55. A method of epitope discovery comprising the steps of: providing a neoplastic cell and a sequence, wherein the sequence comprises or encodes an antigen associated with the neoplastic cell; identifying a population of peptide fragments of the antigen, wherein the population of peptide fragments is predicted to have an affinity for a major histocompatibility complex class I receptor peptide binding cleft; selecting an epitope from the population of peptide fragments, wherein the epitope is determine by in vitro analysis to be a proteasome cleavage reaction product of a proteasome active in the neoplastic cell.
56. The method of item 52,54, or 55, wherein the neoplastic cell is selected from the group consisting of leukemia, carcinoma, lymphoma, astrocytoma, sarcoma, glioma, retinoblastoma, melanoma, Wilm's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, and brain cancer.
57. The method of item 52,54,55 or 56, wherein the antigen is selected from the group consisting of MclanA (MART-I), gplO0 (Pmel 17), tyrosinase, TRF-1, TRP-2, MAGE-1, MAGE- 3, BAGE, GAGB-1, GAGE-2, CEA, RAOE, NY-ESO, SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE- 6, p185erbB2, p180erbB-3, c-met, nm-23H1, PSA, TAG-72-4, CAM 17.1, NuMa, K-ras, beta-Catenin, CDK4, Mum-1, and p16.
58. The method of item 50 or 53, wherein the target cell is infected by an intracellular parasite.
59. The method of item 52, wherein the pathogen is an intracellular parasite.
60. The method of item 58 or 59, wherein the parasite is a virus.
61. The method of item 60, wherein the virus is selected from the group consisting of : adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, and human T cell leukemia virus II, a rotaviruses, a reovirus, a picomavirus, a hepadnavirus, an adenovirus, and a papovavirus.
62. The method of item 60, wherein the antigen is selected from the group consisting of adenovirus, cytomegalovirus, Epstein-Barr virus, herpes simplex virus 1, herpes simplex virus 2, human herpesvirus 6, varicella-zoster virus, hepatitis B virus, hepatitis D virus, papilloma virus, parvovirus B19, polyomavirus BK, polyomavirus JC, hepatitis C virus, measles virus, rubella virus, human immunodeficiency virus (HIV), human T cell leukemia virus I, and human T cell leukemia virus II.
63. The method of item 52, wherein the pathogen is selected from the group consisting of a bacterium, a fungus, a protozoan, and a prion.
64. The method of item 58, wherein the intracellular parasite is a bacterium, a protozoan, a fungus, or a prion.
65. The method of item 63 or 64, wherein the pathogen or intracellular parasite is selected from the group consisting of *Chlamydia*, *Listeria, Salmonella, Legionella, Brucella, Coxiella, Rickettsia, Mycobacterium, Leishmania, Trypanasoma, Toxoplasrna, and Plasmodium.*
66. The method of item 58, the antigen is a parasite-associated antigen.
67. The method of item 50, 53, or 55, wherein the proteasome is a housekeeping proteasome.
68. The method of item 50, 53, or 55, the proteasome is an immune proteasome.
69. The method of item 49 or 51, wherein the proteolytic cleavage comprises an activity of a protease in a of the host.
70. The method of item 49 or 51, wherein the proteolytic cleavage comprises an activity of a protease in an endosome of the host.
71. The method of any of items 49-70, wherein, the affinity is determined by an in vitro binding assay.
72. The method of any of items 49-70, wherein the affinity is determined by an algorithm.
73. The method of any of items 49-72, wherein the product is determined by in vitro analysis.
74. An epitope discovered according to the method of any of items 49-73.
75. The epitope of item 74, having a sequence of SEQ ID NO: 78.
76. An MHC class I-resrricted epitope discovered by the process comprising the steps of: determining the type of proteasome expressed by a target cell, and identifying epitopes expressed by said target cell.
77. The epitope of item 76 wherein the determining step comprises a technique selected from the group consisting of RT-PCR, ELISA, western blotting, polyacrylamide gel electrophoresis, immunohistochemistry, and cleavage of standard substrates with proteasomes purified from said target cell.
78. The epitope of item 76 wherein the determining step comprises comparing untreated and treated target cells.
79. The epitope of item 76 wherein the identifying step comprises a technique selected from the group consisting of peptide elution, in vitro proteolysis, mass spectrometry, HPLC, amino acid sequencing, and determination of immunoreactivity.
80. The epitope of item 79 wherein the determination of immunoreactivity comprises an assay to detect a condition selected from the group consisting of cytotoxicity, proliferation, and cytokine production.
81. A vaccine comprising the epitope of any of items 74-80.
82. An epitopes cluster, the cluster being derived from an antigen associated with a target, the cluster comprising or encoding at least two sequences having a known or predicted affinity for an MHC receptor peptide binding cleft, wherein the cluster is an incomplete fragment of the antigen.
83. The epitope cluster of item 82, wherein the target is a neoplastic cell.
84. The epitope cluster of item 82, wherein the target is a cell infected by an intracellular parasite.
85. The epitope cluster of item 84, wherein the intracellular parasite is a virus.
86. The epitope cluster of item 84, wherein the intracellular parasite is a bacterium or a protozoan.
87. The epitope cluster of item 82, wherein the target is a pathogenic agent.
88. The epitope cluster of item 87, wherein the pathogenic agent is selected from the group consisting of a virus, a bacterium, a fungus, a protozoan, a prion, a toxin, a venom, and an allergen.
89. The epitope cluster of item 82, wherein the MHC receptor is a class I HLA receptor.
90. The epitope cluster of item 82, wherein the MHC receptor is a class II HLA receptor.
91. The epitope cluster of item 82, wherein the cluster comprises or encodes a polypeptide having a length, wherein the length is at least 10 amino acids.
92. The epitope cluster of item 91, wherein the length of the polypeptide is less than about 75 amino acids.
93. The epitope cluster of item 82, the antigen having a length, wherein the cluster consists of or encodes a polypeptide having a length, wherein the length of the polypeptide is less than about 80% of the length of the antigen.
94. The epitope cluster of item 93, the antigen having a length, wherein the cluster consists of or encodes a polypeptide having a length, wherein the length of the polypeptide is less than about 50% of the length of the antigen.
95. The epitope cluster of item 94, the antigen having a length, wherein the cluster consists of or encodes a polypeptide having a length, wherein the length of the polypeptide is less than about 20% of the length of the antigen.
96. A method of epitope cluster discovery comprising the steps of: providing a sequence of an antigen associated with a target; scoring candidate peptides within the sequence, based on known or predicted affinity for an MBC receptor peptide binding cleft, to identity putative MHC epitopes; and identifying an epitope cluster within the antigen, wherein the cluster comprises at least two of the putative MHC epitopes, and wherein the cluster comprises a higher density of putative MHC epitopes than a density of putative MHC epitopes in the antigen as a whole.
97. The method of item 96, wherein the target is a neoplastic cell.
98. The method of item 96, wherein the target is a cell infected by an intracellular parasite.
99. The method of item 98, wherein the intracellular parasite is a virus.
100. The method of item 98, wherein the intracellular parasite is a bacterium or a protozoan.
101. The method of item 96, wherein the target is a pathogenic agent.
102. The method of item 96, wherein the MHC receptor binding cleft is a class I HLA receptor binding cleft.
103. The method of item 96, wherein the MHC receptor binding cleft is a class II HLA receptor binding cleft.
104. The method of item 96, wherein the cluster comprises or encodes a polypeptide having a length, wherein the length is at least 10 amino acids.
105. The method of item 104, wherein the length of the polypeptide is less than about 75 ammo acids.
106. The method of item 96, the antigen having a length, wherein the cluster consists essentially of or encodes a polypeptide having a length, wherein the length of the polypeptide is less than about 80% of the length of the antigen.
107. The method of item 106, the antigen having a length, wherein the cluster consists essentially of or encodes a polypeptide having a length, wherein the length of the polypeptide is less than about 50% of the length of the antigen.
108. The method of item 107, the antigen having a length, wherein the cluster consists essentially of or encodes a polypeptide having a length, wherein the length of the polypeptide is less than about 20% of the length of the antigen.
109. A nucleic acid construct comprising a first coding region, wherein the first coding region comprises a first sequence encoding at least a first polypeptide, wherein the first polypeptide comprises a first housekeeping epitope derived from a first antigen associated with a first target, cell.
110. The nucleic acid construct of item 109, wherein the first, coding region further comprises a second sequence encoding at least a second polypeptide, wherein the second polypeptide comprises a second epitope derived from a second antigen associated with a second target cell.
111. The nucleic acid construct of item 110, wherein the first polypeptide and the second polypeptide are contiguous.
112. The acid construct of item 110, wherein the first polypeptide and the second polypeptide are not contiguous.
113. The nucleic acid construct of item 110, the second epitope is a housekeeping epitope.
114. The nucleic acid construct of item 110, wherein the second epitope is an immune epitope.
115. The nucleic acid construct of item 110, wherein the first antigen and the second antigen are the same.
116. The nucleic acid construct of item 110, wherein the first antigen and the second antigen are not the same.
117. The nucleic acid construct of item 110, wherein the first, target cell and the second target cell are the same.
118. The nucleic acid construct of item 110, wherein the first target cell and the second target cell are not the same.
119. The nucleic acid construct of item 109, wherein the first target cell is a neoplastic cell.
120. The nucleic acid construct of item 119, wherein the neoplastic cell is selected from the group consisting of leukemia, carcinoma, lymphoma, astrocytoma, sarcoma, glioma, retinoblastoma, melanoma, Wilm's tumor, bladder cancer, breast cancer, colon cancer, hepatocellular cancer, pancreatic cancer, prostate cancer, lung cancer, liver cancer, stomach cancer, cervical cancer, testicular cancer, renal cell cancer, and brain cancer.
121. The nucleic acid construct of item 119, wherein the first antigen is selected from the group consisting of MART-I/MelanA, gp 100 (Pmel 17), tyrosinase, TRP-1, TRP-2, MAGE-1, MAGE-3, BAGE, GAGE-1, GAGE-2, CEA, RAGE, SCP-1, Hom/Mel-40, PRAME, p53, H-Ras, HER-2/neu, BCR-ABL, E2A-PRL, h4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1 PSA, TAG-72-4, CAM 17.1, NuMa, K-ras, beta-Catenin, CDK4, Mum-1, p16, p15, NY-ESO, products of an SSX gene family member, CT-7, and products of an SCP gene family member.
122. The nucleic acid construct of item 109, wherein the target cell is infected by an intracellular parasite.
123. The nucleic acid construct of item 122, wherein the intracellular parasite is a virus.
124. The nucleic arid construct of item 123, wherein the virus is selected from the group consisting of adenovirus, cytomegalovirus, Epstein-Barr virus, herpes simplex virus 1 and 2, human herpesvirus 6, varicella-zoster virus, hepatitis B virus, hepatitis D virus, papillomavirus, parvovirus B19, polyomavirus BK, polyomavirus JC, hepatitis C virus, measles virus, rubella virus, human immunodeficiency virus (HIV), human T-cell leukemia virus I, and human T-cell leukemia virus II.
125. The nucleic arid construct of item 122, wherein the intracellular parasite is a bacterium, a protozoan, a fungus, or a prion.
126. The nucleic acid construct of item 125, wherein the intracellular parasite is selected from the group consisting of *Chlamydia, Listeria, Salmonella, Legionella, Brucella*, *Coxiella, Rickettsia, Mycobacterium, Leishmania, Trypanasoma, Toxoplasma, and Plasmodium*.
127. The nucleic acid construct of item 109, further comprising a first promoter sequence operably linked to the first coding region.
128. The nucleic acid construct of item 127 wherein the promoter is derived from cytomegalovirus (CMV), SV40, or a retroviral long terminal repeat (LTR).
129. The nucleic acid construct of Item 127 wherein the promoter is a bidirectional promoter.
130. The nucleic acid construct of item 127, further comprising a second promoter sequence operably linked to a second coding region.
131. The nucleic acid construct of item 109 further comprising a poly-A sequence operably linked to the first coding region.
132. The nucleic acid construct of item 110, further comprising an internal ribosome entry site (IRES) sequence.
133. The nucleic acid construct of item 132, wherein the IRES sequence is between the first and the second coding sequences.
134. The nucleic acid construct of item 110, further comprising a ubiquitin sequence.
135. The nucleic acid construct of item 134, wherein the ubiquitin sequence is between the first and the second coding sequences.
136. The nucleic acid construct of item 110, further comprising a sequence encoding an autocatalytic peptide.
137. The nucleic acid construct of item 136, wherein the autocatalytic peptide-encoding sequence is between the first and the second coding sequences.
138. The nucleic acid construct of item 109, further comprising a sequence selected from the group consisting of enhancers, nuclear import sequences, immunostimulatory sequences, and expression cassettes for cytokines, selection markers, or reporter molecules.
139. The nucleic acid construct of item 109, wherein the first polypeptide is about 7 to 15 amino acids in length.
140. The nucleic acid construct of item 109, wherein the first polypeptide is 9 or 10 amino acids in length.
141. The nucleic acid construct of item 110, the second polypeptide is 9 or 10 amino acids in length.
142. The nucleic acid construct of item 110, wherein the second polypeptide is an epitope cluster between about 10 and about 75 amino acids in length.
143. The nucleic acid construct of item 110, wherein the first epitope has a binding affinity for a first MHC allele, and wherein the second epitopes has a binding affinity for a second MHC allele.
144. The nucleic acid construct of item 143, wherein the first allele and the second allele are the same.
145. The nucleic acid construct of item 143, wherein the first allele and the second allele are not the same.
146. The nucleic acid construct of item 109 further comprising at least a second coding region wherein the second coding region comprises a second sequence encoding at least a second polypeptide, wherein the second polypeptide comprises a second epitope derived from a second antigen associated with a second target cell.
147. The nucleic acid construct of item 146 wherein said first and second coding regions are operably linked to either side of a single bidirectional promoter.
148. The nucleic acid construct of item 146 wherein at least one coding region comprises multiple sequences encoding multiple polypeptides, wherein each polypeptide comprises at least one epitope derived from an antigen associated with a target cell.
149. The nucleic acid construct of item 129 wherein the bidirectional promoter is further operably linked to a second sequence, said second sequence encoding an adjuvant.
150. The nucleic acid construct of item 136 wherein the encoded peptide possesses autoproteolytic activity.
151. The nucleic acid construct of item 136 wherein the encoded peptide inhibits peptide bond formation.
152. A vaccine comprising the nucleic acid construct of any of items 109-151.
153. A method of making a vaccine, comprising the step of: preparing a vaccine composition comprising the nucleic acid construct of any of items 109-151.
154. A vaccine made according to the method of item 41 or 153.
155. A method of treating an animal comprising: administering to an animal the vaccine any of items 1-40,81,152, or 154.
156. The method of item 155, wherein the administering step comprises a mode of delivery selected from the group consisting of transdermal, intranodal, perinodal, oral, intravenous, intradermal, intramuscular, intraperitoneal, and mucosal.
157. The method of item 155, further comprising a step of assaying to determine a characteristic indicative of a state of the target cells.
158. The method of item 157, comprising a first assaying step and a second assaying step, wherein the first assaying step precedes the administering step, and wherein the second assaying step follows the administering step.
159. The method of item 158, further comprising a step of comparing the characteristic determined in the first assaying step with the characteristic determined in the second assaying step to obtain a result.
160. The method of item 159, wherein the result is selected from the group consisting of: a diminution in number of target cells, a loss of mass or size of a tumor comprising target cells, or a decrease in number or concentration of an intracellular parasite infecting target cells.
161. The use of a housekeeping epitope in the manufacture of a vaccine for use in the method of treatment in any items 155-160.

## Claims

1. An epitope cluster, the cluster being derived from an antigen associated with a target, the cluster comprising or encoding at least two sequences having a known or predicted affinity for an MHC receptor peptide binding cleft, wherein the cluster is an incomplete fragment of the antigen.

2. The epitope cluster of claim 1, wherein the target is selected from the group consisting of a neoplastic cell and a pathogenic agent.

3. The epitope cluster of claim 1, wherein the target is a cell infected by an intracellular parasite, such as a virus, a bacterium, or a protozoan.

4. The epitope cluster of claim 2, wherein the pathogenic agent is selected from the group consisting of a virus, a bacterium, a fungus, a protozoan, a prion, a toxin, a venom, and an allergen.

5. The epitope cluster of claim 1, wherein the MHC receptor is selected from the group consisting of a class I HLA receptor and a class II HLA receptor.

6. The epitope cluster of claim 1, wherein the cluster comprises or encodes a polypeptide having a length, wherein the length is at least 10 amino acids and/or less than about 75 amino acids.

7. The epitope cluster of claim 1, wherein the cluster consists of or encodes a polypeptide having a length, wherein the antigen has a length, and wherein the length of the polypeptide is less than about 80% of the length of the antigen, such as less than about 50% of the length of the antigen, such as less than about 20% of the length of the antigen.

8. A method of epitope cluster discovery comprising the steps of: providing a sequence of an antigen associated with a target; scoring candidate peptides within the sequence, based on known or predicted affinity for an MHC receptor peptide binding cleft, to identify putative MHC epitopes; and identifying an epitope cluster within the antigen, wherein the cluster comprises at least two of the putative MHC epitopes, and wherein the cluster comprises a higher density of putative MHC epitopes than a density of putative MHC epitopes in the antigen as a whole.

9. The method of claim 8, wherein the target is selected from the group consisting of a neoplastic cell and a pathogenic agent.

10. The method of claim 8, wherein the target is a cell infected by an intracellular parasite, such as a virus, a bacterium, or a protozoan.

11. The method of claim 8, wherein the MHC receptor binding cleft is selected from the group consisting of a class I HLA receptor and a class II HLA receptor.

12. The method of claim 8, wherein the cluster comprises or encodes a polypeptide having a length, wherein the length is at least 10 amino acids and/or less than about 75 amino acids.

13. The method of claim 8, wherein the cluster consists essentially of or encodes a polypeptide having a length, wherein the antigen has a length, and wherein the length of the polypeptide is less than about 80% of the length of the antigen, such as less than about 50% of the length of the antigen, such as less than about 20% of the length of the antigen.
